(19) **Europäisches Patentamt / European Patent Office / Office européen des brevets**

(11) **EP 4 267 767 B1**

(12) **EUROPEAN PATENT SPECIFICATION**

(45) Date of publication and mention
of the grant of the patent:
**01.04.2026 Bulletin 2026/14**

(21) Application number: **21912061.5**

(22) Date of filing: **21.12.2021**

(51) International Patent Classification (IPC):
**C12Q 1/6886** (2018.01)     **C12Q 1/6806** (2018.01)
**G16B 20/20** (2019.01)     **A61K 31/713** (2006.01)

(52) Cooperative Patent Classification (CPC):
**C12Q 1/6886; A61K 31/713; C12Q 2600/156; C12Q 2600/158**

(86) International application number:
**PCT/US2021/064657**

(87) International publication number:
**WO 2022/140420 (30.06.2022 Gazette 2022/26)**

(54) **IGH REARRANGEMENTS AND USES THEREOF**

IGH-NEUANORDNUNGEN UND VERWENDUNGEN DAVON

RÉARRANGEMENTS IGH ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.12.2020 US 202063129286 P**

(43) Date of publication of application:
**01.11.2023 Bulletin 2023/44**

(73) Proprietor: **Foundation Medicine, Inc.**
**Boston, MA 02210 (US)**

(72) Inventors:
• **ROSENZWEIG, Mark**
**Cambridge, Massachusetts 02141 (US)**
• **ZHONG, Lei**
**Cambridge, Massachusetts 02141 (US)**
• **ERBACH, Rachel**
**Cambridge, Massachusetts 02141 (US)**

(74) Representative: **CMS Cameron McKenna Nabarro Olswang LLP**
**Cannon Place**
**78 Cannon Street**
**London EC4N 6AF (GB)**

(56) References cited:
WO-A1-2013/033420     US-A- 5 024 934
US-A1- 2007 009 917     US-A1- 2012 237 930
US-A1- 2018 363 066

• HE JIE ET AL: "Integrated genomic DNA/RNA profiling of hematologic malignancies in the clinical setting", BLOOD, AMERICAN SOCIETY OF HEMATOLOGY, US, vol. 127, no. 24, 16 June 2016 (2016-06-16), pages 3004 - 3014, XP086509672, ISSN: 0006-4971, [retrieved on 20201126], DOI: 10.1182/BLOOD-2015-08-664649
• WALKER B A ET AL: "Translocations at 8q24 juxtapose MYC with genes that harbor superenhancers resulting in overexpression and poor prognosis in myeloma patients", vol. 4, no. 3, 14 March 2014 (2014-03-14), GB, pages e191 - e191, XP093215016, ISSN: 2044-5385, Retrieved from the Internet <URL:http://www.nature.com/articles/bcj201413> DOI: 10.1038/bcj.2014.13
• LÓPEZ CRISTINA, KLEINHEINZ KORTINE, AUKEMA SIETSE M., ROHDE MARIUS, BERNHART STEPHAN H., HÜBSCHMANN DANIEL, WAGENER RABEA, TOPRAK : "Genomic and transcriptomic changes complement each other in the pathogenesis of sporadic Burkitt lymphoma", NATURE COMMUNICATIONS, vol. 10, no. 1, 1 December 2019 (2019-12-01), XP055953313, DOI: 10.1038/s41467-019-08578-3

**Description**

CROSS-REFERENCE TO RELATED APPLICATION

This application claims the benefit of U.S. Provisional Application 63/129,286, filed December 22, 2020,

FIELD

Provided herein are methods related to detecting overexpression of an oncogene through translocations in the immunoglobulin heavy (*IGH*) locus, as well as methods of diagnosis/treatment, uses, and kits related thereto.

BACKGROUND

Translocations involving the immunoglobulin heavy (*IGH*) locus at the chromosome band 14q32 have been reported in a wide range of hematological malignancies, most frequently in B-cell lymphomas (Küppers, R. (2005) Nat. Rev. Cancer 5:251-262) and multiple myeloma (MM) (Jiménez, C. et al. (2016) J. Mol. Diagnostics 19:1-8; Kuehl, W.M. and Bergsagel, P.L. (2002) Nat. Rev. Cancer 2:175-187), and less commonly, but recurrently, in acute lymphoblastic leukemia (ALL) (Jeffries, S.J. et al. (2014) Haematologica 99:1334-1342; Chapiro, E. et al. (2013) Cancer Genet. 206:162-173; Woo, J.S. et al. (2014) Erp. Hematol. Oncol. 3:16). Juxtaposition of oncogenes to the IGH locus is thought to drive oncogene overexpression due to the influence of strong IGH transcriptional enhancers, which is essential for oncogenesis.

Multiple IGH rearrangements have established diagnostic, prognostic, and/or therapeutic roles in the routine clinical management of a variety of hematological malignancies (Dupain, C. et al. (2017) Mol. Ther. Nucleic Acid 6:315-326; Copie-Bergman, C. et al. (2015) Blood 126:2466-2474; Xu-Monette, Z.Y. et al. (2015) Mod. Pathol. 28:1555-1573; De Braekeleer, M. et al. (2016) Mod. Clin. Oncol. 4:682-694; Manier, S. et al. (2016) Nat. Rev. Clin. Oncol. doi:10.1038/nrclinonc.2016.122; Sawyer, J. R. (2011) Cancer Genet. 204:3-12). For example, t(8;14)(q24;q32) (IGH-MYC) is the diagnostic chromosomal translocation in Burkitt lymphoma, observed in ~80% of these tumors (Hecht, J.L. et al. (2000) J. Clin, Oncol. 18:3707-3721). The t(X;14)(p22;q32) (IGH-CRLF2) is associated with poor prognosis in patients with ALL (Moorman, A. V. et al. (2012) J. Clin. Oncol. 30:3100-3108; Russell, L.I. et al. (2014) J. Clin. Oncol. 32:1453-1462), whereas co-occurrence of IGH-MYC with IGH-BCL2 and/or IGH-BCL6 translocations, known as "double hit" or "triple hit," correlates with poor prognosis in patients with diffuse large B-cell lymphoma (DLBCL) (Zelenetz, A.D. et al. (2016) J. Natl. Compr. Canc. Netw. 14:196-231).

Moreover, the presence of IGH translocations may suggest different treatment options. For example, t(4;14) (IGH-FGFR3/MMSET), t(14;16) (IGH-MAF), or t(14;20) (IGH-MAFB) translocations define a subgroup of high-risk MM patients with poor prognosis using conventional therapies, whereas patients with t(4;14) have been reported to achieve better outcome with bortezomib- or lenalidomide-containing regimens (Kalff, A. et al. (2012) Blood Cancer J.. 2:e89-8). Patients with t(11;14)(q 13;q32) (IGH-CCND1)-positive mantle cell lymphoma (MCL) have benefitted from the CDK4/6 inhibitor palbociclib (Leonard, J.P. et al. (2013) Blood 119:4597-4607). IGH translocation with 18q21 may result in upregulation of either BCL2 (Nunez, G. et al. (1989) Proc. Natl. Acad. Sci. 86:4589-4593; Han, Y. et al. (2013) Zhonghua Yi Xue Yi Chuan Xue Za Zhi 30:143-147; Monni, O. et al. (1999) Leuk. Lymphoma 34:45-52; Maeshima, A.M. et al. (2013) Cancer Sci. 104:952-957), which may suggest increased sensitivity to BCL2 inhibitors such as venetoclax (Souers, A.J. et al. (2013) Nat. Med. 19:202-208; Ross, J. et al. (2015) Blood 126:2975), or of MALT1 (Sanchez-Izquierdo, D. et al. (2003) Blood 101:4539-4546; Ye, H. et al. (2005) J. Pathol. 205:293-301), a positive regulator of NF-kB signaling (Ho, L. et al. (2005) Blood doi: 10.1 182/blood-2004-06-2297; Du, M.Q. (2017) Best Pract. Res. Clin. Haematol.. doi:10.1016/j.beha.2016.09.002; Schulze-Luehrmann, J. and Ghosh, S. (2006) Immunity 25:701-715) located 4.5 Mb from *BCL2* and not appreciably linked to venetoclax sensitivity. Therefore, accurate and consistent detection and description of IGH translocations is of special clinical importance in disease diagnosis, classification, and management.

Currently, fluorescence in situ hybridization (FISH) is routinely used in clinical care to detect chromosomal translocations. This technique involves sequential use of several commercially available break-apart fluorescent probes, followed by expert interpretation of the signals. Although FISH has high sensitivity and specificity, dependency on probes restricts the analysis to the translocations for which specific probes are designed, and only a limited number of translocations can be assessed at a time (Gozzetti, A. and Le Beau, M.M. (2000) Semin. Hematol. 37:320-333). FISH does not provide precise breakpoint information, due to the low-resolution nature of hybridization, and limited data suggests that not all observed IGH translocations lead to oncogene overexpression (Santra, M. et al. (2003) Blood 101:2374-2376), possibly because the oncogenes are located too far away from the translocation breakpoints to be efficiently upregulated by IGH enhancers. However, there have been no systematic large-scale studies analyzing the impact of the distance between IGH and an oncogene on oncogene overexpression.

Therefore, there remains a need for methods related to detecting overexpression of an oncogene, e.g., in order to provide individualized diagnoses, assessments, and/or treatment options for cancer using targeted approaches.

SUMMARY

To meet these and other needs, provided herein are methods related to detecting overexpression of an oncogene through translocations in the immunoglobulin heavy (IGH) locus, as well as methods of diagnosis/assessment/selection/patient stratification/treatment, uses, and kits related thereto.

In certain aspects, provided herein is a method of detecting overexpression of an oncogene. In some embodiments, the method comprises detecting in a sample from an individual a presence of a translocation comprising a first breakpoint in the immunoglobulin heavy (IGH) locus and a second breakpoint, wherein the second breakpoint of the translocation is 1.3Mb or less from a transcription start site (TSS) of the oncogene.

In other aspects, provided herein is a method of assessing or diagnosing overexpression of an oncogene in an individual. In some embodiments, the method comprises (a) detecting in a sample from an individual a presence of a translocation comprising a first breakpoint in the immunoglobulin heavy (IGH) locus and a second breakpoint; and (b) providing an assessment or diagnosis of overexpression of the oncogene when the second breakpoint is detected 1.3Mb or less from the TSS of the oncogene. In some embodiments, the second breakpoint is 1.3Mb or less from a transcription start site (TSS) of the oncogene. In some embodiments, the method comprises providing an assessment or diagnosis of lack of overexpression of the oncogene when the second breakpoint is detected more than 1.3Mb from the TSS of the oncogene.

In other aspects, provided herein is a method of detecting overexpression of an oncogene. In some embodiments, the method comprises (a) detecting in a sample from an individual a presence of a translocation comprising a first breakpoint in the IGH locus and a second breakpoint, wherein the second breakpoint is 1.3Mb or less from a TSS of the oncogene, and wherein the translocation is detected in DNA from the sample; and (b) measuring expression of the oncogene in a sample from the individual, wherein expression of the oncogene is measured in RNA from the sample. In some embodiments, the DNA and RNA are from the same sample. In some embodiments, the DNA and RNA are from different samples from the individual.

In other aspects, provided herein is a method of monitoring an individual having cancer. In some embodiments, the method comprises acquiring knowledge of a translocation comprising a first breakpoint in the IGH locus and a second breakpoint in a sample from the individual, wherein the second breakpoint is 1.3Mb or less from a TSS of an oncogene, and wherein, responsive to the acquisition of said knowledge, the individual is predicted to have increased risk of overexpression of the oncogene, as compared to an individual whose cancer does not exhibit a translocation comprising a breakpoint 1.3Mb or less from the TSS.

In other aspects, provided herein is a method of screening an individual having cancer. In some embodiments, the method comprises acquiring knowledge of a translocation comprising a first breakpoint in the IGH locus and a second breakpoint in a sample from the individual, wherein the second breakpoint is 1.3Mb or less from a TSS of an oncogene, and wherein, responsive to the acquisition of said knowledge, the individual is predicted to have increased risk of overexpression of the oncogene, as compared to an individual whose cancer does not exhibit a translocation comprising a breakpoint 1.3Mb or less from the TSS.

In other aspects, provided herein is a method of evaluating an individual having cancer. In some embodiments, the method comprises: acquiring genotype information that identifies a translocation comprising a first breakpoint in the IGH locus and a second breakpoint in the individual, wherein the second breakpoint is 1.3Mb or less from a TSS of an oncogene, and wherein the presence of the translocation identifies the subject as having an increased risk of overexpression of the oncogene, as compared to an individual whose cancer does not exhibit a translocation comprising a breakpoint 1.3Mb or less from the TSS.

In other aspects, provided herein is a method of generating a personalized cancer treatment report. In some embodiments, the method comprises (a) obtaining a sample from an individual; (b) detecting a presence of a translocation comprising a first breakpoint in the IGH locus and a second breakpoint in the sample, wherein the second breakpoint is 1.3Mb or less from a TSS of an oncogene; and (c) providing a report comprising information on the oncogene, the translocation, or both. In some embodiments, the method further comprises selecting one or more treatments based on the detection of the second breakpoint 1.3Mb or less from the TSS of oncogene, wherein the report further comprises information on the one or more treatments.

In other aspects, provided herein is a method of identifying an individual having cancer who may benefit from a treatment comprising a targeted therapy. In some embodiments, the method comprises detecting a presence of a translocation comprising a first breakpoint in the IGH locus and a second breakpoint in a sample from the individual, wherein the second breakpoint is 1.3Mb or less from a transcription start site (TSS) of an oncogene, and wherein presence of the second breakpoint 1.3Mb or less from the TSS of the oncogene in the sample identifies the individual as one who may benefit from a targeted therapy that inhibits expression and/or activity of a protein product of the oncogene.

In other aspects, provided herein is a method of selecting a therapy for an individual having cancer. In some embodiments, the method comprises detecting a presence of a translocation comprising a first breakpoint in the IGH locus and a second breakpoint in a sample from the individual, wherein the second breakpoint is 1.3Mb or less from a transcription start site (TSS) of an oncogene, and wherein presence of the second breakpoint 1.3Mb or less from the TSS of the oncogene in the sample identifies the individual as one who may benefit from a targeted therapy that inhibits expression and/or activity of a protein product of the oncogene. In some embodiments, the method comprises detecting absence of a translocation comprising a breakpoint in the IGH locus in a sample from the individual, wherein the absence of the translocation identifies the individual as one who may benefit from a chemotherapy or non-targeted therapy.

In other aspects, provided herein is a method of identifying one or more treatment options for an individual having cancer. In some embodiments, the method comprises (a) acquiring knowledge of a presence of a translocation comprising a first breakpoint in the IGH locus and a second breakpoint in a sample from an individual, wherein the second breakpoint is 1.3Mb or less from a transcription start site (TSS) of an oncogene; and (b) generating a report comprising one or more treatment options identified for the individual based at least in part on said knowledge, wherein the one or more treatment options comprise a targeted therapy that inhibits expression and/or activity of a protein product of the oncogene. In some embodiments, the method comprises (a) detecting a presence of a translocation comprising a first breakpoint in the IGH locus and a second breakpoint, wherein the second breakpoint is 1.3Mb or less from a transcription start site (TSS) of an oncogene; and (b) generating a report comprising one or more treatment options identified for the individual based at least in part on the detection of the second breakpoint 1.3Mb or less from the TSS of the oncogene in the sample, wherein the one or more treatment options comprise a targeted therapy that inhibits expression and/or activity of a protein product of the oncogene. In some embodiments. the method comprises (a) acquiring knowledge of absence of a translocation comprising a first breakpoint in the IGH locus and a second breakpoint in a sample from an individual; and (b) generating a report comprising one or more treatment options identified for the individual based at least in part on said knowledge, wherein the one or more treatment options comprise a chemotherapy or non-targeted therapy.

In other aspects, provided herein is a method of selecting a therapy for an individual having cancer. In some embodiments, the method comprises detecting a presence of a translocation comprising a first breakpoint in the IGH locus and a second breakpoint in a sample from the individual, wherein the second breakpoint is 1.3Mb or less from a TSS of an oncogene, and wherein the detection of the second breakpoint 1.3Mb or less from the TSS of the oncogene in the sample identifies the individual as one who may benefit from a targeted therapy comprising an agent that inhibits expression and/or activity of a protein product of the oncogene.

In other aspects, provided herein is a method of treating or delaying progression of cancer. In some embodiments, the method comprises administering to an individual an effective amount of a targeted therapy that inhibits expression and/or activity of a protein product of an oncogene, wherein the cancer comprises a translocation comprising a first breakpoint in the IGH locus and a second breakpoint, and wherein the second breakpoint is 1.3Mb or less from a transcription start site (TSS) of the oncogene. In some embodiments, the method comprises, responsive to knowledge of a translocation comprising a first breakpoint in the IGH locus and a second breakpoint in a sample from an individual, wherein the second breakpoint is 1.3Mb or less from a transcription start site (TSS) of an oncogene (e.g., a targeted oncogene), administering to the individual an effective amount of a targeted therapy that inhibits expression and/or activity of a protein product of an oncogene. In some embodiments, the method comprises (a) detecting a presence of a translocation comprising a first breakpoint in the IGH locus and a second breakpoint in a sample from an individual, wherein the second breakpoint is 1.3Mb or less from a transcription start site (TSS) of an oncogene (e.g., a targeted oncogene); and (b) administering to the individual an effective amount of a targeted therapy that inhibits expression and/or activity of a protein product of the oncogene based at least in part on the detection of the second breakpoint 1.3Mb or less from the TSS of the oncogene in the sample. In some embodiments, the method comprises (a) acquiring knowledge of a translocation comprising a first breakpoint in the IGH locus and a second breakpoint in a sample from an individual, wherein the second breakpoint is 1.3Mb or less from a transcription start site (TSS) of an oncogene (e.g., a targeted oncogene); and (b) responsive to said knowledge, administering to the individual an effective amount of a targeted therapy that inhibits expression and/or activity of a protein product of the oncogene. In some embodiments, the method comprises responsive to knowledge of a translocation comprising a first breakpoint in the IGH locus and a second breakpoint in a sample from an individual, wherein the second breakpoint is 1.3Mb or less from a transcription start site (TSS) of an oncogene (e.g., a targeted oncogene), administering to the individual an effective amount of a targeted therapy that inhibits expression and/or activity of a protein product of an oncogene. In some embodiments, the method comprises (a) detecting a presence of a translocation comprising a first breakpoint in the IGH locus and a second breakpoint in a sample from an individual, wherein the second breakpoint is 1.3Mb or less from a transcription start site (TSS) of an oncogene (e.g., a targeted oncogene); and (b) administering to the individual an effective amount of a targeted therapy that inhibits expression and/or activity of a protein product of the oncogene. In some embodiments, the method comprises (a) acquiring knowledge of absence of a translocation comprising a first breakpoint in the IGH locus and a second breakpoint in a sample from an individual; and (b) responsive to said knowledge, administering to the individual an effective amount of a chemotherapy or non-targeted therapy. In some embodiments, the method comprises (a) detecting absence of a translocation comprising a first breakpoint in the IGH locus and a second breakpoint in a sample from an individual; and (b) administering to the individual an effective amount of a chemotherapy or non-targeted therapy. In some embodiments, the method comprises responsive to knowledge of absence of a translocation comprising a first breakpoint in the IGH locus and a second breakpoint in a sample from an individual, administering to the individual an effective amount of a chemotherapy or non-targeted therapy. In some embodiments, the method comprises (a) detecting presence or absence of a translocation comprising a first breakpoint in the IGH locus and a second breakpoint in a sample from an individual, wherein the second breakpoint is 1.3Mb or less from a transcription start site (TSS) of an oncogene (*e.g.,* a targeted oncogene); and (b) if the translocation is present, administering to the individual an effective amount of a targeted therapy that inhibits expression and/or activity of a protein product of the oncogene; or if the translocation is absent, administering to the individual an effective amount of a chemotherapy or non-targeted therapy.

In other aspects, provided herein is a method of selecting treatment for a subject having cancer. In some embodiments, the method comprises acquiring knowledge of a translocation comprising a first breakpoint in the IGH locus and a second breakpoint in a sample from an individual having cancer (*e.g.,* by sequencing, such as next-generation sequencing or sequencing by mass spectrometry), wherein the second breakpoint is 1.3Mb or less from a transcription start site (TSS) of an oncogene (*e.g.,* a targeted oncogene), and wherein responsive to the acquisition of said knowledge: (i) the subject is classified as a candidate to receive treatment with a targeted therapy that inhibits expression and/or activity of a protein product of the oncogene; (ii) the subject is identified as likely to respond to a treatment that comprises a targeted therapy that inhibits expression and/or activity of a protein product of the oncogene; (iii) the subject is classified as a candidate to receive treatment with one or more selected chemotherapies; and/or (iv) the subject is classified as not a candidate to receive treatment with a selected therapy. In some embodiments, the method further comprises providing a report to another party. In some embodiments, the report comprises information on the role of the translocation and/or oncogene, or wild-type sequence(s) thereof, in disease; information on prognosis, resistance, or potential or suggested therapeutic options for the subject (*e.g.,* one or more targeted and/or non-targeted therapies of the present disclosure); information on the likely effectiveness of a therapeutic option (*e.g.,* one or more targeted therapies of the present disclosure), the acceptability of a therapeutic option, or the advisability of applying a therapeutic option to the subject; or information, or a recommendation on, the administration of a drug (*e.g.,* one or more targeted therapies of the present disclosure) to the subject.

In some embodiments according to any of the embodiments described herein, the methods comprise administering a targeted therapy to the individual. In some embodiments, the targeted therapy comprises (a) an antibody that specifically binds a protein product of the oncogene, (b) a compound that inhibits an activity of a protein product of the oncogene and/or an activity of a signaling pathway of the oncogene, or (c) a nucleic acid that inhibits expression of a protein product of the oncogene. In some embodiments, the targeted therapy comprises an antibody that inhibits expression and/or activity of a protein product of the oncogene. In some embodiments, the targeted therapy comprises an antibody that inhibits expression and/or activity of a protein product of a component of an oncogenic signaling pathway in which the oncogene participates and/or interacts. In some embodiments, the targeted therapy comprises a compound that inhibits an enzymatic activity of the protein product of the oncogene and/or of the affected oncogenic signaling pathway. In some embodiments, the compound is a competitive inhibitor of the protein product. In some embodiments, the compound is a non-competitive inhibitor of the protein product. In some embodiments, the targeted therapy comprises an antisense nucleic acid, ribozyme, siRNA, shRNA, miRNA, gRNA, or triple helix nucleic acid that inhibits expression of a protein product of the oncogene. In some embodiments according to any of the embodiments described herein, the presence or absence of an IGH translocation is used to stratify a patient for appropriate chemotherapy or non-targeted therapy.

In some embodiments according to any of the embodiments described herein, the targeted therapy inhibits expression and/or activity of a protein product of, or activation of one or more signaling pathways stimulated by, CCND1, MYEOV, MYC, MAF, MAFB, BCL6, BCL2, FGFR3, WHSC1, BCL3, NBEAP1, BCL10, BCL11A, CCND2, CCND3, CCNE1, CD44, CDK6, CEBPA, CEBPB, CEBPD, CEBPE, CRLF2, ID4, DDX6, DUX4, EPOR, GPR34, FCRL4, FCGR2B, IRF4, IRF8, CD274 (encodes PD-L1), PDCD1LG2 (encodes PD-L2), LHX2, LHX4, PAX5, IGF2BP1, TNFSF13, MALT1, FOXP1, IL3, miR-125b-1, MUC1, SOX5, or MYCN. In some embodiments, the targeted therapy comprises a cyclin-dependent kinase (CDK) inhibitor, an inhibitor of expression and/or activity of a protein product of MYC, a MAF inhibitor, a BCL6 inhibitor, a BCL2 inhibitor, a BCL3 inhibitor, BCL8 inhibitor, a BCL10 inhibitor, a BCL11A inhibitor, an FGFR3 inhibitor, a pan-FGFR inhibitor, a WHSC1 inhibitor, an anti-CD44 antibody or antibody:drug conjugate (ADC), a chimeric antigen receptor (CAR) targeting CD44 and/or a T cell expressing a CAR targeting CD44, a CEBPB inhibitor, a CEBPD inhibitor, a DUX4 inhibitor, an EPOR inhibitor, an anti-FCGR2B antibody, a PD-1 antagonist, a PD-L1 antagonist, a PD-L2 antagonist, a PAX5 inhibitor, an IGF2BP1 inhibitor, a TNTSF13 inhibitor, a BCMA inhibitor, a MALT1 inhibitor, an anti-IL3 antibody, an anti-IL3R antibody, an anti-MUC1 antibody, a CAR targeting MUC1 and/or a T cell expressing a CAR targeting MUC1, a MUC1 antigenic peptide, a dendritic cell loaded with one or more MUC1 antigenic peptide(s), a virus or viral vector encoding one or more human MUC1 peptide(s) or polypeptide(s), a BET inhibitor, an alcohol dehydrogenase inhibitor, or a MYCN inhibitor. In some embodiments, the methods further comprise administering a second therapeutic agent to the individual. In some embodiments, the second therapeutic agent is selected from the group consisting of a chemotherapeutic agent, an anti-hormonal agent, an antimetabolite chemotherapeutic agent, a kinase inhibitor, a methyltransferase inhibitor, a peptide, a gene therapy, a vaccine, a platinum-based chemotherapeutic agent, an immunotherapy, an antibody, and a checkpoint inhibitor.

In some embodiments according to any of the embodiments described herein, the oncogene is CCND1, MYEOV, MYC, MAF, MAFB, BCL6, BCL2, FGFR3, WHSC1 (also known as MMSET and NSD2), BCL3, NBEAP1 (encodes BCL8), BCL10, BCL11A, CCND2, CCND3, CCNE1, CD44, CDK6, CEBPA, CEBPB, CEBPD, CEBPE, CRLF2, ID4, DDX6, DUX4, EPOR, GPR34, FCRL4, FCGR2B, IRF4, IRF8, CD274 (encodes PD-L1), PDCD1LG2 (encodes PD-L2), LHX2, LHX4, PAX5, IGF2BP1, TNFSF13, MALT1, FOXP1, IL3, miR-125b-1, MUC1, SOX5 or MYCN.

In some embodiments according to any of the embodiments described herein, the cancer is multiple myeloma (MM), B-cell lymphoma, diffuse large B-cell lymphoma (DLBCL), B-cell lymphoma unclassified, acute myeloid leukemia (AML), follicular lymphoma (FL), Burkitt lymphoma, acute lymphoblastic leukemia (ALL), B-cell acute lymphoblastic leukemia (B-ALL), non-Hodgkin's lymphoma (NHL), B-cell non-Hodgkin's lymphoma (B-NHL), Waldenstroms lymphoma, extranodal lymphoma, mantle cell lymphoma (MCL), mature B cell neoplasm, hairy cell leukemia (HCL), hairy cell leukemia variant (HCL-v), splenic marginal zone lymphoma (SMZL), nodal marginal zone lymphoma, extranodal marginal zone lymphoma, MALT lymphoma, myeloproliferative neoplasm (MPN), skin lymphoma, lymphoproliferative disease, primary mediastinal large B-cell lymphoma (PMBCL), plasmablastic lymphoma, chronic lymphocytic leukemia (CLL), small cell lymphoma (SLL), Hodgkin's lymphoma (HL), bone marrow lymph proliferative disease, myelodysplastic syndrome, or angioimmunoblastic T-cell lymphoma (AITL).

In some embodiments, the oncogene is MYC; the targeted therapy comprises a MYC inhibitor; and the cancer is DLBCL, B-cell lymphoma, Burkitt lymphoma, multiple myeloma, ALL, B-ALL, NHL, MCL, mature B cell neoplasm, MPN, skin lymphoma, CLL, or lymphoproliferative disease. In some embodiments, the oncogene is BCL2; the targeted therapy comprises a BCL2 inhibitor; and the cancer is DLBCL, FL, B-cell lymphoma, ALL, CLL, HL, NHL, bone marrow lymph proliferative disease, or lymphoproliferative disease. In some embodiments, the oncogene is BCL6; the targeted therapy comprises a BCL6 inhibitor; and the cancer is DLBCL, FL, multiple myeloma, SMZL, NHL, PMBCL, or plasmablastic lymphoma. In some embodiments, the oncogene is CCND1; the targeted therapy comprises a CDK inhibitor; and the cancer is multiple myeloma, B-cell lymphoma, AML, DLBCL, or FL. In some embodiments, the oncogene is MAF; the targeted therapy comprises a MAF inhibitor; and the cancer is multiple myeloma, CLL, ALL, AITL, or DLBCL. In some embodiments, the oncogene is MAFB; the targeted therapy comprises a MAFB inhibitor; and the cancer is multiple myeloma, ALL, B-ALL, or DLBCL. In some embodiments, the oncogene is WHSC1 or FGFR3; the targeted therapy comprises a WHSC 1 inhibitor, an FGFR3 inhibitor, or a pan-FGFR inhibitor; and the cancer is multiple myeloma or DLBCL.

In some embodiments according to any of the embodiments described herein, the IGH translocation results in at least a 2-fold, 3-fold, 5-fold, 7-fold, 10-fold, 15-fold, 20-fold, 25-fold, or 50-fold increase in expression of the oncogene, as compared to a reference. In some embodiments, expression of the oncogene is expression of oncogene RNA. In some embodiments, expression of the oncogene is measured by next-generation sequencing (NGS), real-time or quantitative PCR, microarray analysis, comparative genomic hybridization, or in situ hybridization. In some embodiments, expression of the oncogene is measured by fluorescence *in situ* hybridization (FISH). In some embodiments, expression of the oncogene is expression of oncogene polypeptide. In some embodiments, expression of the oncogene is measured by Western blotting, ELISA, or mass spectrometry. In some embodiments, the IGH translocation comprises translocation of one or more enhancers of the IGH locus with a different region of the genome. In some embodiments, the IGH translocation leads to IGH enhancers being juxtaposed 1.0Mb or less, 500kb or less, 400kb or less, 300kb or less, 200kb or less, 100kb or less, 90kb or less, 80kb or less, 70kb or less, 60kb or less, 50kb or less, 40kb or less, 30kb or less, 20kb or less, 10kb or less, 5kb or less, or 1kb or less from the TSS of the oncogene.

In some embodiments according to any of the embodiments described herein, the IGH translocation is detected in DNA from the sample. In some embodiments, the IGH translocation is detected in the sample by one or more methods selected from the group consisting of next-generation sequencing (NGS), a nucleic acid hybridization assay, an amplification-based assay, a PCR-RFLP assay, real-time or quantitative PCR, sequencing, a screening analysis, FISH, spectral karyotyping or MFISH, comparative genomic hybridization, in situ hybridization, sequence-specific priming (SSP) PCR, HPLC, and mass-spectrometric genotyping.

In some embodiments according to any of the embodiments described herein, the sample is a nucleic acid sample or comprises nucleic acids. In some embodiments, the nucleic acid sample comprises cell-free DNA (cfDNA) or circulating tumor DNA (ctDNA). In some embodiments, the sample comprises fluid, protein, cells, or tissue. In some embodiments, the sample is from a tumor biopsy or tumor specimen. In some embodiments, the sample comprises a circulating tumor cell. In some embodiments, the IGH translocation comprises translocation of one or more enhancers of the IGH locus. In some embodiments, the individual is a human. In some embodiments, the individual has been diagnosed with cancer.

In other aspects, provided herein is a method of identifying an individual having cancer who may benefit from a treatment comprising a chemotherapy or non-targeted therapy. In some embodiments, the method comprises detecting absence of a translocation comprising a first breakpoint in the IGH locus and a second breakpoint in a sample from the individual, wherein absence of a breakpoint in the IGH locus in the sample identifies the individual as one who may benefit from a chemotherapy or non-targeted therapy.

In other aspects, provided herein is a method of selecting a therapy for an individual having cancer. In some embodiments, the method comprises detecting absence of a translocation comprising a first breakpoint in the IGH locus and a second breakpoint in a sample from the individual, wherein the detection of absence of the breakpoint in the IGH locus in the sample identifies the individual as one who may benefit from a chemotherapy or non-targeted therapy.

In other aspects, provided herein is a method of identifying one or more treatment options for an individual having cancer. In some embodiments, the method comprises (a) acquiring knowledge of absence of a translocation comprising a first breakpoint in the IGH locus and a second breakpoint in a sample from an individual; and (b) generating a report comprising one or more treatment options identified for the individual based at least in part on said knowledge, wherein the one or more treatment options comprise a chemotherapy or non-targeted therapy.

In other aspects, provided herein is a method of identifying one or more treatment options for an individual having cancer. In some embodiments, the method comprises (a) detecting absence of a translocation comprising a first breakpoint in the IGH locus and a second breakpoint in a sample from the individual; and (b) generating a report comprising one or more treatment options identified for the individual based at least in part on the detection of absence of a breakpoint in the IGH locus in the sample, wherein the one or more treatment options comprise a chemotherapy or non-targeted therapy.

In other aspects, provided herein is a method of treating or delaying progression of cancer. In some embodiments, the method comprises, responsive to knowledge of an absence of a translocation comprising a first breakpoint in the IGH locus and a second breakpoint in a sample from an individual, administering to the individual an effective amount of a chemotherapy or non-targeted therapy.

In other aspects, provided herein is a method of treating or delaying progression of cancer. In some embodiments, the method comprises (a) detecting absence of a translocation comprising a first breakpoint in the IGH locus and a second breakpoint in a sample from an individual; and (b) administering to the individual an effective amount of a chemotherapy or non-targeted therapy based at least in part on the detection of the absence of a breakpoint in the IGH locus in the sample.

In other aspects, provided herein is a method of treating or delaying progression of cancer. In some embodiments, the method comprises (a) acquiring knowledge of an absence of a translocation comprising a first breakpoint in the IGH locus and a second breakpoint in a sample from an individual; and (b) responsive to said knowledge, administering to the individual an effective amount of a chemotherapy or non-targeted therapy.

In other aspects, provided herein is a method of selecting treatment for a subject having cancer. In some embodiments, the method comprises acquiring knowledge of an absence of a translocation comprising a first breakpoint in the IGH locus and a second breakpoint in a sample from an individual having cancer, wherein responsive to the acquisition of said knowledge: (i) the subject is classified as a candidate to receive treatment with a chemotherapy or non-targeted therapy; (ii) the subject is identified as likely to respond to a chemotherapy or non-targeted therapy; (iii) the subject is classified as a candidate to receive treatment with one or more selected chemotherapies; and/or (iv) the subject is classified as not a candidate to receive treatment with a selected therapy.

In other aspects, provided herein is a kit or article of manufacture comprising one or more oligonucleotides for detecting a presence of a translocation comprising a first breakpoint in the IGH locus and a second breakpoint and instructions for administration of a targeted therapy comprising an agent that inhibits expression and/or activity of a protein product of an oncogene, based at least in part on the detection of the second breakpoint 1.3Mb or less from a transcription start site (TSS) of the oncogene.

In some embodiments, provided herein is a targeted therapy that inhibits expression and/or activity of a protein product of an oncogene for use in any of the methods disclosed herein.

In some embodiments, provided herein is a targeted therapy that inhibits expression and/or activity of a protein product of an oncogene and/or an activity of a signaling pathway of the oncogene for use in a method of treating or delaying progression of cancer, wherein the method comprises administering the agent to an individual, and wherein a translocation comprising a first breakpoint in the IGH locus and a second breakpoint that is 1.3Mb or less from a transcription start site (TSS) of the oncogene has been detected in a sample obtained from the individual.

In some embodiments, provided herein is a targeted therapy that inhibits expression and/or activity of a protein product of an oncogene and/or an activity of a signaling pathway of the oncogene for use in the manufacture of a medicament for treating or delaying progression of cancer, wherein the medicament is to be administered to an individual, wherein a translocation comprising a first breakpoint in the IGH locus and a second breakpoint that is 1.3Mb or less from a transcription start site (TSS) of the oncogene has been detected in a sample obtained from the individual.

It is to be understood that one, some, or all of the properties of the various embodiments described herein may be combined to form other embodiments of the present invention. These and other aspects of the invention will become apparent to one of skill in the art. These and other embodiments of the invention are further described by the detailed description that follows

BRIEF DESCRIPTION OF THE DRAWINGS

**FIG. 1A** shows the distance distribution of IGH translocation breakpoints. The distribution of IGH translocation breakpoint distance from TSS (defined as the distance between IGH breakpoint and transcriptional start site, TSS, of targeted oncogene) (Y-axis) of each IGH translocation (X-axis) were plotted in the dot plot. Each dot represents one patient sample.

**FIG. 1B** shows the distribution of distances between IGH translocation breakpoints and TSS's. Percentages of samples in each distance range are provided, along with total numbers (out of 1000).

**FIG. 1C** shows the distribution of IGH-BCL2 breakpoints. Each triangle represents one breakpoint.

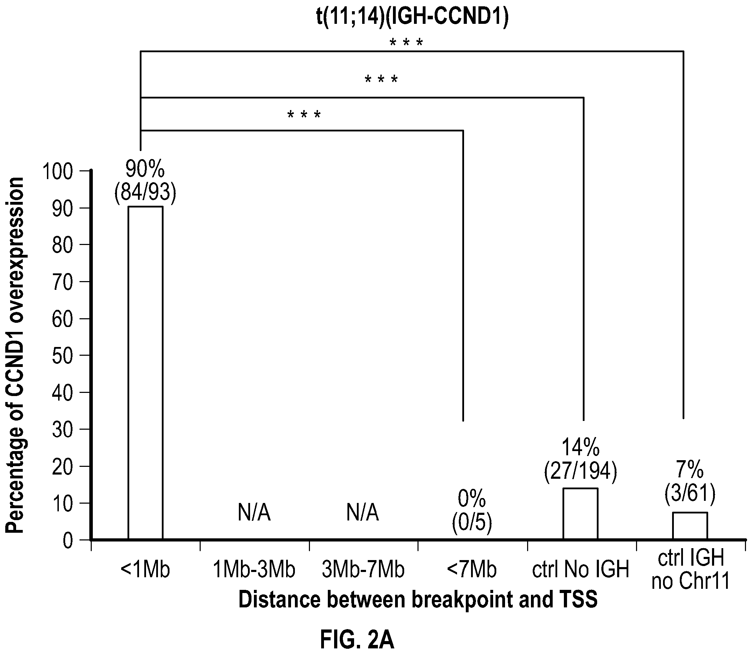

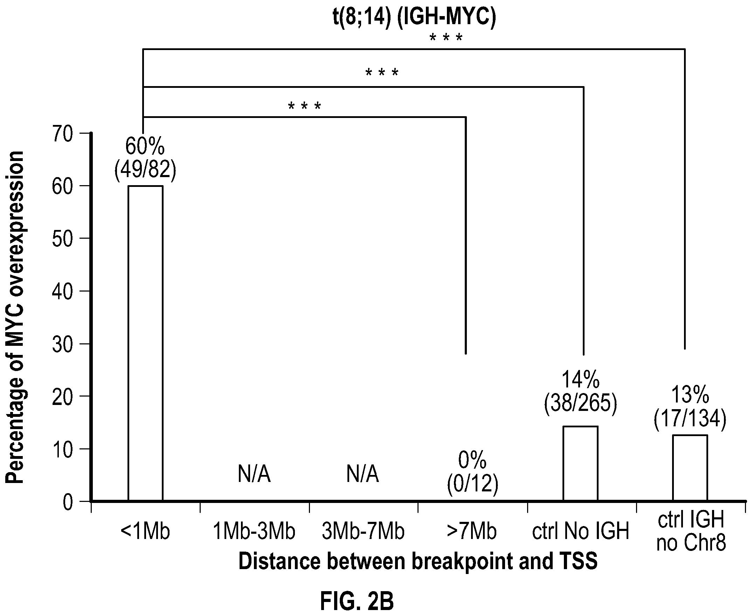

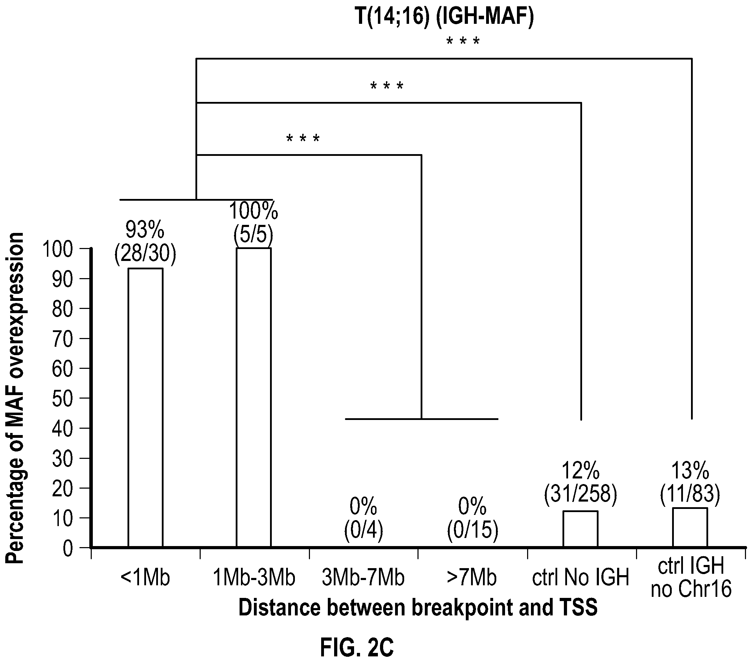

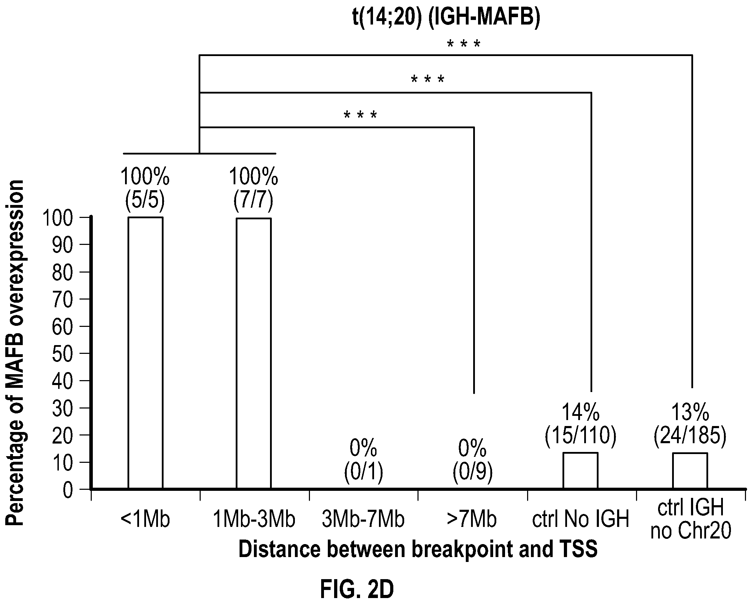

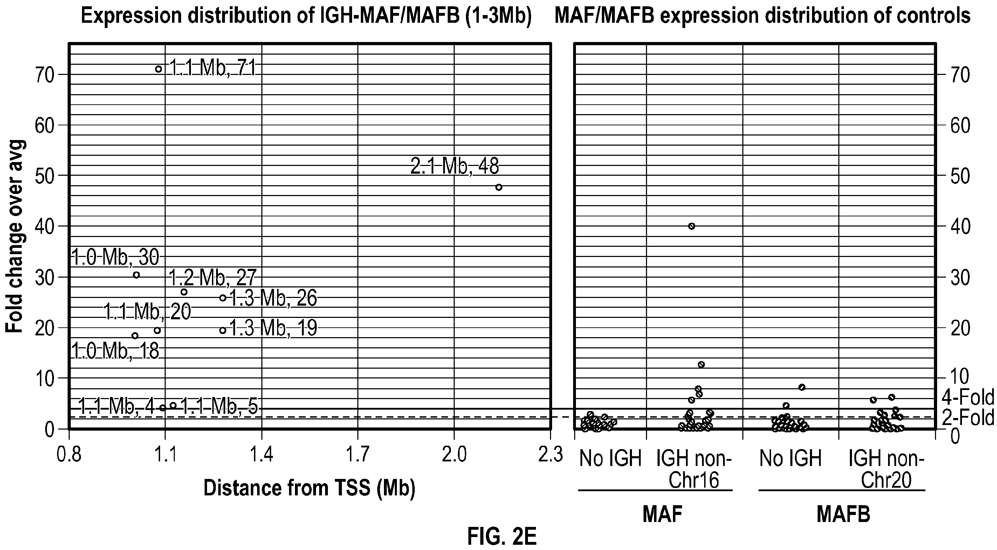

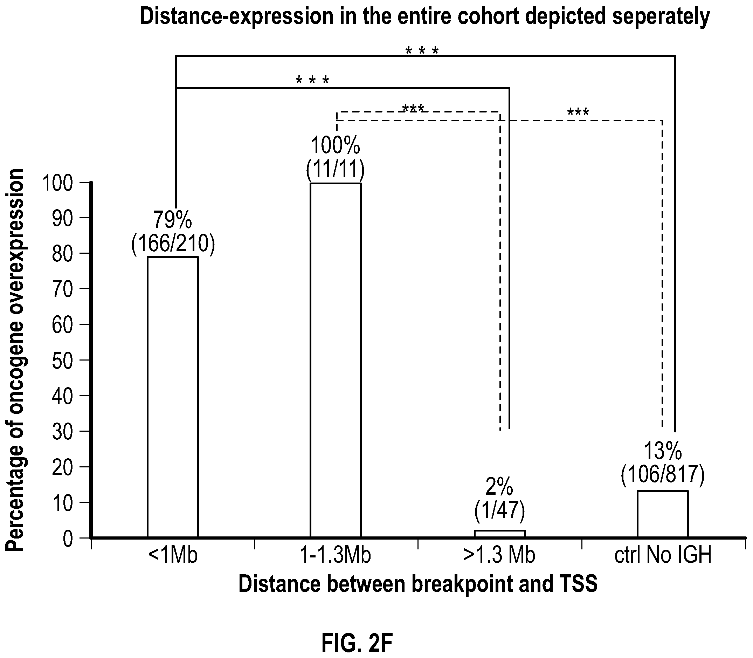

**FIGS. 2A-2F** show a correlation between oncogene overexpression and IGH translocation breakpoint distance from TSS (defined as the distance between IGH breakpoint and TSS of targeted oncogene). **FIGS. 2A-2D** show percentage of overexpression (greater than 2-fold normalized expression over control) of oncogenes **(FIG. 2A:** *CCND1,* **FIG. 2B:** *MYC,* **FIG. 2C:** *MAF,* **FIG. 2D:** *MAFB)* compared by IGH translocation breakpoint distance from corresponding TSS. The distances are organized into four bins (<1 Mb, 1-3 Mb, 3-7 Mb, and >7 Mb). Controls are cases without IGH translocations, and cases where IGH translocations involved a chromosome other than the one where the target oncogene is located. Percentage and number of cases are labeled above each bar. **FIG. 2E:** Left panel: Correlation between expression levels (fold change over normalized control average, Y-axis) of *MAF* and *MAFB* and IGH translocation breakpoint distance (X-axis) in the range between 1.0Mb - 2.2Mb. Distance and expression fold change are labeled above each dot. Right panel: Distribution of expression fold change of *MAF* and *MFAB* in control samples. Both panels: Solid line denotes 4-fold change and dotted line denotes 2-fold change. **FIG. 2F** shows data from **FIGS. 2A-2D** combined (*i.e.,* distance-expression relationship in the entire cohort depicted separately in **FIGS. 2A-2D).** Percentage and number of cases are labeled above each bar. ***, $p < 0.001$.

**FIG. 3A** shows the normalized RNA expression levels of *CCND1, MYC, MAF* and *MAFB* within the 0-1.3 Mb range from the IGH breakpoint to TSS of targeted oncogene. Dot plot shows expression level fold change (Y-axis) of *CCND1, MYC, MAF* and *MAFB* in cases harboring IGH-translocation with breakpoint distance within 0-1.3 Mb range from TSS's of corresponding oncogenes (filled symbols) or controls without IGH-translocation (open symbols). Each dot represents one sample. Dotted line indicates 2-fold change of expression level over average.

**FIG. 3B** shows fold normalized RNA overexpression vs. distance between IGH translocation breakpoints and TSS for cases with breaks within 0-1.3 Mb range from TSS's of corresponding oncogenes. Scatter plots with trend lines illustrate the relationship between normalized RNA overexpression levels and distance between IGH translocation breakpoints and TSS for *CCND1, MYC, MAF* and *MAFB.* Each point represents one sample.

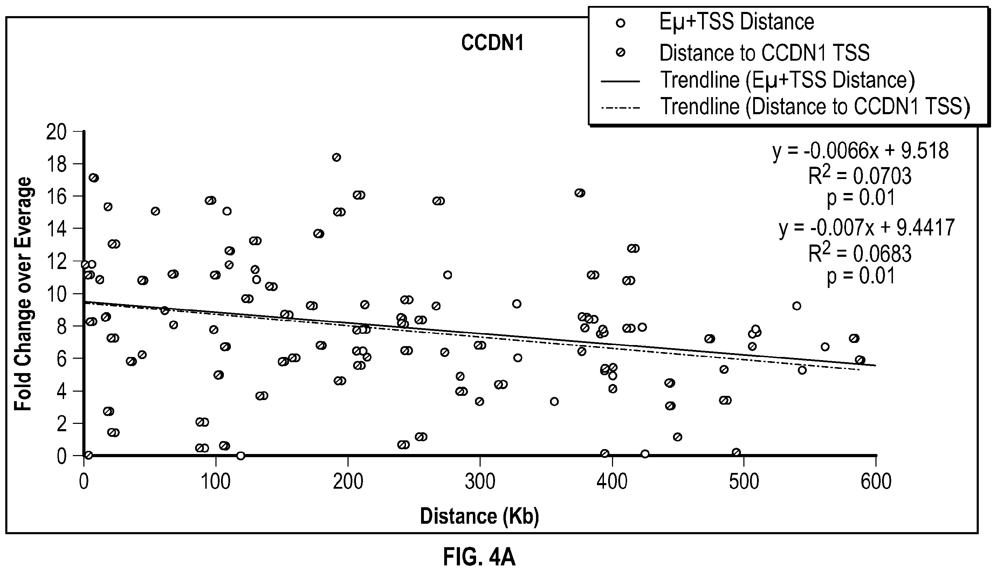

**FIG. 4A** shows a comparison between $E\mu$+TSS distance (distance between breakpoint within the IGH locus to $E\mu$ plus distance between breakpoint on chromosome 11 to *CCND1* TSS) (darker) or distance to *CCND1* TSS (lighter). P value between the 2 sets of distances = 0.25. Scatter plot with trendlines illustrates the relationship between normalized *CCND1* RNA overexpression levels and either $E\mu$+TSS distance (distance between breakpoint within the IGH locus to $E\mu$ plus distance between breakpoint on chromosome 11 to *CCND1* TSS; darker) or distance between breakpoints to *CCND1* TSS (lighter).

**FIG. 4B** shows the correlation between $E\mu$+TSS distance and overexpression levels of *CCND1.*

**FIG. 4C** shows the distribution of IGH translocation breakpoints at the IGH locus. Shown is the distribution of IGH translocation breakpoints of MM (blue) or lymphomas (red) at the IGH locus grouped by IGH-CCND1, IGH-MYC, IGH-MAF, IGH-MAFB, or combined. Each dot represents one sample. P values of each pair of comparison were displayed on the left side.

**FIG. 4D** shows IGH breakpoint distance by group. Shown are box plots of IGH translocation breakpoints of MM or lymphomas (Lym) at the IGH locus grouped by IGH-CCND1, IGH-MYC, IGH-MAF, IGH-MAFB, or combined.

**FIG. 5A** shows oncogene expression vs. tumor purity.

**FIG. 5B** shows the correlation between IGH translocation breakpoint distance and overexpression levels of *CCND1* after normalization by tumor purity.

**FIG. 5C** shows a scatter plot with trendline to illustrate the relationship between *CCND1* RNA overexpression fold change normalized by tumor purity and $E\mu$+TSS distance.

DETAILED DESCRIPTION

The present disclosure relates generally to detecting overexpression of an oncogene through translocations in the immunoglobulin heavy (IGH) locus. The present disclosure demonstrates that oncogene overexpression is highly dependent on the distance between a breakpoint in the IGH locus and a second breakpoint near a targeted oncogene TSS. As demonstrated herein, the majority of IGH breakpoints that were studied localized within 1 megabase (Mb) of the transcription start sites (TSS's) of the targeted oncogenes, but in 6.9% of cases such distance was greater than 1 Mb. IGH-driven oncogene overexpression was highly dependent on the distance from the IGH breakpoint to targeted oncogene TSS, observed in 79% (166/210) of the cases with the distance of less than 1 Mb, 100% (11/11) of cases with the distance 1-1.3 Mb, and in 2.1% (1/47) of cases with the distance larger than 1.3 Mb, compared to 13% (106/817) of control cases. Location of the breakpoints within the IGH locus did not influence oncogene overexpression, suggesting that the distance from the IGH breakpoint to the targeted oncogene TSS's is the major determinant of IGH-medicated oncogene overexpression. Remarkably, oncogenes targeted by IGH translocations were overexpressed to a similar extent when breakpoint distances were less than 1.3 Mb.

Without wishing to be bound to theory, it is thought that detecting translocations involving a breakpoint in the IGH locus and a breakpoint 13Mb or less from an oncogene TSS may find use, *e.g..* in detecting oncogene overexpression, providing assessment/diagnosis, identifying individuals for treatment, selecting therapies, identifying treatment options, and treating or delaying progression of cancer, *e.g.,* using a targeted approach.

## I. General Techniques

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies. A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

## II. Definitions

As used in this specification and the appended claims, the singular forms "a", "an" and "the" include plural referents unless the content clearly dictates otherwise. Thus, for example, reference to "a molecule" optionally includes a combination of two or more such molecules, and the like.

The term "about" as used herein refers to the usual error range for the respective value readily known to the skilled person in this technical field. Reference to "about" a value or parameter herein includes (and describes) embodiments that are directed to that value or parameter per se.

It is understood that aspects and embodiments of the invention described herein include "comprising," "consisting," and "consisting essentially of" aspects and embodiments.

The terms "cancer" and "cancerous" refer to or describe the physiological condition in mammals that is typically characterized by unregulated cell growth. Included in this definition are benign and malignant cancers. By "early stage cancer" or "early stage **tumor**" is meant a cancer that is not invasive or metastatic or is classified as a Stage 0, 1 , or 2 cancer. Examples of a cancer include, but are not limited to, a lung cancer (e.g., a non-small cell lung cancer (NSCLC)), a kidney cancer (e.g., a kidney urothelial carcinoma), a bladder cancer (e.g., a bladder urothelial (transitional cell) carcinoma), a breast cancer, a colorectal cancer (e.g., a colon adenocarcinoma), an ovarian cancer, a pancreatic cancer, a gastric carcinoma, an esophageal cancer, a mesothelioma, a melanoma (e.g., a skin melanoma), a head and neck cancer (e.g., a head and neck squamous cell carcinoma (HNSCC)), a thyroid cancer, a sarcoma (e.g., a soft-tissue sarcoma, a fibrosarcoma, a myxosarcoma, a liposarcoma, an osteogenic sarcoma, an osteosarcoma, a chondrosarcoma, an angiosarcoma, an endotheliosarcoma, a lymphangiosarcoma, a lymphangioendotheliosarcoma, a leiomyosarcoma, or a rhabdomyosarcoma), a prostate cancer, a glioblastoma, a cervical cancer, a thymic carcinoma, a leukemia (e.g., an acute lymphocytic leukemia (ALL), an acute myelocytic leukemia (AML), a chronic myelocytic leukemia (CML), a chronic eosinophilic leukemia, or a chronic lymphocytic leukemia (CLL)), a lymphoma (e.g., a Hodgkin lymphoma or a non-Hodgkin lymphoma (NHL)), a myeloma (e.g., a multiple myeloma (MM)), a mycoses fungoides, a merkel cell cancer, a hematologic malignancy, a cancer of hematological tissues, a B cell cancer, a bronchus cancer, a stomach cancer, a brain or central nervous system cancer, a peripheral nervous system cancer, a uterine or endometrial cancer, a cancer of the oral cavity or pharynx, a liver cancer, a testicular cancer, a biliary tract cancer, a small bowel or appendix cancer, a salivary gland cancer, an adrenal gland cancer, an adenocarcinoma, an inflammatory myofibroblastic tumor, a gastrointestinal stromal tumor (GIST), a colon cancer, a myelodysplastic syndrome (MDS), a myeloproliferative disorder (MPD), a polycythemia Vera, a chordoma, a synovioma, an Ewing's tumor, a squamous cell carcinoma, a basal cell carcinoma, an adenocarcinoma, a sweat gland carcinoma, a sebaceous gland carcinoma, a papillary carcinoma, a papillary adenocarcinoma, a medullary carcinoma. a bronchogenic carcinoma, a renal cell carcinoma, a hepatoma, a bile duct carcinoma, a choriocarcinoma, a seminoma, an embryonal carcinoma, a Wilms' tumor, a bladder carcinoma, an epithelial carcinoma, a glioma, an astrocytoma, a medulloblastoma, a craniopharyngioma, an ependymoma, a pinealoma, a hemangioblastoma, an acoustic neuroma, an oligodendroglioma, a meningioma, a neuroblastoma, a retinoblastoma, a follicular lymphoma, a diffuse large B-cell lymphoma, a mantle cell lymphoma, a hepatocellular carcinoma, a thyroid cancer, a small cell cancer, an essential thrombocythemia, an agnogenic myeloid metaplasia, a hypereosinophilic syndrome, a systemic mastocytosis, a familiar hypereosinophilia, a neuroendocrine cancer, or a carcinoid tumor.

The term "tumor," as used herein, refers to all neoplastic cell growth and proliferation, whether malignant or benign, and all pre-cancerous and cancerous cells and tissues. The terms "cancer," "cancerous," and "tumor" are not mutually exclusive as referred to herein.

As used herein, the term "immunoglobulin heavy (*IGH*)" refers to a gene encoding an IGH mRNA or polypeptide, or a genetic locus thereof, as well as translocations or rearrangements thereof. *IGH* encodes the immunoglobulin heavy chain, including variable (V), diversity (D), joining (J), and constant (C) segments. IGH is also known as IGD1, IGH@, IGHJ, IGHV, IGHD@, IGHJ@, IGHV@, IGH.1@, and IGHDY1. In some embodiments, an *IGH* gene is a human *IGH* gene. An exemplary *IGH* gene is represented by NCBI Gene ID No. 3492

"Polynucleotide." or "nucleic acid." as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide The term "polynucleotide" specifically includes cDNAs.

A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps," substitution of one or more of the naturally-occurring nucleotides with an analog, internucleotide modifications such as, for example, those with uncharged linkages (e.g., methyl phosphonates, phosphotriesters, phosphoamidates, carbamates, and the like) and with charged linkages (e.g., phosphorothioates, phosphorodithioates, and the like), those containing pendant moieties, such as, for example, proteins (e.g., nucleases, toxins, antibodies, signal peptides, poly-L-lysine, and the like), those with intercalators (e.g., acridine, psoralen, and the like), those containing chelators (e.g., metals, radioactive metals, boron, oxidative metals, and the like), those containing alkylators, those with modified linkages (e.g., alpha anomeric nucleic acids), as well as unmodified forms of the polynucleotide(s) Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-0-methyl-, 2'-0-allyl-, 2'-fluoro-, or 2'-azido-ribose, carbocyclic sugar analogs, a-anomeric sugars, epimeric sugars such as arabinose, xyloses or lyxoses, pyranose sugars, furanose sugars, sedoheptuloses, acyclic analogs, and abasic nucleoside analogs such as methyl riboside. One or more phosphodiester linkages may be replaced by alternative linking groups. These alternative linking groups include, but are not limited to, embodiments wherein phosphate is replaced by P(0)S ("thioate"), P(S)S ("dithioate"), "(0)NR$_2$ ("amidate"), P(0)R, P(0)OR'. CO or CH$_2$ ("formacetal"), in which each R or R' is independently H or substituted or unsubstituted alkyl (1 -20 C) optionally containing an ether (-0-) linkage, aryl, alkenyl, cycloalkyl, cycloalkenyl or araldyl. Not all linkages in a polynucleotide need be identical. A polynucleotide can contain one or more different types of modifications as described herein and/or multiple modifications of the same type. The preceding description applies to all polynucleotides referred to herein, including RNA and DNA.

"Oligonucleotide," as used herein, generally refers to short, single stranded, polynucleotides that are, but not necessarily, less than about 250 nucleotides in length. Oligonucleotides may be synthetic. The terms "oligonucleotide" and "polynucleotide" are not mutually exclusive. The description above for polynucleotides is equally and fully applicable to oligonucleotides.

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "isolated" antibody is one which has been identified and separated and/or recovered from a component of its natural environment. Contaminant components of its natural environment are materials which would interfere with research, diagnostic, and/or therapeutic uses for the antibody, and may include enzymes, hormones, and other proteinaceous or nonproteinaceous solutes. In some embodiments, an antibody is purified (1) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of, for example, a spinning cup sequenator, or (3) to homogeneity by SDS-PAGE under reducing or nonreducing conditions using, for example, Coomassie blue or silver stain. An isolated antibody includes the antibody in situ within recombinant cells since at least one component of the antibody's natural environment will not be present. Ordinarily, however, an isolated antibody will be prepared by at least one purification step

"Native antibodies" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

The "light chains" of antibodies (immunoglobulins) from any mammalian species can be assigned to one of two clearly distinct types, called kappa ("x") and lambda ("λ"), based on the amino acid sequences of their constant domains.

The term "constant domain" refers to the portion of an immunoglobulin molecule having a more conserved amino acid sequence relative to the other portion of the immunoglobulin, the variable domain, which contains the antigen binding site. The constant domain contains the CH1, CH2, and CH3 domains (collectively, CH) of the heavy chain and the CHL (or CL) domain of the light chain.

The "variable region" or "variable domain" of an antibody refers to the amino-terminal domains of the heavy or light chain of the antibody. The variable domain of the heavy chain may be referred to as "VH." The variable domain of the light chain may be referred to as "VL." These domains are generally the most variable parts of an antibody and contain the antigen-binding sites.

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions (HVRs) both in the light chain and the heavy chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen-binding site of antibodies (see Kabat et al., Sequences of Proteins of Immunological Interest, Fifth Edition, National Institute of Health, Bethesda, Md (1991)). The constant domains are not involved directly in the binding of an antibody to an antigen, but exhibit various effector functions, such as participation of the antibody in antibody-dependent cellular toxicity.

The term "hypervariable region," "HVR," or "HV," as used herein, refers to the regions of an antibody variable domain which are hypervariable in sequence and/or form structurally defined loops. Generally, antibodies comprise six HVRs; three in the VH (H1, H2, H3), and three in the VL (L1, L2, L3). In native antibodies. H3 and L3 display the most diversity of the six HVRs, and H3 in particular is believed to play a unique role in conferring fine specificity to antibodies. See, for example, Xu et al., Immunity 13:37-45 (2000); Johnson and Wu, in Methods in Molecular Biology 248.1-25 (Lo, ed., Human Press, Totowa, N.J., 2003). Indeed, naturally occurring camelid antibodies consisting of a heavy chain only are functional and stable in the absence of light chain. See, for example, Hamers-Casterman et al., Nature 363:446-448 (1993); Sheriff et al., Nature Struct. Biol. 3:733-736 (1996).

A number of HVR delineations are in use and are encompassed herein. The Kabat Complementarity Determining Regions (CDRs) are based on sequence variability and are the most commonly used (Kabat et al., Sequences of Proteins of Immunological Interest, 5th Ed. Public Health Service, National Institutes of Health, Bethesda, Md. (1991)). Chothia

refers instead to the location of the structural loops (Chothia and Lesk J. Mol. Biol. 196:901 -917 (1987)). The AbM HVRs represent a compromise between the Kabat HVRs and Chothia structural loops. and are used by Oxford Molecular's AbM antibody modeling software. The "contact" HVRs are based on an analysis of the available complex crystal structures. The residues from each of these HVRs are noted below.

| Loop | Kabat | AbM | Chothia | Contact |
|------|-------|-----|---------|---------|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat numbering) |
| H1 | H31-H35 | H26-H35 | H26-H32 | H30-H35 (Chothia numbering) |
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (L1), 46-56 or 50-56 (L2) and 89-97 or 89-96 (L3) in the VL and 26-35 (H1), 50-65 or 49-65 (H2) and 93-102, 94-102, or 95-102 (H3) in the VH. The variable domain residues are numbered according to Kabat et al., supra, for each of these definitions.

"Framework" or "FR" residues are those variable domain residues other than the HVR residues as herein defined.

The term "variable domain residue numbering as in Kabat" or "amino acid position numbering as in Kabat," and variations thereof, refers to the numbering system used for heavy chain variable domains or light chain variable domains of the compilation of antibodies in Kabat et al., supra. Using this numbering system, the actual linear amino acid sequence may contain fewer or additional amino acids corresponding to a shortening of, or insertion into, a FR or HVR of the variable domain. For example, a heavy chain variable domain may include a single amino acid insert (residue 52a according to Kabat) after residue 52 of H2 and inserted residues (e.g., residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

The Kabat numbering system is generally used when referring to a residue in the variable domain (approximately residues 1 -107 of the light chain and residues 1 -1 13 of the heavy chain) (e.g., Kabat et al., Sequences of Immunological Interest. 5th Ed. Public Health Service, National Institutes of Health, Bethesda, Md. (1991 )). The "EU numbering system" or "EU index" is generally used when referring to a residue in an immunoglobulin heavy chain constant region (e.g., the EU index reported in Kabat et al., supra). The "EU index as in Kabat" refers to the residue numbering of the human IgG1 EU antibody

The terms "full-length antibody," "intact antibody," and "whole antibody" are used herein interchangeably to refer to an antibody in its substantially intact form, not antibody fragments as defined below. The terms particularly refer to an antibody with heavy chains that contain an Fc region.

"Antibody fragments" comprise a portion of an intact antibody, preferably comprising the antigen-binding region thereof. In some embodiments, the antibody fragment described herein is an antigen-binding fragment. Examples of antibody fragments include Fab, Fab', F(ab')2, and Fv fragments; diabodies; linear antibodies; single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, whose name reflects its ability to crystallize readily. Pepsin treatment yields an F(ab')2 fragment that has two antigen-combining sites and is still capable of cross-linking antigen. "Fv" is the minimum antibody fragment which contains a complete antigen-binding site In one embodiment, a two-chain Fv species consists of a dimer of one heavy- and one light-chain variable domain in tight, non-covalent association. In a single-chain Fv (scFv) species, one heavy- and one light-chain variable domain can be covalently linked by a flexible peptide linker such that the light and heavy chains can associate in a "dimeric" structure analogous to that in a two-chain Fv species. It is in this configuration that the three HVRs of each variable domain interact to define an antigen-binding site on the surface of the VH-VL dimer. Collectively, the six HVRs confer antigen-binding specificity to the antibody However, even a single variable domain (or half of an Fv comprising only three HVRs specific for an antigen) has the ability to recognize and bind antigen, although at a lower affinity than the entire binding site. The Fab fragment contains the heavy- and light-chain variable domains and also contains the constant domain of the light chain and the first constant domain (CH1) of the heavy chain Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')2 antibody fragments originally were produced as pairs of Fab' fragments which have hinge cysteines between them. Other chemical couplings of antibody fragments are also known.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains which enables the scFv to form the desired structure for antigen binding. For a review of scFv, see, e.g., Pluckthun, in The Pharmacology of Monoclonal Antibodies, vol. 1 13, Rosenburg and Moore eds., (Springer-Verlag, New York, 1994), pp. 269-315.

The term "diabodies" refers to antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains on the same chain, the domains are forced to pair with the complementary domains of another chain and create two antigen-binding sites Diabodies may be bivalent or bispecific. Diabodies are described more fully in, for example, EP 404,097, WO 1993/01 161 ; Hudson et al., Nat. Med. 9:129-134 (2003); and Hollinger et al., Proc. Natl. Acad. Sci. USA 90. 6444-6448 (1993) Triabodies and tetrabodies are also described in Hudson et al., Nat. Med 9:129-134 (2003).

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1 , IgG2, IgG3, IgG4, IgA1 , and IgA2. The heavy chain constant domains that correspond to the different classes of antibodies are called $\alpha$, $\delta$, $\epsilon$, $\gamma$, and $\mu$, respectively.

The term "monoclonal antibody" as used herein refers to an antibody obtained from a population of substantially homogeneous antibodies, e.g., the individual antibodies comprising the population are identical except for possible mutations, e.g., naturally occurring mutations, that may be present in minor amounts. Thus, the modifier "monoclonal" indicates the character of the antibody as not being a mixture of discrete antibodies In certain embodiments, such a monoclonal antibody typically includes an antibody comprising a polypeptide sequence that binds a target, wherein the target-binding polypeptide sequence was obtained by a process that includes the selection of a single target-binding polypeptide sequence from a plurality of polypeptide sequences. For example, the selection process can be the selection of a unique clone from a plurality of clones, such as a pool of hybridoma clones, phage clones, or recombinant DNA clones. It should be understood that a selected target-binding sequence can be further altered, for example, to improve affinity for the target, to humanize the target-binding sequence, to improve its production in cell culture, to reduce its immunogenicity in vivo, to create a multispecific antibody, etc., and that an antibody comprising the altered target-binding sequence is also a monoclonal antibody of this invention. In contrast to polyclonal antibody preparations, which typically include different antibodies directed against different determinants (epitopes), each monoclonal antibody of a monoclonal antibody preparation is directed against a single determinant on an antigen. In addition to their specificity, monoclonal antibody preparations are advantageous in that they are typically uncontaminated by other immunoglobulins.

The modifier "monoclonal" indicates the character of the antibody as being obtained from a substantially homogeneous population of antibodies, and is not to be construed as requiring production of the antibody by any particular method. For example, the monoclonal antibodies to be used in accordance with the invention may be made by a variety of techniques, including, for example, the hybridoma method (e.g, Kohler and Milstein, Nature 256:495-97 (1975); Hongo et al., Hybridoma 14 (3): 253-260 (1995), Harlow et al., Antibodies: A Laboratory Manual (Cold Spring Harbor Laboratory Press, 2nd ed. 1988), Hammerling et al., in: Monoclonal Antibodies and T-Cell Hybridomas 563-681 (Elsevier, N.Y., 1981 )), recombinant DNA methods (see, e.g, U.S. Pat. No. 4,816,567), phage-display technologies (see, e.g., Clackson et al., Nature, 352: 624-628 (1991 ); Marks et al., J. Mol. Biol. 222: 581 -597 (1992); Sidhu et al., J. Mol. Biol. 338(2): 299-31 0 (2004); Lee et al., J. Mol. Biol. 340(5): 1073-1093 (2004); Fellouse, Proc. Natl. Acad Sci USA 101 (34): 12467-12472 (2004), and Lee et al., J Immunol. Methods 284(1 -2): 1 1 9-132 (2004)). and technologies for producing human or human-like antibodies in animals that have parts or all of the human immunoglobulin loci or genes encoding human immunoglobulin sequences (see, e.g., WO 1998/24893; WO 1996/34096; WO 1996/33735; WO 1991 /10741 ; Jakobovits et al., Proc. Natl. Acad. Sci. USA 90: 2551 (1993); Jakobovits et al., Nature 362: 255-258 (1993); Bruggemann et al., Year in Immunol. 7:33 (1 993); U.S. Pat. Nos 5,545,807; 5,545,806; 5,569,825; 5,625,126; 5,633,425; and 5,661 ,016; Marks et al., Bio/Technology 10: 779-783 (1992); Lonberg et al., Nature 368: 856-859 (1994); Morrison, Nature 368: 812-813 (1994); Fishwild et al., Nature Biotechnol. 14: 845-851 (1996); Neuberger, Nature Biotechnol. 14: 826 (1996); and Lonberg et al., Intern. Rev. Immunol. 13. 65-93 (1995)).

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see, e.g., U.S. Pat. No. 4,816,567; and Morrison et al., Proc. Natl. Acad. Sci. USA 81 .6851 -6855 (1984)) Chimeric antibodies include PRIMATIZED® antibodies wherein the antigen-binding region of the antibody is derived from an antibody produced by, e g., immunizing macaque monkeys with the antigen of interest

A "human antibody" is one which possesses an amino acid sequence which corresponds to that of an antibody produced by a human or a human cell or derived from a non-human source that utilizes human antibody repertoires or other human antibody-encoding sequences This definition of a human antibody specifically excludes a humanized antibody comprising non-human antigen-binding residues.

A "humanized" antibody refers to a chimeric antibody comprising amino acid residues from non-human HVRs and amino acid residues from human framework regions (FRs). In certain embodiments, a humanized antibody will comprise substantially all of at least one, and typically two, variable domains, in which all or substantially all of the HVRs (e.g., CDRs) correspond to those of a non-human antibody, and all or substantially all of the Fits correspond to those of a human antibody. A humanized antibody optionally may comprise at least a portion of an antibody constant region derived from a human antibody.

A "humanized form" of an antibody, e.g., a non-human antibody, refers to an antibody that has undergone humanization

A "blocking" antibody or an "antagonist" antibody is one which inhibits or reduces biological activity of the antigen it binds For example, blocking antibodies or antagonist antibodies substantially or completely inhibit the biological activity of the antigen.

"Affinity" refers to the strength of the sum total of noncovalent interactions between a single binding site of a molecule (e g., an antibody) and its binding partner (e g., an antigen). Unless indicated otherwise, as used herein, "binding affinity" refers to intrinsic binding affinity which reflects a 1 :1 interaction between members of a binding pair (e.g., antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

As used herein, the term "binds", "specifically binds to" or is "specific for" refers to measurable and reproducible interactions such as binding between a target and an antibody, which is determinative of the presence of the target in the presence of a heterogeneous population of molecules including biological molecules. For example, an antibody that binds to or specifically binds to a target (which can be an epitope) is an antibody that binds this target with greater affinity, avidity, more readily, and/or with greater duration than it binds to other targets. In one embodiment, the extent of binding of an antibody to an unrelated target is less than about 1 0% of the binding of the antibody to the target as measured, e.g., by a radioimmunoassay (RIA). In certain embodiments, an antibody that specifically binds to a target has a dissociation constant (Kd) of < 1 $\mu$M, < 100 nM, < 10 nM, < 1 nM, or < 0.1 nM. In certain embodiments, an antibody specifically binds to an epitope on a protein that is conserved among the protein from different species. In another embodiment, specific binding can include, but does not require exclusive binding.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more.

An "immunoconjugate" is an antibody conjugated to one or more heterologous molecule(s), including but not limited to a cytotoxic agent.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG1 , IgG2 (including IgG2A and IgG2B), IgG3, or IgG4 subtypes, IgA (including IgA1 and IgA2), IgE, IgD or IgM. The Ig fusions include the substitution of a domain of a polypeptide or antibody described herein in the place of at least one variable region within an Ig molecule. In some embodiments, the immunoglobulin fusion includes the hinge, CH2 and CH3, or the hinge, CH1 , CH2 and CH3 regions of an IgG1 molecule. For the production of immunoglobulin fusions see also US Patent No. 5,428,130. For example, useful immunoadhesins as medicaments useful for therapy herein include polypeptides that comprise the extracellular domain (ECD) or PD-1 -binding portions of PD-L1 or PD-L2, or the extracellular or PD-L1 - or PD-L2-binding portions of PD-1 , fused to a constant domain of an immunoglobulin sequence, such as a PD-L1 ECD-Fc, a PD-L2 ECD-Fc, and a PD-1 ECD-Fc, respectively. Immunoadhesin combinations of Ig Fc and ECD of cell surface receptors are sometimes termed soluble receptors.

A "fusion protein" and a "fusion polypeptide" refer to a polypeptide having two portions covalently linked together, where each of the portions is a polypeptide having a different property. The property may be a biological property, such as activity in vitro or in vivo. The property may also be a simple chemical or physical property, such as binding to a target molecule, catalysis of a reaction, and the like. The two portions may be linked directly by a single peptide bond or through a peptide linker but are in reading frame with each other.

"Percent (%) amino acid sequence identity" with respect to the polypeptide sequences identified herein is defined as the percentage of amino acid residues in a candidate sequence that are identical with the amino acid residues in the polypeptide being compared, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are generated using the sequence comparison computer program ALIGN-2. The ALIGN-2 sequence comparison computer program was authored by Genentech, Inc. and the source code has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California. The ALIGN-2 program should be compiled for use on a UNIX operating system, for example, digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

$$100 \text{ times the fraction } X/Y$$

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "detection" includes any means of detecting, including direct and indirect detection. The term "biomarker" as used herein refers to an indicator, e.g., predictive, diagnostic, and/or prognostic, which can be detected in a sample. The biomarker may serve as an indicator of a particular subtype of a disease or disorder (e.g., cancer) characterized by certain, molecular, pathological, histological, and/or clinical features (e.g., responsiveness to therapy including a checkpoint inhibitor). In some embodiments, a biomarker is a collection of genes or a collective number of mutations/alterations (e.g., somatic mutations) in a collection of genes. Biomarkers include, but are not limited to, polynucleotides (e.g., DNA and/or RNA), polynucleotide alterations (e.g., polynucleotide copy number alterations, e.g., DNA copy number alterations), polypeptides, polypeptide and polynucleotide modifications (e.g., post-translational modifications), carbohydrates, and/or glycolipid-based molecular markers.

The "amount" or "number" of somatic mutations associated with an increased clinical benefit to an individual is a detectable level in a biological sample. These can be measured by methods known to one skilled in the art and also disclosed herein. The amount of a somatic mutation assessed can be used to determine the response to the treatment.

"Amplification," as used herein generally refers to the process of producing multiple copies of a desired sequence. "Multiple copies" mean at least two copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

The technique of "polymerase chain reaction" or "PCR" as used herein generally refers to a procedure wherein minute amounts of a specific piece of nucleic acid, RNA and/or DNA, are amplified as described, for example, in U.S. Pat. No. 4,683,195. Generally, sequence information from the ends of the region of interest or beyond needs to be available, such that oligonucleotide primers can be designed; these primers will be identical or similar in sequence to opposite strands of the template to be amplified. The 5' terminal nucleotides of the two primers may coincide with the ends of the amplified material. PCR can be used to amplify specific RNA sequences, specific DNA sequences from total genomic DNA, and cDNA transcribed from total cellular RNA, bacteriophage, or plasmid sequences, etc. See generally Mullis et al., Cold Spring Harbor Symp. Quant. Biol. 51 :263 (1987) and Erlich, ed., PCR Technology (Stockton Press, NY, 1989). As used herein, PCR is considered to be one, but not the only, example of a nucleic acid polymerase reaction method for amplifying a nucleic acid test sample, comprising the use of a known nucleic acid (DNA or RNA) as a primer and utilizes a nucleic acid polymerase to amplify or generate a specific piece of nucleic acid or to amplify or generate a specific piece of nucleic acid which is complementary to a particular nucleic acid.

The term "diagnosis" is used herein to refer to the identification or classification of a molecular or pathological state, disease or condition (e.g., cancer). For example, "diagnosis" may refer to identification of a particular type of cancer. "Diagnosis" may also refer to the classification of a particular subtype of cancer, for instance, by histopathological criteria, or by molecular features (e.g., a subtype characterized by expression of one or a combination of biomarkers (e.g., particular genes or proteins encoded by said genes)).

The term "aiding diagnosis" is used herein to refer to methods that assist in making a clinical determination regarding the presence, or nature, of a particular type of symptom or condition of a disease or disorder (e.g., cancer). For example, a method of aiding diagnosis of a disease or condition (e.g., cancer) can comprise measuring certain somatic mutations in a biological sample from an individual.

The term "sample," as used herein, refers to a composition that is obtained or derived from a subject and/or individual of interest that contains a cellular and/or other molecular entity that is to be characterized and/or identified, for example, based on physical, biochemical, chemical, and/or physiological characteristics. For example, the phrase "disease sample" and variations thereof refers to any sample obtained from a subject of interest that would be expected or is known to contain the cellular and/or molecular entity that is to be characterized. Samples include, but are not limited to, tissue samples, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, plasma, serum, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, and combinations thereof. In some instances, the sample is a whole blood sample, a plasma sample, a serum sample, or a combination thereof. In some embodiments, the sample is from a tumor (e.g., a "tumor sample"), such as from a biopsy. In some embodiments, the sample is a formalin-fixed paraffin-embedded (FFPE) sample.

A "tumor cell" as used herein, refers to any tumor cell present in a tumor or a sample thereof. Tumor cells may be distinguished from other cells that may be present in a tumor sample, for example, stromal cells and tumor-infiltrating immune cells, using methods known in the art and/or described herein.

A "reference sample," "reference cell," "reference tissue," "control sample," "control cell," or "control tissue," as used herein, refers to a sample, cell, tissue, standard, or level that is used for comparison purposes.

By "correlate" or "correlating" is meant comparing, in any way, the performance and/or results of a first analysis or protocol with the performance and/or results of a second analysis or protocol. For example, one may use the results of a first analysis or protocol in carrying out a second protocol and/or one may use the results of a first analysis or protocol to determine whether a second analysis or protocol should be performed. With respect to the embodiment of polypeptide analysis or protocol, one may use the results of the polypeptide expression analysis or protocol to determine whether a specific therapeutic regimen should be performed. With respect to the embodiment of polynucleotide analysis or protocol, one may use the results of the polynucleotide expression analysis or protocol to determine whether a specific therapeutic regimen should be performed.

"Obtaining a sequence" as the term is used herein, refers to obtaining possession of a nucleotide sequence or amino acid sequence, by "directly obtaining" or "indirectly obtaining" the sequence. "Directly obtaining a sequence" means performing a process (e.g., performing a synthetic or analytical method) to obtain the sequence, such as performing a sequencing method (e.g., a Next Generation Sequencing (NGS) method). "Indirectly obtaining a sequence" refers to receiving information or knowledge of, or receiving, the sequence from another party or source (e.g., a third party laboratory that directly acquired the sequence). The sequence obtained need not be a full sequence, e.g., sequencing of at least one nucleotide, or obtaining information or knowledge, that identifies a fusion molecule disclosed herein as being present in a subject constitutes obtaining a sequence.

Directly obtaining a sequence includes performing a process that includes a physical change in a physical substance, e.g., a starting material, such as a tissue sample, e.g., a biopsy, or a nucleic acid (e.g., DNA or RNA) sample. Exemplary changes include making a physical entity from two or more starting materials, shearing or fragmenting a substance, such as a genomic DNA fragment; separating or purifying a substance (e.g., isolating a nucleic acid sample from a tissue); combining two or more separate entities into a mixture, performing a chemical reaction that includes breaking or forming a covalent or non-covalent bond.

"Obtaining a sample" as the term is used herein, refers to obtaining possession of a sample, e.g., a tissue sample or nucleic acid sample, by "directly obtaining" or "indirectly obtaining" the sample. "Directly obtaining a sample" means performing a process (e.g., performing a physical method such as a surgery or extraction) to obtain the sample. "Indirectly obtaining a sample" refers to receiving the sample from another party or source (e.g., a third-party laboratory that directly obtained the sample). Directly obtaining a sample includes performing a process that includes a physical change in a physical substance, e.g., a starting material, such as a tissue, e.g., a tissue in a human patient or a tissue that has was previously isolated from a patient. Exemplary changes include making a physical entity from a starting material, dissecting or scraping a tissue; separating or purifying a substance (e.g., a sample tissue or a nucleic acid sample); combining two or more separate entities into a mixture; performing a chemical reaction that includes breaking or forming a covalent or non-covalent bond. Directly obtaining a sample includes performing a process that includes a physical change in a sample or another substance, *e.g.*, as described above.

"Individual response" or "response" can be assessed using any endpoint indicating a benefit to the individual, including, without limitation, (1) inhibition, to some extent, of disease progression (e.g., cancer progression), including slowing down or complete arrest; (2) a reduction in tumor size; (3) inhibition (i.e., reduction, slowing down, or complete stopping) of cancer cell infiltration into adjacent peripheral organs and/or tissues; (4) inhibition (i.e. reduction, slowing down, or complete stopping) of metastasis; (5) relief, to some extent, of one or more symptoms associated with the disease or disorder (e.g., cancer); (6) increase or extension in the length of survival, including overall survival and progression free survival; and/or (7) decreased mortality at a given point of time following treatment.

An "effective response" of a patient or a patient's "responsiveness" to treatment with a medicament and similar wording refers to the clinical or therapeutic benefit imparted to a patient at risk for, or suffering from, a disease or disorder. such as cancer. In one embodiment. such benefit includes any one or more of: extending survival (including overall survival and/or progression-free survival); resulting in an objective response (including a complete response or a partial response); or improving signs or symptoms of cancer.

An "effective amount" refers to an amount of a targeted therapy or therapeutic agent to treat or prevent a disease or disorder in a mammal. In the case of cancers, the therapeutically effective amount of the therapy or agent may reduce the number of cancer cells; reduce the primary tumor size; inhibit (i.e., slow to some extent and in some embodiments stop) cancer cell infiltration into peripheral organs; inhibit (i.e., slow to some extent and in some embodiments stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, time to disease progression (TTP), response rates (e.g., CR and PR), duration of response, and/or quality of life.

The term "tumor," as used herein, refers to all neoplastic cell growth and proliferation, whether malignant or benign, and all pre-cancerous and cancerous cells and tissues. The terms "cancer," "cancerous," and "tumor" are not mutually exclusive as referred to herein.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies (e.g., antibody-based checkpoint inhibitors) are used to delay development of a disease or to slow the progression of a disease.

As used herein, the terms "individual," "patient," or "subject" are used interchangeably and refer to any single animal, *e.g.*, a mammal (including such non-human animals as, for example, dogs, cats, horses, rabbits, zoo animals. cows, pigs, sheep, and non-human primates) for which treatment is desired. In particular embodiments, the patient herein is a human.

As used herein, "administering" is meant a method of giving a dosage of a compound (e.g., an antagonist) or a pharmaceutical composition (e.g., a pharmaceutical composition including an antagonist) to a subject (e.g., a patient). Administering can be by any suitable means, including parenteral, intrapulmonary, and intranasal, and, if desired for local treatment, intralesional administration. Parenteral infusions include, for example, intramuscular, intravenous, intraarterial, intraperitoneal, or subcutaneous administration. Dosing can be by any suitable route, e.g., by injections, such as intravenous or subcutaneous injections, depending in part on whether the administration is brief or chronic. Various dosing schedules including but not limited to single or multiple administrations over various time-points, bolus administration, and pulse infusion are contemplated herein.

The term "concurrently" is used herein to refer to administration of two or more therapies or therapeutic agents, where at least part of the administration overlaps in time. Accordingly, concurrent administration includes a dosing regimen when the administration of one or more agent(s) continues after discontinuing the administration of one or more other agent(s).

By "reduce or inhibit" is meant the ability to cause an overall decrease of 20%, 30%, 40%, 50%, 60%, 70%, 75%, 80%, 85%, 90%, 95%, or greater. Reduce or inhibit can refer, for example, to the symptoms of the disorder being treated, the presence or size of metastases, or the size of the primary tumor.

The term "package insert" is used to refer to instructions customarily included in commercial packages of therapeutic products, that contain information about the indications, usage, dosage, administration, combination therapy, contraindications, and/or warnings concerning the use of such therapeutic products.

An "article of manufacture" is any manufacture (e.g., a package or container) or kit comprising at least one reagent, e.g., a medicament for treatment of a disease or disorder (e.g., cancer), or a probe for specifically detecting a biomarker (e.g., a neoantigen or *EWSR1-WT1* gene fusion) described herein. In certain embodiments, the manufacture or kit is promoted, distributed, or sold as a unit for performing the methods described herein.

The phrase "based on" when used herein means that the information about one or more biomarkers is used to inform a treatment decision, information provided on a package insert, or marketing/promotional guidance, etc.

## III. Methods

In one aspect, provided herein are methods of detecting overexpression of an oncogene, *e.g.,* in an individual, or in a cancer or tumor. In some embodiments, the methods comprise detecting a presence of a translocation comprising a first breakpoint in the IGH locus and a second breakpoint (*e.g.*, in a sample). In some embodiments, the second breakpoint is 1.3Mb or less from a TSS of an oncogene.

In another aspect, provided herein are methods of assessing or diagnosing overexpression of an oncogene, *e.g.*, in an individual, or in a cancer or tumor. In some embodiments, the methods comprise detecting a presence of a translocation comprising a first breakpoint in the IGH locus and a second breakpoint (*e.g.*, in a sample); and providing an assessment or diagnosis of overexpression of the oncogene when the second breakpoint is detected 1.3Mb or less from the TSS of the oncogene. In some embodiments, the methods optionally comprise providing an assessment or diagnosis of lack of overexpression of the oncogene when the second breakpoint is detected more than 1.3Mb from the TSS of the oncogene.

In another aspect, provided herein are methods of detecting overexpression of an oncogene. In some embodiments, the method comprises (a) detecting a presence of a translocation comprising a first breakpoint in the IGH locus and a second breakpoint in a sample from an individual, wherein the second breakpoint is 1.3Mb or less from a TSS of the oncogene, and wherein the translocation or breakpoint(s) is/are detected in DNA from the sample; and (b) measuring expression of the oncogene in a sample from the individual, wherein expression of the oncogene is measured in RNA from the sample.

In another aspect, provided herein are methods of monitoring an individual having cancer. In some embodiments, the method comprises acquiring knowledge of a translocation comprising a first breakpoint in the IGH locus and a second breakpoint in a sample from the individual, wherein the second breakpoint is 1.3Mb or less from a TSS of an oncogene, and wherein, responsive to the acquisition of said knowledge, the individual is predicted to have increased risk of overexpression of the oncogene, as compared to an individual whose cancer does not exhibit a breakpoint 1.3Mb or less from the TSS.

In another aspect, provided herein are methods of screening an individual having cancer. In some embodiments, the method comprises acquiring knowledge of a translocation comprising a first breakpoint in the IGH locus and a second breakpoint in a sample from the individual, wherein the second breakpoint is 1.3Mb or less from a TSS of an oncogene, and wherein, responsive to the acquisition of said knowledge, the individual is predicted to have increased risk of overexpression of the oncogene, as compared to an individual whose cancer does not exhibit a breakpoint 1.3Mb or less from the TSS.

In another aspect, provided herein are methods of evaluating an individual having cancer. In some embodiments, the method comprises: acquiring genotype information that identifies a first breakpoint in the IGH locus and a second breakpoint in the individual, wherein the second breakpoint is 1.3Mb or less from a TSS of an oncogene, and wherein the presence of the second breakpoint identifies the subject as having an increased risk of overexpression of the oncogene, as compared to an individual whose cancer does not exhibit a breakpoint 1.3Mb or less from the TSS.

In another aspect, provided herein are methods of generating a personalized cancer treatment report. In some embodiments, the method comprises (a) obtaining a sample from an individual; (b) detecting a presence of a translocation comprising a first breakpoint in the IGH locus and a second breakpoint in the sample, wherein the second breakpoint is 1.3Mb or less from a TSS of an oncogene; and (c) providing a report comprising information on the oncogene, the breakpoint(s), the IGH locus, or combinations thereof. In some embodiments, the method further comprises selecting one or more treatments based on the detection of the breakpoint 1.3Mb or less from the TSS of oncogene, wherein the report further comprises information on the one or more treatments.

In another aspect, provided herein are methods of identifying an individual having cancer who may benefit from a treatment comprising a targeted therapy. In some embodiments, the methods comprise detecting a presence of a translocation comprising a first breakpoint in the IGH locus and a second breakpoint (e.g., in a sample), wherein a second breakpoint detected 1.3Mb or less from the TSS of an oncogene identifies the individual as one who may benefit from a targeted therapy of the present disclosure. In some embodiments, the methods further comprise, after detection of the translocation, providing the individual with a recommendation of a treatment comprising the targeted therapy (*e.g.*, responsive at least in part to detection of the breakpoint 1.3Mb or less from the TSS of the oncogene).

In another aspect, provided herein are methods of selecting a therapy for an individual having cancer. In some embodiments, the methods comprise detecting a presence of a translocation comprising a first breakpoint in the IGH locus and a second breakpoint (*e.g.*, in a sample), wherein a second breakpoint detected 1.3Mb or less from the TSS of an oncogene identifies the individual as one who may benefit from a targeted therapy of the present disclosure. In some embodiments, the methods further comprise, after detection of the translocation, selecting a therapy for the individual that targets the oncogene (*e.g.*, responsive at least in part to the detection of the breakpoint 1.3Mb or less from the TSS of the oncogene). In some embodiments, the methods comprise detecting a presence of a translocation comprising a first breakpoint in the IGH locus and a second breakpoint in a sample from the individual, wherein the second breakpoint is 1.3Mb or less from a TSS of an oncogene, and wherein the detection of the breakpoint 1.3Mb or less from the TSS of the oncogene in the sample identifies the individual as one who may benefit from a targeted therapy comprising an agent that inhibits expression and/or activity of a protein product of the oncogene.

In another aspect, provided herein are methods of selecting a therapy or treatment for an individual having cancer. In some embodiments, the methods comprise acquiring knowledge of a translocation comprising a first breakpoint in the IGH locus and a second breakpoint (*e.g.*, in a sample). In some embodiments, the second breakpoint is 1.3Mb or less from a TSS of an oncogene. In some embodiments, the methods further comprise, after detection of the translocation, selecting a therapy or treatment for the individual that targets the oncogene (*e.g.,* responsive at least in part to the detection of the breakpoint 1.3Mb or less from the TSS of the oncogene). In some embodiments, the subject is classified as a candidate to receive treatment with a targeted therapy of the present disclosure (*e.g.*, responsive at least in part to the acquisition of the knowledge) and/or the subject is identified as likely to respond to a treatment that comprises a targeted therapy of the present disclosure (*e.g.*, responsive at least in part to the acquisition of the knowledge).

In another aspect, provided herein are methods of identifying one or more treatment options for an individual having cancer. In some embodiments, the methods comprise detecting a presence of a translocation comprising a first breakpoint in the IGH locus and a second breakpoint (*e.g.*. in a sample); and generating a report that may contain one or more treatment options identified as to be considered for the treatment of an individual, e.g., based at least in part on detection of the translocation. In some embodiments, the second breakpoint is 1.3Mb or less from a TSS of an oncogene. In some embodiments, the one or more treatment options comprise a targeted therapy of the present disclosure.

In another aspect, provided herein are methods of identifying one or more treatment options for an individual having cancer. In some embodiments, the methods comprise acquiring knowledge of a translocation comprising a first breakpoint in the IGH locus and a second breakpoint (*e.g.*. in a sample); and generating a report that may contain one or more treatment options identified as to be considered for the treatment of an individual, *e.g.*, based at least in part on knowledge of the translocation. In some embodiments, the second breakpoint is 1.3Mb or less from a TSS of an oncogene. In some embodiments, the one or more treatment options comprise a targeted therapy of the present disclosure.

In some embodiments, the methods of the present disclosure further comprise providing a report (*e.g.*, to another party). In some embodiments, the report comprises: information on the role of the oncogene and/or IGH translocation, or wild-type sequence, in disease; information on prognosis, resistance, or potential therapeutic options that may be considered for the subject; information on the potential effectiveness of a therapeutic option, the acceptability of a therapeutic option, or the advisability of applying a therapeutic option to the subject *(e.g.,* a targeted therapy of the present disclosure) to the subject.

Methods described herein can include providing a report, such as, in electronic, web-based, or paper form, to the patient or to another person or entity, *e.g.,* a caregiver, *e.g., a* physician, e.g., an oncologist, a hospital, clinic, third-party payor, insurance company or government entity. The report can include output from the method, *e.g.*, the indication of the presence or absence of an IGH breakpoint or an oncogene whose expression is known or likely to be increased by an IGH breakpoint. In one embodiment, a report is generated, such as in paper or electronic form, which identifies the presence or absence of an IGH breakpoint, and optionally includes an identifier for the patient from which the sequence was obtained. The report can also include information on the role of an IGH breakpoint or oncogene described herein, or wild-type sequence, in disease. Such information can include information on prognosis, resistance, or potential therapeutic options. The report can include information on the potential effectiveness of a therapeutic option (*e.g.*, in the patient's cancer type, potentially with similar genomic alterations as the patient), *e.g.*, a patient having a sequence, alteration or mutation identified in the test, and in some embodiments, identified in the report. In an embodiment, not all mutations identified in the method are identified in the report. For example, the report can be limited to disclosing mutations in genes having a level of correlation with the occurrence, prognosis, stage, or susceptibility of a cancer to a treatment, *e.g.*, with a therapeutic option. The report can be delivered, *e.g.*, to an entity described herein, within about or approximately 7, 14, or 21 days from receipt of the sample by an entity practicing the method of providing a report.

In some embodiments, the methods of the present disclosure further comprise generating a report, with information specific to the genomic alterations detected in the patient's sample, by obtaining a sample, *e.g.,* a tumor sample, from a subject, and detecting a translocation of the present disclosure in the sample. In one embodiment, a report is generated that annotates a potential treatment option, or that lists, *e.g.,* in alphabetical order for each actionable gene, two or more treatment options based on the mutation identified.

In some embodiments, the methods of the present disclosure further comprise one or more of (e.g., responsive at least in part to the detection of the breakpoint 1.3Mb or less from the TSS of the oncogene, or responsive at least in part to acquisition of knowledge thereof):

(1) stratifying a patient population *(e.g.,* assigning a subject, *e.g.,* a patient, to a group or class);
(2) identifying or selecting a subject as likely or unlikely to respond to a treatment, *e.g.*, a targeted therapy as described herein;
(3) selecting a treatment option, *e.g.*, administering or not administering a targeted therapy as described herein; or
4) prognosticating the time course of a disease in the subject (*e.g.*, evaluating the likelihood of increased or decreased patient survival).

In certain embodiments, responsive to the determination of the presence of an IGH translocation as described herein, the patient is classified as a candidate to receive treatment with a targeted therapy as described herein. In one embodiment, responsive to the determination of the presence of an IGH translocation described herein, the patient can further be assigned to a particular class if an IGH translocation is identified in a sample of the patient. For example, a patient identified as having an IGH translocation described herein can be classified as a candidate to receive treatment with a therapy targeting an oncogene or its gene product, *e.g,* if a breakpoint is 1.3Mb or less from a TSS of the oncogene. In one embodiment, the patient is assigned to a second class if the translocation is not present. For example, the patient who has a cancer that does not contain an IGH translocation described herein may be determined as not being a candidate to receive a targeted therapy described herein.

In another aspect, provided herein are methods of treating or delaying progression of cancer. In some embodiments, the methods comprise administering to an individual an effective amount of a targeted therapy of the present disclosure. In some embodiments, the cancer comprises a translocation comprising a first breakpoint in the IGH locus and a second breakpoint. In some embodiments, the second breakpoint is 1.3Mb or less from a transcription start site (TSS) of an oncogene. In some embodiments, the targeted therapy is administered responsive at least in part to knowledge and/or detection of a translocation comprising a first breakpoint in the IGH locus and a second breakpoint, *e.g.,* in a sample from the individual. In some embodiments, the methods further comprise detecting a presence of a translocation comprising a first breakpoint in the IGH locus and a second breakpoint, *e.g.,* in a sample from an individual. In some embodiments, the translocation is detected *in vitro, e.g.,* as described herein. In some embodiments, the methods further comprise acquiring knowledge of a translocation comprising a first breakpoint in the IGH locus and a second breakpoint, *e.g.*, in a sample from an individual. In some embodiments, the methods further comprise obtaining a sample from the individual.

The methods of the present disclosure are contemplated for use with a variety of cancers. In some embodiments, a cancer of the present disclosure is multiple myeloma (MM), B-cell lymphoma, diffuse large B-cell lymphoma (DLBCL), B-cell lymphoma unclassified, acute myeloid leukemia (AML), follicular lymphoma (FL), Burkitt lymphoma, acute lymphoblastic leukemia (ALL), B-cell acute lymphoblastic leukemia (B-ALL), non-Hodgkin's lymphoma (NHL), B-cell non-Hodgkin's lymphoma (B-NHL), Waldenstroms lymphoma, extranodal lymphoma, mantle cell lymphoma (MCL), mature B cell neoplasm, hairy cell leukemia (HCL), hairy cell leukemia variant (HCL-v), splenic marginal zone lymphoma (SMZL), nodal marginal zone lymphoma, extranodal marginal zone lymphoma, MALT lymphoma, myeloproliferative neoplasm (MPN), skin lymphoma, lymphoproliferative disease, primary mediastinal large B-cell lymphoma (PMBCL), plasmablastic lymphoma, chronic lymphocytic leukemia (CLL), small cell lymphoma (SLL), Hodgkin's lymphoma (HL), bone marrow lymph proliferative disease, myelodysplastic syndrome, or angioimmunoblastic T-cell lymphoma (AITL).

Exemplary formulations for targeted therapies and methods of administration thereof are provided *infra*.

In other aspects, provided herein is a method of identifying an individual having cancer who may benefit from a treatment comprising a chemotherapy or non-targeted therapy. In some embodiments, the method comprises detecting absence of a translocation comprising a first breakpoint in the IGH locus and a second breakpoint in a sample from the individual, wherein absence of an IGH translocation in the sample identifies the individual as one who may benefit from a chemotherapy or non-targeted therapy.

In other aspects, provided herein is a method of selecting a therapy for an individual having cancer. In some embodiments, the method comprises detecting absence of a translocation comprising a first breakpoint in the IGH locus and a second breakpoint in a sample from the individual, wherein the detection absence of the IGH translocation in the sample identifies the individual as one who may benefit from a chemotherapy or non-targeted therapy.

In other aspects, provided herein is a method of identifying one or more treatment options for an individual having cancer. In some embodiments, the method comprises (a) acquiring knowledge of absence of a translocation comprising a first breakpoint in the IGH locus and a second breakpoint in a sample from an individual; and (b) generating a report comprising one or more treatment options identified for the individual based at least in part on said knowledge, wherein the one or more treatment options comprise a chemotherapy or non-targeted therapy.

In other aspects, provided herein is a method of identifying one or more treatment options for an individual having cancer. In some embodiments, the method comprises (a) detecting absence of a translocation comprising a first breakpoint in the IGH locus and a second breakpoint in a sample from the individual; and (b) generating a report comprising one or more treatment options identified for the individual based at least in part on the detection of absence of an IGH translocation in the sample, wherein the one or more treatment options comprise a chemotherapy or non-targeted therapy.

In other aspects, provided herein is a method of treating or delaying progression of cancer. In some embodiments, the method comprises, responsive to knowledge of an absence of a translocation comprising a first breakpoint in the IGH locus and a second breakpoint in a sample from an individual, administering to the individual an effective amount of a chemotherapy or non-targeted therapy.

In other aspects, provided herein is a method of treating or delaying progression of cancer. In some embodiments, the method comprises (a) detecting absence of a translocation comprising a first breakpoint in the IGH locus and a second breakpoint in a sample from an individual; and (b) administering to the individual an effective amount of a chemotherapy or non-targeted therapy based at least in part on the detection of the absence of an IGH translocation in the sample.

In other aspects, provided herein is a method of treating or delaying progression of cancer. In some embodiments, the method comprises (a) acquiring knowledge of an absence of a translocation comprising a first breakpoint in the IGH locus and a second breakpoint in a sample from an individual; and (b) responsive to said knowledge, administering to the individual an effective amount of a chemotherapy or non-targeted therapy.

In other aspects, provided herein is a method of selecting treatment for a subject having cancer. In some embodiments, the method comprises acquiring knowledge of an absence of a translocation comprising a first breakpoint in the IGH locus and a second breakpoint in a sample from an individual having cancer, wherein responsive to the acquisition of said knowledge: (i) the subject is classified as a candidate to receive treatment with a chemotherapy or non-targeted therapy; (ii) the subject is identified as likely to respond to a chemotherapy or non-targeted therapy; (iii) the subject is classified as a candidate to receive treatment with one or more selected chemotherapies; and/or (iv) the subject is classified as not a candidate to receive treatment with a selected therapy.

### *Detection of IGH Translocations*

Certain aspects of the present disclosure relate to detecting a presence of a translocation comprising a breakpoint in the immunoglobulin heavy chain (IGH) locus, *e.g.,* in a sample (such as a patient sample), and a second breakpoint (*e.g.,* in proximity to the TSS of an oncogene). In some embodiments, the translocation is detected *in vitro.*

In some embodiments, a translocation of the present disclosure comprises a breakpoint that is 1.3Mb or less from a TSS of an oncogene. In some embodiments, a translocation of the present disclosure comprises a breakpoint that is greater than 0Mb and less than or equal to 1.3Mb from a TSS of an oncogene. In some embodiments, a translocation of the present disclosure comprises a breakpoint that is greater than or equal to 1.0Mb and less than or equal to 1.3Mb from a TSS of an oncogene. In some embodiments, a translocation of the present disclosure comprises a breakpoint that is greater than or equal to 0.5Mb and less than or equal to 1.3Mb from a TSS of an oncogene. In some embodiments, a translocation of the present disclosure comprises a breakpoint that is greater than or equal to 0.7Mb and less than or equal to 1.3Mb from a TSS of an oncogene. In some embodiments, a translocation of the present disclosure comprises a breakpoint that is greater than or equal to 0.2Mb and less than or equal to 1.3Mb from a TSS of an oncogene. In some embodiments, a translocation of the present disclosure comprises a breakpoint that is greater than or equal to 0.3Mb and less than or equal to 1.3Mb from a TSS of an oncogene. In some embodiments, a translocation of the present disclosure comprises a breakpoint that is 1.3Mb or less, 1.2Mb or less, 1.1Mb or less, 1.0Mb or less, 0.9Mb or less, 0.8Mb or less, 0.7Mb or less, 0.6Mb or less, 0.5Mb or less, 0.4Mb or less, 0.3Mb or less, 0.2Mb or less, or 0.1Mb or less from a TSS of an oncogene. In some embodiments, a translocation of the present disclosure comprises a breakpoint that is 2.1Mb or less from a TSS of an oncogene. In some embodiments, the IGH translocation is 1.0Mb or less, 500kb or less, 400kb or less, 300kb or less, 200kb or less, 100kb or less, 90kb or less, 80kb or less, 70kb or less, 60kb or less, 50kb or less, 40kb or less, 30kb or less, 20kb or less, 10kb or less, 5kb or less, or 1kb or less from the TSS of the oncogene. In some embodiments, a distance between a breakpoint and the TSS of an oncogene refers to a distance from the edge of the breakpoint to the TSS, *e.g.,* based on genomic coordinates as determined *infra.* In some embodiments, the breakpoint is part of a translocation involving the IGH locus. In some embodiments, the breakpoint is part of a translocation involving another breakpoint, e.g., located in the IGH locus.

In some embodiments, the genomic location or coordinates of a of the present disclosure is determined by comparison to a reference genome, e.g., a reference human genome. An exemplary reference human genome may be found, *e.g.,* at the Human Genome Browser at UCSC *(see* genome.ucsc.edu).

In some embodiments, the genomic location or coordinates of an oncogene's TSS of the present disclosure is determined by comparison to a reference genome, *e.g.,* a reference human genome. An exemplary reference human genome may be found, *e.g.,* at the Human Genome Browser at UCSC (see genome.ucsc.edu).

In some embodiments, an IGH translocation of the present disclosure results in at least a 2-fold increase in expression of an oncogene of the present disclosure (*e.g.*, an oncogene whose TSS is less than or equal to 1.3Mb from the IGH breakpoint). In some embodiments, an IGH translocation of the present disclosure results in at least a 2-fold, 3-fold, 4-fold, 5-fold, 6-fold, 7-fold, 8-fold, 9-fold, 10-fold, 15-fold, 20-fold, 25-fold, 50-fold, or 80-fold increase in expression of an oncogene of the present disclosure (*e.g.*, an oncogene whose TSS is less than or equal to 1.3Mb from the IGH breakpoint), *e.g.*, as compared to expression of the oncogene in the absence of an IGH translocation, or as compared to a reference or reference value.

In some embodiments, expression of an oncogene is compared to a reference or reference value. In some embodiments, expression of an oncogene in a patient sample or nucleic acid of the present disclosure is compared to expression of the oncogene in a reference sample, *e.g.,* a sample that does not comprise an IGH breakpoint. In some embodiments, expression of an oncogene in a patient sample or nucleic acid of the present disclosure is compared to a reference level of expression of the oncogene. In some embodiments, expression of an oncogene in a patient sample or nucleic acid of the present disclosure is compared to a reference value of expression of the oncogene. In some embodiments, expression of an oncogene in a patient sample or nucleic acid of the present disclosure is compared to expression of another gene, *e.g.*, in the patient sample, in a reference sample, or a reference value for expression of the other gene. In some embodiments, expression of an oncogene in a patient sample or nucleic acid of the present disclosure is normalized to a reference value, *e.g.,* as described in He, J. (2016) Blood 127:3004-3014. For example, number of sequence reads corresponding to the oncogene per a predetermined number of total sequence reads may be calculated and optionally compared to a reference value, such as mean or median number of sequence reads corresponding to the oncogene from one or multiple samples, or from a sample that does not comprise an IGH breakpoint.

In some embodiments, expression of an oncogene is RNA expression. RNA expression may be quantified by methods known in the art. In some embodiments, RNA expression is quantified by next-generation sequencing (NGS). In some embodiments, RNA expression is quantified by fluorescence *in situ* hybridization (FISH).

In some embodiments, expression of an oncogene is protein expression. In some embodiments, protein expression is quantified by Western blotting, enzyme-linked immunosorbent assay (ELISA), or mass spectrometry.

In some embodiments, a translocation of the present disclosure comprises a translocation of one or more enhancers of the IGH locus. In some embodiments, a translocation of the present disclosure results in translocation of one or more enhancers of the IGH locus with a different region of the genome, *e.g.*, such that the IGH translocation leads to IGH enhancers being juxtaposed into proximity with an oncogene TSS as described herein. In some embodiments, the enhancer is an Eμ enhancer, also known as the intronic enhancer. In some embodiments, the enhancer is a 3'-RR enhancer.

In some embodiments, a translocation comprising an IGH breakpoint is detected in a sample (such as a patient sample). In one embodiment, the sample is a nucleic acid sample. In one embodiment, the nucleic acid sample comprises DNA (e.g., cfDNA or ctDNA), *e.g.*, genomic DNA or cDNA, or RNA, *e.g,* mRNA. In one embodiment, the sample is a protein sample. In some embodiments, the sample comprises fluid (e.g., blood or serum), cells, or tissue *(e.g.,* a tumor tissue). In some embodiments, the sample is acquired from a subject (e.g., a human subject). In certain embodiments, the sample comprises a tumor biopsy, a circulating tumor cell, or a circulating tumor nucleic acid. In some embodiments, the IGH translocation is detected in DNA from the sample.

In one embodiment, the sample has been obtained from a subject (*e.g.*, a subject having or at risk of having a cancer, *e.g.,* a patient), or alternatively, the methods of the present disclosure further comprise obtaining a sample from a patient. In one embodiment, the sample has been indirectly obtained from a subject (*e.g.*, a subject having or at risk of having a cancer, *e.g.,* a patient), or alternatively, the methods of the present disclosure further comprise directly obtaining a sample from a patient. The sample can be chosen from one or more of: tissue, e.g, cancerous tissue *(e.g.,* a tissue biopsy), whole blood, serum, plasma, buccal scrape, sputum, saliva, cerebrospinal fluid, urine, stool, circulating tumor cells, circulating nucleic acids (*e.g.*, cfDNA or ctDNA), or bone marrow. In certain some embodiments, the sample comprises a tissue (e.g., a tumor biopsy), a circulating tumor cell (CTC), or a nucleic acid. In some embodiments, the sample is a tumor sample or otherwise comprises tumor DNA from a cancer of the present disclosure, e.g., multiple myeloma (MM), B-cell lymphoma, diffuse large B-cell lymphoma (DLBCL), B-cell lymphoma unclassified, acute myeloid leukemia (AML), follicular lymphoma (FL), Burkitt lymphoma, acute lymphoblastic leukemia (ALL), B-cell acute lymphoblastic leukemia (B-ALL), non-Hodgkin's lymphoma (NHL), B-cell non-Hodgkin's lymphoma (B-NHL), Waldenstroms lymphoma, extranodal lymphoma, mantle cell lymphoma (MCL), mature B cell neoplasm, hairy cell leukemia (HCL), hairy cell leukemia variant (HCL-v), splenic marginal zone lymphoma (SMZL), nodal marginal zone lymphoma, extranodal marginal zone lymphoma, MALT lymphoma, myeloproliferative neoplasm (MPN), skin lymphoma, lymphoproliferative disease, primary mediastinal large B-cell lymphoma (PMBCL), plasmablastic lymphoma, chronic lymphocytic leukemia (CLL), small cell lymphoma (SLL), Hodgkin's lymphoma (HL), bone marrow lymph proliferative disease, myelodysplastic syndrome, or angioimmunoblastic T-cell lymphoma (AITL).

In some embodiments, the methods of the present disclosure comprise: contacting a nucleic acid sample, *e.g.,* a genomic DNA sample *(e.g.,* a chromosomal sample or a fractionated, enriched or otherwise pre-treated sample) or a gene product (mRNA, cDNA), obtained from a patient, with a nucleic acid fragment *(e.g.,* a probe or primer as described herein) under conditions suitable for hybridization, and determining the presence or absence of the fusion nucleic acid molecule. The method can, optionally, include enriching a sample for the gene or gene product.

In some embodiments, the methods of the present disclosure comprise detection and/or measurement of DNA and RNA. For example, in some embodiments, the methods comprise detecting an IGH translocation of the present disclosure in DNA from a sample, and detecting or measuring expression *(e.g.,* overexpression) of an oncogene of the present disclosure in RNA from a sample. In some embodiments, DNA and RNA are measured from the same sample. In some embodiments, DNA and RNA are measured from different samples.

A variety of materials (such as tissues) can be the source of the nucleic acid samples used in the present methods. Genomic or subgenomic DNA fragments can be isolated from a subject's sample *(e.g.,* a tumor sample, a normal adjacent tissue (NAT), a blood sample or any normal control)). In certain embodiments, the tissue is preserved as a frozen sample or as formaldehyde- or paraformaldehyde-fixed paraffin-embedded (FFPE) tissue preparation. For example, the sample can be embedded in a matrix, *e.g.,* an FFPE block or a frozen sample. The isolating step can include flow-sorting of individual chromosomes; and/or micro-dissecting a subject's sample *(e.g.,* a tumor sample, a NAT, or a blood sample). In some embodiments, the sample is free, or essentially free, of cells. In some embodiments, the sample comprises cell-free DNA (cfDNA) or circulating tumor DNA (ctDNA).

The nucleic acid samples *(e.g.,* genomic DNA samples) can be fragmented or sheared by practicing routine techniques. For example, genomic DNA can be fragmented by physical shearing methods, enzymatic cleavage methods, chemical cleavage methods, and other methods well known to those skilled in the art.

Methods can further include isolating a nucleic acid sample to provide a library *(e.g.,* a nucleic acid library). In certain embodiments, the nucleic acid sample includes whole genomic, subgenomic fragments, or both. The nucleic acid samples can be used to prepare nucleic acid libraries. Thus, in one embodiment, the methods featured in the disclosure further include isolating a nucleic acid sample to provide a library *(e.g.,* a nucleic acid library as described herein). Protocols for isolating and preparing libraries from whole genomic or subgenomic fragments are known in the art. The nucleic acid library can contain all or substantially all of the complexity of the genome. The term "substantially all" in this context refers to the possibility that there can in practice be some unwanted loss of genome complexity during isolation and/or preparation of the library. The methods described herein also are useful in cases where the nucleic acid library is a portion of the genome, *i.e.,* where the complexity of the genome is reduced by design. In some embodiments, any selected portion of the genome can be used with the methods described herein. In some embodiments, the entire exome or a subset thereof is isolated. In certain embodiments, the genomic or subgenomic DNA fragment is isolated from a subject's sample *(e.g.,* a tumor sample, a normal adjacent tissue (NAT), a blood sample or any normal control)). In one embodiment, the sample *(e.g.,* the tumor or NAT sample) is a preserved. For example, the sample is embedded in a matrix, *e.g.,* an FFPE block or a frozen sample. In certain embodiments, the isolating step includes flow-sorting of individual chromosomes; and/or microdissecting a subject's sample *(e.g.,* a tumor sample, a NAT, a blood sample). In certain embodiments, the nucleic acid sample used to generate the nucleic acid library is less than 5 micrograms, less than 1 microgram, less than 500 ng, less than 200 ng, less than 100 ng, less than 50 ng or less than 20 ng *(e.g.,* 10 ng or less).

In still other embodiments, the nucleic acid sample used to generate the library includes RNA or cDNA derived from RNA. In some embodiments, the RNA includes total cellular RNA. In other embodiments, certain abundant RNA sequences *(e.g.,* ribosomal RNAs) have been depleted. In some embodiments, the poly(A)-tailed mRNA fraction in the total RNA preparation has been enriched. In some embodiments, the cDNA is produced by random-primed cDNA synthesis methods. In other embodiments, the cDNA synthesis is initiated at the poly(A) tail of mature mRNAs by priming by oligo(dT)-containing oligonucleotides. Methods for depletion, poly(A) enrichment, and cDNA synthesis are well known to those skilled in the art.

The method can further include amplifying the nucleic acid sample *(e.g.,* a DNA or RNA sample) by specific or non-specific nucleic acid amplification methods that are well known to those skilled in the art. In some embodiments, the nucleic acid sample is amplified, e.g., as used with shotgun sequencing methods.

In other embodiments, the nucleic acid sample is fragmented or sheared by physical or enzymatic methods and ligated to synthetic adapters, size-selected *(e.g.,* by preparative gel electrophoresis) and amplified *(e.g.,* by PCR). In other embodiments, the fragmented and adapter-ligated group of nucleic acids is used without explicit size selection or amplification prior to hybrid selection.

In other embodiments, the isolated DNA *(e.g.,* the genomic DNA) is fragmented or sheared. In some embodiments, the library includes less than 50% of genomic DNA, such as a subfraction of genomic DNA that is a reduced representation or a defined portion of a genome, *e.g.,* that has been subfractionated by other means. In other embodiments, the library includes all or substantially all genomic DNA.

In some embodiments, the library includes less than 50% of genomic DNA, such as a subfraction of genomic DNA that is a reduced representation or a defined portion of a genome, *e.g.,* that has been subfractionated by other means. In other embodiments, the library includes all or substantially all genomic DNA. Alternative DNA shearing methods can be more automatable and/or more efficient (*e.g.*, with degraded FFPE samples). Alternatives to DNA shearing methods can also be used to avoid a ligation step during library preparation.

The methods described herein can be performed using a small amount of nucleic acids, *e.g.,* when the amount of source DNA is limiting (*e.g.,* even after whole-genome amplification). In one embodiment, the nucleic acid comprises less than about 5 μg, 4 μg, 3 μg, 2 μg, 1 μg, 0.8 μg, 0.7 μg, 0.6 μg, 0.5 μg, or 400 ng, 300 ng, 200 ng, 100 ng, 50 ng, or 20 ng or less of nucleic acid sample. For example, to prepare 500 ng of hybridization-ready nucleic acids, one typically begins with 3 μg of genomic DNA. One can start with less, however, if one amplifies the genomic DNA (*e.g.,* using PCR) before a hybridization step, e.g., a solution hybridization. Thus, it is possible, but not essential, to amplify the genomic DNA before hybridization, e.g., before solution hybridization.

In some embodiments, a library is generated using DNA (e.g., genomic DNA) from a sample tissue, and a corresponding library is generated with RNA (or cDNA) isolated from the same sample tissue.

In a related aspect, a method for determining the presence of an IGH breakpoint as described herein is provided. The method includes: acquiring a read for a position in a nucleic acid from a sample, e.g., by sequencing at least one nucleotide of the nucleic acid, thereby determining that the IGH breakpoint is present. Optionally, the read acquired is compared to a reference sequence, or a wild-type reference sequence, e.g., to determine that an IGH breakpoint is present, and/or to determine a distance from the IGH breakpoint to the TSS of an oncogene.

In another aspect, the disclosure features a method of analyzing a sample. The method includes acquiring a nucleic acid sample; and sequencing, *e.g.,* by a next generation sequencing method, a nucleic acid, *e.g.,* a nucleic acid that includes an IGH breakpoint as described herein.

A variety of methods are contemplated for use herein to detect an IGH translocation and/or determine a distance between a breakpoint and the TSS of an oncogene. In some embodiments, next-generation sequencing (NGS) can be used to detect a translocation and/or determine a distance between the breakpoint and the TSS of an oncogene, *e.g.,* as exemplified herein. Next-generation sequencing includes any sequencing method that determines the nucleotide sequence of either individual nucleic acid molecules or clonally expanded proxies for individual nucleic acid molecules in a highly parallel fashion (*e.g.,* greater than $10^5$ molecules are sequenced simultaneously). In one embodiment, the relative abundance of the nucleic acid species in the library can be estimated by counting the relative number of occurrences of their cognate sequences in the data generated by the sequencing experiment. Next generation sequencing methods are known in the art, and are described, *e.g.,* in Metzker, M. (2010) Nature Biotechnology Reviews 11:31-46. In one embodiment, the next-generation sequencing allows for the determination of the nucleotide sequence of an individual nucleic acid molecule (*e.g.,* Helicos BioSciences' HeliScope Gene Sequencing system, and Pacific Biosciences' PacBio RS system). In other embodiments, the sequencing method determines the nucleotide sequence of clonally expanded proxies for individual nucleic acid molecules, e.g., massively parallel short-read sequencing, which generates more bases of sequence per sequencing unit than other sequencing methods that generate fewer but longer reads. Other methods or machines for next-generation sequencing include, e.g., the sequencers provided by 454 Life Sciences (Branford, Conn.), Applied Biosystems (Foster City, Calif.; SOLiD sequencer), and Helicos BioSciences Corporation (Cambridge, Mass.). Platforms for next-generation sequencing include, e.g., Roche/454's Genome Sequencer (GS) FLX System, Illumina/Solexa's Genome Analyzer (GA), Illumina's HiSeq 2500, HiSeq 3000, HiSeq 4000 and NovaSeq 6000 Sequencing Systems, Life/APG's Support Oligonucleotide Ligation Detection (SOLiD) system, Polonator's G.007 system, Helicos BioSciences' HeliScope Gene Sequencing system, and Pacific Biosciences' PacBio RS system. NGS technologies can include one or more of steps, *e.g.,* template preparation, sequencing and imaging, and data analysis. Methods for template preparation can include steps such as randomly breaking nucleic acids (*e.g.,* genomic DNA or cDNA) into smaller sizes and generating sequencing templates (*e.g.*, fragment templates or mate-pair templates). The spatially separated templates can be attached or immobilized to a solid surface or support, allowing massive amounts of sequencing reactions to be performed simultaneously. Types of templates that can be used for NGS reactions include, *e.g.*, clonally amplified templates originating from single DNA molecules, and single DNA molecule templates. Exemplary sequencing and imaging steps for NGS include, e.g., cyclic reversible termination (CRT), sequencing by ligation (SBL), single-molecule addition (pyrosequencing), and real-time sequencing. After NGS reads have been generated, they can be aligned to a known reference sequence or assembled *de novo.* For example, identifying genetic variations such as single-nucleotide polymorphism and structural variants in a sample (*e.g.,* a tumor sample) can be accomplished by aligning NGS reads to a reference sequence (*e.g.*, a wildtype sequence). Methods of sequence alignment for NGS are described *e.g.*, in Trapnell C. and Salzberg S.L. Nature Biotech., 2009, 27:455-457. Examples of *de novo* assemblies are described, *e.g.,* in Warren R. et al., Bioinformatics, 2007, 23:500-501; Butler J. et al., Genome Res., 2008, 18:810-820; and Zerbino D.R. and Birney E., Genome Res., 2008, 18:821-829. Sequence alignment or assembly can be performed using read data from one or more NGS platforms, *e.g.*, mixing Roche/454 and Illumina/Solexa read data.

In some embodiments, an IGH translocation and/or a distance between a breakpoint and the TSS of an oncogene is detected in a nucleic acid molecule by a method chosen from one or more of: a nucleic acid hybridization assay, an amplification-based assay (*e.g.*, polymerase chain reaction (PCR)), a PCR-RFLP assay, real-time PCR, sequencing, screening analysis (including metaphase cytogenetic analysis by standard karyotype methods, FISH (e.g., break away FISH), spectral karyotyping or MFISH, comparative genomic hybridization), *in situ* hybridization, SSP, HPLC, or mass-spectrometric genotyping. Additional exemplary methods include, traditional "direct probe" methods such as Southern blots or *in situ* hybridization *(e.g.,* fluorescence in situ hybridization (FISH) and FISH plus SKY), and "comparative probe" methods such as comparative genomic hybridization (CGH), *e.g.*, cDNA-based or oligonucleotide-based CGH. The methods can be used in a wide variety of formats including, but not limited to, substrate (e.g., membrane or glass) bound methods or array-based approaches.

In certain embodiments, the evaluation methods include the probes/primers described herein. In one embodiment, probes/primers can be designed to detect an IGH translocation described herein. Probes/primers are suitable, *e.g.,* for FISH or PCR amplification. For PCR, *e.g.,* to amplify a region including an IGH translocation described herein, forward primers can be designed to hybridize to a gene sequence from nucleotides corresponding to one of the genes or breakpoints of a fusion molecule described herein *(e.g.,* an IGH locus), and reverse primers can be designed to hybridize to a sequence from nucleotides corresponding to the other gene or breakpoint involved in the fusion *(e.g.,* an oncogene or its TSS).

Hybridization protocols suitable for use with the methods featured in the disclosure are described, *e.g.,* in Albertson (1984) EEBO J. 3: 1227-1234; Pinkel (1988) Proc. Natl. Acad. Sci. USA 85: 9138-9142; EPO Pub. No. 430,402, Methods in Molecular Biology, Vol. 33: In situ Hybridization Protocols, Choo, ed., Humana Press, Totowa, N.J. (1994), *etc.*

***Targeted Therapies***

Certain aspects of the present disclosure relate to targeted therapies. In some embodiments, a targeted therapy inhibits expression and/or activity of a protein product of an oncogene of the present disclosure. For example, in some embodiments, a targeted therapy of the present disclosure may inhibit expression and/or activity of a protein product of, or activation of one or more signaling pathways stimulated by, CCND1, MYEOV, MYC, MAF, MAFB, BCL6, BCL2, FGFR3, WHSCI (also known as MMSET and NSD2), BCL3, NBEAPI (encodes BCL8), BCL10, BCL11A, CCND2, CCND3, CCNE1, CD44, CDK6, CEBPA, CEBPB, CEBPD, CEBPE, CRLF2, ID4, DDX6, DUX4, EPOR, GPR34, FCRL4, FCGR2B, IRF4, IRF8, CD274 (encodes PD-L1), PDCD1LG2 (encodes PD-L2), LHX2, LHX4, PAX5, IGF2BP1, TNFSF13, MALT1, FOXP1, IL3, miR-125b-1, MUC1, SOX5, MYCN, or other oncogenes activated by IgH-dependent mechanisms. The terms targeted therapy, therapeutic agent, or agent (when used in relation to therapy) may be used interchangeably herein. Exemplary and non-limiting descriptions of targeted therapies are provided *infra.*

As used herein, the term "CCND1" refers to a gene encoding Cyclin D1 mRNA or polypeptide. CCND1 is also known as BCL1, PRAD1, U21B31, and D11S287E. In some embodiments, a CCND1 gene is a human CCND1 gene. An exemplary CCND1 gene is represented by NCBI Gene ID No. 595, and an exemplary CCND1 polypeptide is represented by NCBI Reference Sequence NP_444284. In some embodiments, a translocation of the present disclosure results in an IGH breakpoint that is 1.3Mb or less from a TSS of CCND1. In some embodiments, a translocation of the present disclosure results in overexpression of CCND1, *e.g.,* 2-fold, 3-fold, 5-fold, 10-fold, or greater than 10-fold overexpression of CCND1.

In some embodiments, a targeted therapy inhibits expression and/or activity of a protein product of CCND1. For example, in some embodiments, the targeted therapy is a cyclin-dependent kinase (CDK) inhibitor, *e.g.*, an inhibitor of CDK4 and/or CDK6. In some embodiments, the targeted therapy is an inhibitor that inhibits CDK4 and CDK6 *(e.g.,* a CDK4/6 inhibitor). Exemplary CDK4/6 inhibitors include, e.g., LEE011 (also known as ribociclib or KISQALI®; Novartis), LY-2835219 (also known as abemaciclib or VERZENIO®; Eli Lilly), PD 0332991 (also known as palbociclib or IBRANCE®; Pfizer), SHR6390, trilaciclib (G1T28), G1T28-1, G1T38, CS3002, HS-10342, and P1446A-05. In one embodiment, the CDK inhibitor inhibits CDK 1, CDK2, CDK7, and/or CDK9. In certain embodiments, the CDK inhibitor is flavopiridol, indisulam, AZD5438, SNS-032, SCH 727965 (Dinaciclib), JNJ-7706621, indirubin, or seliciclib.

As used herein, the term "MYEOV" refers to a gene encoding Myeov mRNA or polypeptide. Myeov, or myeloma overexpressed, is also known as OCIM. In some embodiments, a MYEOV gene is a human MYEOV gene. An exemplary MYEOV gene is represented by NCBI Gene ID No. 26579, and an exemplary MYEOV polypeptide is represented by NCBI Reference Sequence NP_001280220. In some embodiments, a translocation of the present disclosure results in an IGH breakpoint that is 1.3Mb or less from a TSS of MYEOV. In some embodiments, a translocation of the present disclosure results in overexpression of MYEOV, e.g., 2-fold, 3-fold, 5-fold, 10-fold, or greater than 10-fold overexpression of MYEOV.

In some embodiments, a targeted therapy inhibits expression and/or activity of a protein product of MYEOV. In some embodiments, a targeted therapy inhibits expression and/or abundance of a MYEOV RNA, or inhibits an interaction between MYEOV RNA and another RNA, *e.g.,* miR-30c-2-3p.

As used herein, the term "MYC" refers to a gene encoding Myc mRNA or polypeptide. Myc is a proto-oncogene encoding a bHLH transcription factor (the avian myelocytomatosis viral oncogene homolog) that partners with Max and is also known as MRTL, MYCC, c-MYC, and bHLHe39. In some embodiments, a MYC gene is a human MYC gene. An exemplary MYC gene is represented by NCBI Gene ID No. 4609, and an exemplary MYC polypeptide is represented by NCBI Reference Sequence NP_001341799. In some embodiments, a translocation of the present disclosure results in an IGH breakpoint that is 1.3Mb or less from a TSS of MYC. In some embodiments, a translocation of the present disclosure results in overexpression of MYC, *e.g.,* 2-fold, 3-fold, 5-fold, 10-fold, or greater than 10-fold overexpression of MYC.

In some embodiments, a targeted therapy inhibits expression and/or activity of a protein product of MYC. In some embodiments, a targeted therapy inhibits a Myc-Max interaction, or Myc-Max binding to DNA. In some embodiments, a targeted therapy inhibits MYC expression, *e.g.,* by promoting G4 (G-quadruplex structure involving folded guanine tetrads) formation and/or stabilization in a MYC promoter, leading to downregulated MYC transcription, or by promoting epigenetic silencing of MYC *(e.g.,* by inhibiting a histone deacetylase, histone demethylase, histone methyltransferase, DNA methyltransferase, or BET bromodomain). In some embodiments, a targeted therapy alters post-translational modification of Myc, *e.g.*, by targeting Myc phosphorylation/dephosphorylation (*e.g.*, of pS62-MYC), interactions with PP2A or PIN1, or proteasomal degradation. In some embodiments, a MYC inhibitor induces targeting of MYC for degradation via ubiquin ligase activity, such as proteolysis targeting chmieras (PROTAC); *see, e.g.,* Raina, K. et al. (2016) Proc. Natl. Acad. Sci. USA 113:7124 and Winter, G.E. et al. (2015) Science 348:1376-1381. Exemplary MYC inhibitors include, *e.g.,* 10058-F4, 10074-A4, 10074-G5, JY-3-094, 3jc48-3, Mycro3, KJ-Pyr-9, sAJM589, MYCMI-6, MYRA-A, NSC308848, JKY-2-169, KSI-3716, PKUMDL-YC-1201, PKUMDL-YC-1202, PKUMDL-YC-1203, PKUMDL-YC-1204, 7594-0035, VPC-70063, NSC131615, NSC292215, NSC13728, CX-3543, CX-5461, DC-34, IZCZ-3, OmoMYC, SF2523, stauprimide, GQC-05, CZ1, KI-MS2-008, MYCi361, and MYCi975. *See. e.g..* Carabet, L.A. et al. (2019) Int. J. Mol. Sci. 20:120; Petersen, B.L. and Sears, R.C. (2019) BioDrugs 33:539-553; and Han, H. et al. (2019) Cancer Cell 36:483-497.

As used herein, the term "MAF" refers to a gene encoding MAF mRNA or polypeptide. MAF encodes a basic leucine zipper (bZIP) transcription factor and is also known as CCA4, AYGRP, c-MAF, and CTRCT21. In some embodiments, a MAF gene is a human MAF gene. An exemplary MAF gene is represented by NCBI Gene ID No. 4094, and an exemplary MAF polypeptide is represented by NCBI Reference Sequence NP_001026974. In some embodiments, a translocation of the present disclosure results in an IGH breakpoint that is 1.3Mb or less from a TSS of MAF. In some embodiments, a translocation of the present disclosure results in overexpression of MAF, *e.g.,* 2-fold, 3-fold, 5-fold, 10-fold, or greater than 10-fold overexpression of MAF.

In some embodiments, a targeted therapy inhibits expression and/or activity of a protein product of MAF. In some embodiments, a targeted therapy inhibits Maf dimerization *(e.g.,* homodimerization and/or heterodimerization, *e.g.,* with other bZIP proteins such as Jun or Fos) and/or DNA binding. Exemplary MAF inhibitors include, *e.g.,* nivalenol and mebendazole.

As used herein, the term "MAFB" refers to a gene encoding MAFB or Maf-B mRNA or polypeptide. MAFB encodes a bZIP transcription factor and is also known as KRML, MCTO, and DURS3. In some embodiments, a MAFB gene is a human MAFB gene. An exemplary MYC gene is represented by NCBI Gene ID No. 9935, and an exemplary MAFB polypeptide is represented by NCBI Reference Sequence NP_005452. In some embodiments, a translocation of the present disclosure results in an IGH breakpoint that is 1.3Mb or less from a TSS of MAFB. In some embodiments, a translocation of the present disclosure results in overexpression of MAFB, *e.g.,* 2-fold, 3-fold, 5-fold, 10-fold, or greater than 10-fold overexpression of MAFB.

In some embodiments, a targeted therapy inhibits expression and/or activity of a protein product of MAFB. In some embodiments, a targeted therapy inhibits Maf-B dimerization (*e.g.*, homodimerization and/or heterodimerization, *e.g.,* with other bZIP proteins such as Jun or Fos) and/or DNA binding.

As used herein, the term "BCL6" refers to a gene encoding Bcl6 mRNA or polypeptide. BCL6 encodes a zinc finger containing an N-terminal POZ domain and is also known as BCL5, LAZ3, BCL6A, ZNF51, and ZBTB27. In some embodiments, a BCL6 gene is a human BCL6 gene. An exemplary BCL6 gene is represented by NCBI Gene ID No. 604, and an exemplary BCL6 polypeptide is represented by NCBI Reference Sequence NP_001124317. In some embodiments, a translocation of the present disclosure results in an IGH breakpoint that is 1.3Mb or less from a TSS of BCL6. In some embodiments, a translocation of the present disclosure results in overexpression of BCL6, *e.g.,* 2-fold, 3-fold, 5-fold, 10-fold, or greater than 10-fold overexpression of BCL6.

In some embodiments, a targeted therapy inhibits expression and/or activity of a protein product of BCL6. In some embodiments, a BCL6 inhibitor binds BCL6 and blocks recruitment of corepressors (*e.g.*, blocks BCL6:corepressor binding). In some embodiments, a BCL6 inhibitor blocks binding of BCL6 to BCoR, SMRT/NCoR, MTA3, HDAC2, NuRD, or CTBP. In some embodiments, a BCL6 inhibitor induces BCL6 acetylation. BCL6 inhibitors include, *e.g.,* the peptide inhibitors BPI, SMRT, BBD-BPI, L-BPI, RI-BPI, Apt48; 79-6; FX1; Rifamycin SV; Resveratrol; histone deacetylase (HDAC) inhibitors such as romidepsin; and BRD4 inhibitors such as JQ1. *See, e.g.,* Cardenas, M.G. et al. (2017) Clin. Cancer Res. 23:885-893.

As used herein, the term "BCL2" refers to a gene encoding Bcl2 mRNA or polypeptide. BCL2 encodes an integral outer mitochondrial membrane (OMM) protein and is also known as Bcl-2 and PPP1R50. In some embodiments, a BCL2 gene is a human BCL2 gene. An exemplary BCL2 gene is represented by NCBI Gene ID No. 596, and an exemplary BCL2 polypeptide is represented by NCBI Reference Sequence NP_000624. In some embodiments, a translocation of the present disclosure results in an IGH breakpoint that is 1.3Mb or less from a TSS of BCL2. In some embodiments, a translocation of the present disclosure results in overexpression of BCL2, *e.g.,* 2-fold, 3-fold, 5-fold, 10-fold, or greater than 10-fold overexpression of BCL2.

In some embodiments, a targeted therapy inhibits expression and/or activity of a protein product of BCL2. In some embodiments, a BCL2 inhibitor mimics the function of a BH3-only protein that antagonizes the anti-apoptotic activity of Bcl-2, *e.g.,* a BH3-mimetic. In some embodiments, a BCL2 inhibitor inhibits BCL2 expression (*e.g.,* a BCL2 antisense oligonucleotide or an oligonucleotide that binds a sequence near a BLC2 promoter and prevents BCL2 transcription). BCL2 inhibitors include, *e.g.,* venetoclax (also known as ABT-199, VENCLEXTA®, and VENCLYXTO®; Genentech, Abbvie), obatoclax (GX-15-070), HA14-1, ABT-737, navitoclax (ABT-263), oblimersen (also known as G3139, augmerosen, and GENASENSE®), gossypol, AT-101, apogossypol, S1, 2-methoxyantimycin A3, TW37, GDC-0199, BP1002, SPC2996, BGB-11417, LP-108, S55746, S65487, and PNT2258.

As used herein, the term "FGFR3" refers to a gene encoding FGFR3 mRNA or polypeptide. FGFR3 encodes a fibroblast growth factor receptor 3 and is also known as ACH, CEK2, JTK4, CD333, and HSFGFR3EX. In some embodiments, an FGFR3 gene is a human FGFR3 gene. An exemplary FGFR3 gene is represented by NCBI Gene ID No. 2261, and an exemplary FGFR3 polypeptide is represented by NCBI Reference Sequence NP_000133. In some embodiments, a translocation of the present disclosure results in an IGH breakpoint that is 1.3Mb or less from a TSS of FGFR3. In some embodiments, a translocation of the present disclosure results in overexpression of FGFR3, *e.g.,* 2-fold, 3-fold, 5-fold, 10-fold, or greater than 10-fold overexpression of FGFR3.

In some embodiments, a targeted therapy inhibits expression and/or activity of a protein product of FGFR3. In some embodiments, an FGFR3 inhibitor is an anti-FGFR3 antibody or anti-FGFR3 antibody-drug conjugate. In some embodiments, an FGFR3 inhibitor inhibits FGFR3 kinase activity. In some embodiments, an FGFR3 inhibitor is a competitive FGFR3 inhibitor. In some embodiments, an FGFR3 inhibitor binds FGFR3 in an active *(e.g.,* a type I FGFR3 inhibitor) or inactive *(e.g.,* a type II FGFR3 inhibitor) conformation. In some embodiments, an FGFR3 inhibitor is a pan-FGFR inhibitor. In some embodiments, an FGFR3 inhibitor inhibits FGFR3 and one or more of: FGFR1, 2, and 4. In some embodiments, an FGFR3 inhibitor inhibits FGFR1, 2, 3, and 4. In some embodiments, an FGFR3 inhibitor inhibits FGFR1, 2, and 3. Exemplary FGFR3 inhibitors include, *e.g.,* erdafitinib (also known as JNJ-42756493 and BALVERSA®), ponatinib, dovitinib (TKI258), lucitanib, nintedanib (BIBF1120), derazantinib, AZD4547, Ly2874455, CH5183284, BGJ398, BLU-554, H3B-6527, PRN1371, rogaratinib, INCB054828, TAS-120, FGF401, ASP5878, Debio 1347, masitinib, vofatamab (B-701), LY3076226, pemigatinib (PEMAZYRE™,; Incyte), XL999,. *See, e.g.,* Dai, S. *et al.* (2019) *Cells* 8:614.

As used herein, the term "WHSC1" refers to a gene encoding WHSC1 mRNA or polypeptide. WHSC1 encodes a nuclear receptor binding SET domain protein 2 that comprises a PWWP domain, HMG box, SET domain, and PHD-type zinc finger and is also known as NSD2, WHS, TRX5, KMT3F, KMT3G, MMSET, and REIIBP. In some embodiments, a WHSC1 gene is a human WHSC1 gene. An exemplary WHSC1 gene is represented by NCBI Gene ID No. 7468, and an exemplary WHSC1 polypeptide is represented by NCBI Reference Sequence NP_001035889. In some embodiments, a translocation of the present disclosure results in an IGH breakpoint that is 1.3Mb or less from a TSS of WHSC1. In some embodiments, a translocation of the present disclosure results in overexpression of WHSC1, *e.g.,* 2-fold, 3-fold, 5-fold, 10-fold, or greater than 10-fold overexpression of WHSC1.

In some embodiments, a targeted therapy inhibits expression and/or activity of a protein product of WHSC1. In some embodiments, a WHSC1 inhibitor inhibits a histone methyltransferase activity of WHSC1. Exemplary WHSC1 inhibitors include, *e.g.,* PTD2, DA3003-1, PF-03882845, chaetocin, TC LPA5 4, ABT-199, GSK126, and MCTP39. *See, e.g.,* Morrison, M.J. et al. (2018) PLoS One 13:e0197082.

As used herein, the term "BCL3" refers to a gene encoding Bcl3 mRNA or polypeptide. BCL3 encodes a transcriptional coactivator (interacting with, *inter alia,* NFKB) and is also known as BCL4 and D19S37. In some embodiments, a BCL3 gene is a human BCL3 gene. An exemplary BCL3 gene is represented by NCBI Gene ID No. 602, and an exemplary BCL3 polypeptide is represented by NCBI Reference Sequence NP_005169. In some embodiments, a translocation of the present disclosure results in an IGH breakpoint that is 1.3Mb or less from a TSS of BCL3. In some embodiments, a translocation of the present disclosure results in overexpression of BCL3, *e.g.,* 2-fold, 3-fold, 5-fold, 10-fold, or greater than 10-fold overexpression of BCL3.

In some embodiments, a targeted therapy inhibits expression and/or activity of a protein product of BCL3. Exemplary BCL3 inhibitors include, *e.g.,* CB1.

As used herein, the term "BCL8" refers to a gene encoding Bcl8 mRNA or polypeptide. BCL8 encodes a neurobeachin pseudogene and is also known as NBEAP1 and BCL8A. In some embodiments, a BCL8 gene is a human BCL8 gene. An exemplary BCL8 gene is represented by NCBI Gene ID No. 606. In some embodiments, a translocation of the present disclosure results in an IGH breakpoint that is 1.3Mb or less from a TSS of BCL8. In some embodiments, a translocation of the present disclosure results in overexpression of BCL8, *e.g.,* 2-fold, 3-fold, 5-fold, 10-fold, or greater than 10-fold overexpression of BCL8.

In some embodiments, a targeted therapy inhibits expression and/or activity of a protein product of BCL8. In some embodiments, a targeted therapy inhibits expression of a BCL8 gene.

As used herein, the term "BCL10" refers to a gene encoding Bcl10 mRNA or polypeptide. BCL10 encodes an immune signaling adaptor and is also known as CLAP, mEl0, CIPER, IMD37, c-E10, and CARMEN. In some embodiments, a BCL10 gene is a human BCL10 gene. An exemplary BCL10 gene is represented by NCBI Gene ID No. 8915, and an exemplary BCL10 polypeptide is represented by NCBI Reference Sequence NP_001307644. In some embodiments, a translocation of the present disclosure results in an IGH breakpoint that is 1.3Mb or less from a TSS of BCL10. In some embodiments, a translocation of the present disclosure results in overexpression of BCL10, *e.g.,* 2-fold, 3-fold, 5-fold, 10-fold, or greater than 10-fold overexpression of BCL10.

In some embodiments, a targeted therapy inhibits expression and/or activity of a protein product of BCL10. In some embodiments, a BCL10 inhibitor inhibits an activity of a CARMA1-BCL10-MALT1 (CBM) complex. Exemplary BCL10 inhibitors include, *e.g.*, NBP1-76882PEP. *See, e.g.*, Marasco, D. et al. (2009) Biochem J. 422:553-561.

As used herein, the term "BCL11A" refers to a gene encoding Bcl11a mRNA or polypeptide. BCL11A encodes a BAF chromatin remodeling complex subunit and is also known as EV19, CTIP1, DILOS, ZNF856, HBFQTL5, BCL11A-L, BCL11A-S, βCL1la-M, and BCL11A-XL. In some embodiments, a BCL11A gene is a human BCL11A gene. An exemplary BCL11A gene is represented by NCBI Gene ID No. 53335, and an exemplary BCL11A polypeptide is represented by NCBI Reference Sequence NP_001350793. In some embodiments, a translocation of the present disclosure results in an IGH breakpoint that is 1.3Mb or less from a TSS of BCL11A. In some embodiments, a translocation of the present disclosure results in overexpression of BCL11A, *e.g.,* 2-fold, 3-fold, 5-fold, 10-fold, or greater than 10-fold overexpression of BCL11 A.

In some embodiments, a targeted therapy inhibits expression and/or activity of a protein product of BCL11A. In some embodiments, a BCL11A inhibitor inhibits an activity of a BAF chromatin remodeling complex.

As used herein, the term "CCND2" refers to a gene encoding Cyclin D2 mRNA or polypeptide (also known as MPPH3 and KIAK0002). In some embodiments, a CCND2 gene is a human CCND2 gene. An exemplary CCND2 gene is represented by NCBI Gene ID No. 894, and an exemplary CCND2 polypeptide is represented by NCBI Reference Sequence NP_001750. In some embodiments, a translocation of the present disclosure results in an IGH breakpoint that is 1.3Mb or less from a TSS of CCND2. In some embodiments, a translocation of the present disclosure results in overexpression of CCND2, *e.g.*, 2-fold, 3-fold, 5-fold, 10-fold, or greater than 10-fold overexpression of CCND2.

In some embodiments, a targeted therapy inhibits expression and/or activity of a protein product of CCND2. For example, in some embodiments, the targeted therapy is a cyclin-dependent kinase (CDK) inhibitor, *e.g.,* an inhibitor of CDK4 and/or CDK6. In some embodiments, the targeted therapy is an inhibitor that inhibits CDK4 and CDK6 *(e.g.,* a CDK4/6 inhibitor). Exemplary CDK4/6 inhibitors include, e.g., LEE011 (also known as ribociclib or KISQALI®; Novartis), LY-2835219 (also known as abemaciclib or VERZENIO®; Eli Lilly), PD 0332991 (also known as palbociclib or IBRANCE®; Pfizer), SHR6390, trilaciclib (G1T28), G1T28-1, G1T38, CS3002, HS-10342, and P1446A-05. In one embodiment, the CDK inhibitor inhibits CDK1, CDK2, CDK7, and/or CDK9. In certain embodiments, the CDK inhibitor is flavopiridol, indisulam, AZD5438, SNS-032, SCH 727965 (Dinaciclib), JNJ-7706621, indirubin, or seliciclib.

As used herein, the term "CCND3" refers to a gene encoding Cyclin D3 mRNA or polypeptide. In some embodiments, a CCND3 gene is a human CCND3 gene. An exemplary CCND3 gene is represented by NCBI Gene ID No. 896, and an exemplary CCND3 polypeptide is represented by NCBI Reference Sequence NP_001129489. In some embodiments, a translocation of the present disclosure results in an IGH breakpoint that is 1.3Mb or less from a TSS of CCND3. In some embodiments, a translocation of the present disclosure results in overexpression of CCND3, *e.g.,* 2-fold, 3-fold, 5-fold, 10-fold, or greater than 10-fold overexpression of CCND3.

In some embodiments, a targeted therapy inhibits expression and/or activity of a protein product of CCND3. For example, in some embodiments, the targeted therapy is a cyclin-dependent kinase (CDK) inhibitor, *e.g.,* an inhibitor of CDK4 and/or CDK6. In some embodiments, the targeted therapy is an inhibitor that inhibits CDK4 and CDK6 *(e.g.,* a CDK4/6 inhibitor). Exemplary CDK4/6 inhibitors include, e.g., LEE011 (also known as ribociclib or KISQALI®; Novartis), LY-2835219 (also known as abemaciclib or VERZENIO®; Eli Lilly), PD 0332991 (also known as palbociclib or IBRANCE®; Pfizer), SHR6390, trilaciclib (G1T28), G1T28-1, G1T38, CS3002, HS-10342, and P1446A-05. In one embodiment, the CDK inhibitor inhibits CDK1, CDK2, CDK7, and/or CDK9. In certain embodiments, the CDK inhibitor is flavopiridol, indisulam, AZD5438, SNS-032, SCH 727965 (Dinaciclib), JNJ-7706621, indirubin, or seliciclib.

As used herein, the term "CDK6" refers to a gene encoding Cdk6 mRNA or polypeptide and encodes a cyclin-dependent kinase 6 polypeptide (also known as MCPH12 and PLSTIRE). In some embodiments, a CDK6 gene is a human CDK6 gene. An exemplary CDK6 gene is represented by NCBI Gene ID No. 1021, and an exemplary CDK6 polypeptide is represented by NCBI Reference Sequence NP_001138778. In some embodiments, a translocation of the present disclosure results in an IGH breakpoint that is 1.3Mb or less from a TSS of CDK6. In some embodiments, a translocation of the present disclosure results in overexpression of CDK6, *e.g.,* 2-fold, 3-fold, 5-fold, 10-fold, or greater than 10-fold overexpression of CDK6.

In some embodiments, a targeted therapy inhibits expression and/or activity of a protein product of CDK6. For example, in some embodiments, the targeted therapy is a cyclin-dependent kinase (CDK) inhibitor, *e.g.,* an inhibitor of CDK4 and/or CDK6. In some embodiments, the targeted therapy is an inhibitor that inhibits CDK4 and CDK6 *(e.g.,* a CDK4/6 inhibitor). Exemplary CDK4/6 inhibitors include, e.g., LEE011 (also known as ribociclib or KISQALI®; Novartis), LY-2835219 (also known as abemaciclib or VERZENIO®; Eli Lilly), PD 0332991 (also known as palbociclib or IBRANCE®; Pfizer), SHR6390, trilaciclib (G1T28), G1T28-1, G1T38, CS3002, HS-10342, and P1446A-05. In one embodiment, the CDK inhibitor inhibits CDK1, CDK2, CDK7, and/or CDK9. In certain embodiments, the CDK inhibitor is flavopiridol, indisulam, AZD5438, SNS-032, SCH 727965 (Dinaciclib), JNJ-7706621, indirubin, or seliciclib.

As used herein, the term "CCNE1" refers to a gene encoding Ccne1 mRNA or polypeptide and encodes a cyclin E1 polypeptide (also known as CCNE and pCCNE1). In some embodiments, a CCNE1 gene is a human CCNE1 gene. An exemplary CCNEI gene is represented by NCBI Gene ID No. 898, and an exemplary CCNE1 polypeptide is represented by NCBI Reference Sequence NP_001229. In some embodiments, a translocation of the present disclosure results in an IGH breakpoint that is 1.3Mb or less from a TSS of CCNE1. In some embodiments, a translocation of the present disclosure results in overexpression of CCNE1, e.g., 2-fold, 3-fold, 5-fold, 10-fold, or greater than 10-fold overexpression of CCNE1.

In some embodiments, a targeted therapy inhibits expression and/or activity of a protein product of CCNE1 or a cyclin-dependent kinase whose activity is regulated by cyclin E1. For example, in some embodiments, the targeted therapy is a cyclin-dependent kinase (CDK) inhibitor, e.g., an inhibitor ofCDK2. Exemplary CDK2 inhibitors include, e.g., seliciclib (also known as CYC202 and R-roscovitine), GW8510, CYC065, FN-1501, and milciclib maleate. In one embodiment, the CDK inhibitor inhibits CDK1, CDK2, CDK5, CDK7, and/or CDK9. In certain embodiments, the CDK inhibitor is flavopiridol, indisulam, AZD5438, SNS-032, SCH 727965 (Dinaciclib), JNJ-7706621, indirubin, or seliciclib.

As used herein, the term "CD44" refers to a gene encoding CD44 mRNA or polypeptide. CD44 encodes a cell-surface glycoprotein that interacts with ligands such as hyaluronic acid (HA), osteopontin, collagens, and MMPs and is also known as IN, LHR, MC56, MDU2, MDU3, MIC4, Pgp1, CDW44, CSPG8, HCELL, HUTCH-I, and ECMR-III. In some embodiments, a CD44 gene is a human CD44 gene. An exemplary CD44 gene is represented by NCBI Gene ID No. 960, and an exemplary CD44 polypeptide is represented by NCBI Reference Sequence NP_000601. In some embodiments, a translocation of the present disclosure results in an IGH breakpoint that is 1.3Mb or less from a TSS of CD44. In some embodiments, a translocation of the present disclosure results in overexpression of CD44, e.g., 2-fold, 3-fold, 5-fold, 10-fold, or greater than 10-fold overexpression of CD44.

In some embodiments, a targeted therapy inhibits expression and/or activity of a protein product of CD44. In some embodiments, a therapy targeting CD44 is an antibody that binds CD44, e.g., an anti-CD44 antibody, or a chimeric antigen receptor (CAR) targeting CD44 and/or a T cell expressing a CAR targeting CD44. In some embodiments, a therapy targeting CD44 is an inhibitor of CD44-dependent signaling, e.g., activation of RTKs. Exemplary therapies targeting CD44 include, e.g., RO5429083, bivatuzumab and its derivatives (e.g., bivatuzumab mertansine), 4SCAR-CD44v6 T cells, AMC303, A6 peptide, and SPL-108.

As used herein, the term "CEBPA" refers to a gene encoding CEBPA mRNA or polypeptide. CEBPA encodes a CCAAT enhancer binding protein alpha and is also known as CEBP and C/EBP-alpha. In some embodiments, a CEBPA gene is a human CEBPA gene. An exemplary CEBPA gene is represented by NCBI Gene ID No. 1050, and an exemplary CEBPA polypeptide is represented by NCBI Reference Sequence NP_001272758. In some embodiments, a translocation of the present disclosure results in an IGH breakpoint that is 1.3Mb or less from a TSS of CEBPA. In some embodiments, a translocation of the present disclosure results in overexpression of CEBPA, e.g., 2-fold. 3-fold, 5-fold, 10-fold, or greater than 10-fold overexpression of CEBPA.

In some embodiments, a targeted therapy inhibits expression and/or activity of a protein product of CEBPA. In some embodiments, a therapy targeting CEBPA targets downstream signals including CD73, MLL1, and/or A2AR.

As used herein, the term "CEBPB" refers to a gene encoding CEBPB mRNA or polypeptide. CEBPB encodes a CCAAT enhancer binding protein beta and is also known as C/EBP-beta, TCF5, IL6DBP, and NF-IL6. In some embodiments, a CEBPB gene is a human CEBPB gene. An exemplary CEBPB gene is represented by NCBI Gene ID No. 1051, and an exemplary CEBPB polypeptide is represented by NCBI Reference Sequence NP_001272807. In some embodiments, a translocation of the present disclosure results in an IGH breakpoint that is 1.3Mb or less from a TSS of CEBPB. In some embodiments, a translocation of the present disclosure results in overexpression of CEBPB, e.g., 2-fold, 3-fold, 5-fold, 10-fold, or greater than 10-fold overexpression of CEBPB.

In some embodiments, a targeted therapy inhibits expression and/or activity of a protein product of CEBPB. Exemplary CEBPB inhibitors include, e.g., genistein and miR-155.

As used herein, the term "CEBPD" refers to a gene encoding CEBPD mRNA or polypeptide. CEBPD encodes a CCAAT enhancer binding protein delta and is also known as C/EBP-delta, CELF, CRP3, and NF-IL6-beta. In some embodiments, a CEBPD gene is a human CEBPD gene. An exemplary CEBPD gene is represented by NCBI Gene ID No. 1052, and an exemplary CEBPD polypeptide is represented by NCBI Reference Sequence NP_005186. In some embodiments, a translocation of the present disclosure results in an IGH breakpoint that is 1.3Mb or less from a TSS of CEBPD. In some embodiments, a translocation of the present disclosure results in overexpression of CEBPD, e.g., 2-fold, 3-fold, 5-fold, 10-fold, or greater than 10-fold overexpression of CEBPD.

In some embodiments, a targeted therapy inhibits expression and/or activity of a protein product of CEBPD. Exemplary CEBPD inhibitors include, e.g., inotilone and rosmanol.

As used herein, the term "CEBPE" refers to a gene encoding CEBPE mRNA or polypeptide. CEBPE encodes a CCAAT enhancer binding protein epsilon and is also known as C/EBP-epsilon, CRP1, and c/EBP epsilon. In some embodiments, a CEBPE gene is a human CEBPE gene. An exemplary CEBPE gene is represented by NCBI Gene ID No. 1053, and an exemplary CEBPE polypeptide is represented by NCBI Reference Sequence NP_001796. In some embodiments, a translocation of the present disclosure results in an IGH breakpoint that is 1.3Mb or less from a TSS of CEBPE. In some embodiments, a translocation of the present disclosure results in overexpression of CEBPE, *e.g.,* 2-fold, 3-fold, 5-fold, 10-fold, or greater than 10-fold overexpression of CEBPE.

In some embodiments, a targeted therapy inhibits expression and/or activity of a protein product of CEBPE.

As used herein, the term "CRLF2" refers to a gene encoding CRLF2 mRNA or polypeptide. CRLF2 encodes a cytokine receptor-like factor 2 and is also known as CRL2, TSLPR, and CRLF2Y. In some embodiments, a CRLF2 gene is a human CRLF2 gene. An exemplary CRLF2 gene is represented by NCBI Gene ID No. 64109, and an exemplary CRLF2 polypeptide is represented by NCBI Reference Sequence NP_001012288. In some embodiments, a translocation of the present disclosure results in an IGH breakpoint that is 1.3Mb or less from a TSS of CRLF2. In some embodiments, a translocation of the present disclosure results in overexpression of CRLF2, *e.g.,* 2-fold, 3-fold, 5-fold, 10-fold, or greater than 10-fold overexpression of CRLF2.

In some embodiments, a targeted therapy inhibits expression and/or activity of a protein product of CRLF2. In some embodiments, a targeted therapy inhibits expression and/or activity of a ligand of CRLF2, e.g., TSLP (such as the anti-TSLP antibody tezepelumab, also known as MEDI9929 and AMG157. In some embodiments, a targeted therapy inhibits activity of JAK2 *(e.g.,* ruxolitinib, BBT594). In some embodiments, a targeted therapy inhibits activity of the Akt/mTOR pathway, *e.g.,* with an inhibitor of Akt and/or mTOR.

As used herein, the term "ID4" refers to a gene encoding ID4 mRNA or polypeptide. ID4 encodes an inhibitor of DNA binding of bHLH transcription factors and is also known as IDB4 and bHLHb27. In some embodiments, a ID4 gene is a human ID4 gene. An exemplary ID4 gene is represented by NCBI Gene ID No. 3400, and an exemplary ID4 polypeptide is represented by NCBI Reference Sequence NP_001537. In some embodiments, a translocation of the present disclosure results in an IGH breakpoint that is 1.3Mb or less from a TSS of ID4. In some embodiments, a translocation of the present disclosure results in overexpression of ID4, *e.g.,* 2-fold, 3-fold, 5-fold, 10-fold, or greater than 10-fold overexpression of ID4.

In some embodiments, a targeted therapy inhibits expression and/or activity of a protein product of ID4. In some embodiments, an ID4 inhibitor inhibits binding of ID4 to another protein, *e.g.,* a bHLH transcription factor.

As used herein, the term "DDX6" refers to a gene encoding DDX6 mRNA or polypeptide. DDX6 encodes a DEAD-box helicase 6 protein and is also known as P54, RCK, HLR2, IDDILF, and Rck/p54. In some embodiments, a DDX6 gene is a human DDX6 gene. An exemplary DDX6 gene is represented by NCBI Gene ID No. 1656, and an exemplary DDX6 polypeptide is represented by NCBI Reference Sequence NP_001244120. In some embodiments, a translocation of the present disclosure results in an IGH breakpoint that is 1.3Mb or less from a TSS of DDX6. In some embodiments, a translocation of the present disclosure results in overexpression of DDX6, *e.g.*, 2-fold, 3-fold, 5-fold, 10-fold, or greater than 10-fold overexpression of DDX6.

In some embodiments, a targeted therapy inhibits expression and/or activity of a protein product of DDX6.

As used herein, the term "DUX4" refers to a gene encoding DUX4 mRNA or polypeptide. DUX4 encodes a double homeobox 4 protein and is also known as DUX4L. In some embodiments, a DUX4 gene is a human DUX4 gene. An exemplary DUX4 gene is represented by NCBI Gene ID No. 100288687, and an exemplary DUX4 polypeptide is represented by NCBI Reference Sequence NP_001280727. In some embodiments, a translocation of the present disclosure results in an IGH breakpoint that is 1.3Mb or less from a TSS of DUX4. In some embodiments, a translocation of the present disclosure results in overexpression of DUX4, *e.g.,* 2-fold, 3-fold, 5-fold, 10-fold, or greater than 10-fold overexpression of DUX4.

In some embodiments, a targeted therapy inhibits expression and/or activity of a protein product of DUX4. Exemplary DUX4 inhibitors include, *e.g.,* losmapimod, a p38 inhibitor, and a p300 inhibitor (*e.g.,* iP300w).

As used herein, the term "EPOR" refers to a gene encoding EPOR mRNA or polypeptide. EPOR encodes an erythropoietin receptor and is also known as EPO-R. In some embodiments, an EPOR gene is a human EPOR gene. An exemplary EPOR gene is represented by NCBI Gene ID No. 2057, and an exemplary EPOR polypeptide is represented by NCBI Reference Sequence NP_000112. In some embodiments, a translocation of the present disclosure results in an IGH breakpoint that is 1.3Mb or less from a TSS of EPOR. In some embodiments, a translocation of the present disclosure results in overexpression of EPOR, *e.g.,* 2-fold, 3-fold, 5-fold, 10-fold, or greater than 10-fold overexpression of EPOR.

In some embodiments, a targeted therapy inhibits expression and/or activity of a protein product of EPOR. In some embodiments, a targeted therapy inhibits binding of EPO to EPOR. Exemplary EPOR inhibitors include, *e.g.,* EMP9, an inhibitor of JAK/STAT signaling *(e.g.,* a JAK inhibitor), anti-EPOR antibodies, anti-EPO antibodies *(e.g.,* MAB-287), and soluble EPOR molecules.

As used herein, the term "GPR34" refers to a gene encoding GPR34 mRNA or polypeptide. GPR34 encodes a G protein-coupled receptor 34 protein and is also known as LYPSR1. In some embodiments, a GPR34 gene is a human GPR34 gene. An exemplary GPR34 gene is represented by NCBI Gene ID No. 2857, and an exemplary GPR34 polypeptide is represented by NCBI Reference Sequence NP_001091048. In some embodiments, a translocation of the present disclosure results in an IGH breakpoint that is 1.3Mb or less from a TSS of GPR34. In some embodiments, a translocation of the present disclosure results in overexpression of GPR34, *e.g.,* 2-fold, 3-fold, 5-fold, 10-fold, or greater than 10-fold overexpression of GPR34.

In some embodiments, a targeted therapy inhibits expression and/or activity of a protein product of GPR34. Methods for identifying inhibitors of GPR34 are described, *e.g.,* in WO2005/059504.

As used herein, the term "FCRL4" refers to a gene encoding FCRL4 mRNA or polypeptide. FCRL4 encodes an Fc receptor-like 4 protein and is also known as FCRH4, IGFP2, IRTA1, and CD307d. In some embodiments, a FCRL4 gene is a human FCRL4 gene. An exemplary FCRL4 gene is represented by NCBI Gene ID No. 83417, and an exemplary FCRL4 polypeptide is represented by NCBI Reference Sequence NP_112572. In some embodiments, a translocation of the present disclosure results in an IGH breakpoint that is 1.3Mb or less from a TSS of FCRL4. In some embodiments, a translocation of the present disclosure results in overexpression of FCRL4, *e.g.,* 2-fold, 3-fold, 5-fold, 10-fold, or greater than 10-fold overexpression of FCRL4.

In some embodiments, a targeted therapy inhibits expression and/or activity of a protein product of FCRL4.

As used herein, the term "FCGR2B" refers to a gene encoding FCGR2B mRNA or polypeptide. FCGR2B encodes an Fc fragment of IgG receptor IIb protein and is also known as CD32, FCG2, CD32B, FCGR2, IGFR2, FCGR2C, and FcRII-c. In some embodiments, a FCGR2B gene is a human FCGR2B gene. An exemplary FCGR2B gene is represented by NCBI Gene ID No. 2213, and an exemplary FCGR2B polypeptide is represented by NCBI Reference Sequence NT_001002273. In some embodiments, a translocation of the present disclosure results in an IGH breakpoint that is 1.3Mb or less from a TSS of FCGR2B. In some embodiments, a translocation of the present disclosure results in overexpression of FCGR2B, *e.g.,* 2-fold, 3-fold, 5-fold, 10-fold, or greater than 10-fold overexpression of FCGR2B.

In some embodiments, a targeted therapy inhibits expression and/or activity of a protein product of FCGR2B. In some embodiments, a therapy targeting FCGR2B comprises an antibody that binds FCGR2B, *e.g.,* an anti-FCGR2B antibody. Exemplary FCGR2B inhibitors include, *e.g.,* BI-1206 and MGD010 (also known as PRV3279).

As used herein, the term "IRF4" refers to a gene encoding IRF4 mRNA or polypeptide. IRF4 encodes an interferon regulatory factor 4 and is also known as MUM1, LSIRF, SHEP8, and NF-EMS. In some embodiments, a IRF4 gene is a human IRF4 gene. An exemplary IRF4 gene is represented by NCBI Gene ID No. 3662, and an exemplary IRF4 polypeptide is represented by NCBI Reference Sequence NP_001182215. In some embodiments, a translocation of the present disclosure results in an IGH breakpoint that is 1.3Mb or less from a TSS of IRF4. In some embodiments, a translocation of the present disclosure results in overexpression of IRF4, *e.g.,* 2-fold, 3-fold, 5-fold, 10-fold, or greater than 10-fold overexpression of IRF4.

In some embodiments, a targeted therapy inhibits expression and/or activity of a protein product of IRF4.

As used herein, the term "IRF8" refers to a gene encoding IRF8 mRNA or polypeptide. IRF8 encodes an interferon regulatory factor 8 and is also known as ICSBP, IRF-8, ICSBP1, IMD32A, IMD32B, and H-ICSBP. In some embodiments, a IRF8 gene is a human IRF8 gene. An exemplary IRF8 gene is represented by NCBI Gene ID No. 3394, and an exemplary IRF8 polypeptide is represented by NCBI Reference Sequence NP_001350836. In some embodiments, a translocation of the present disclosure results in an IGH breakpoint that is 1.3Mb or less from a TSS of IRF8. In some embodiments, a translocation of the present disclosure results in overexpression of IRF8, *e.g.,* 2-fold, 3-fold, 5-fold, 10-fold, or greater than 10-fold overexpression of IRF8.

In some embodiments, a targeted therapy inhibits expression and/or activity of a protein product of IRF8.

As used herein, the term "PD-L1" refers to a gene encoding PD-L1 mRNA or polypeptide. PD-L1 encodes an immune inhibitory receptor ligand (programmed death-ligand 1) and is also known as CD274, B7-H, B7H1, PDL1, hPD-L1, PDCD1L1, and PDCD1LG1. In some embodiments, a PD-L1 gene is a human PD-L1 gene. An exemplary PD-L1 gene is represented by NCBI Gene ID No. 29126, and an exemplary PD-L1 polypeptide is represented by NCBI Reference Sequence NP_001254635. In some embodiments, a translocation of the present disclosure results in an IGH breakpoint that is 1.3Mb or less from a TSS of PD-L1. In some embodiments, a translocation of the present disclosure results in overexpression of PD-L1, *e.g.,* 2-fold, 3-fold, 5-fold, 10-fold, or greater than 10-fold overexpression of PD-L1.

As used herein, the term "PD-L2" refers to a gene encoding PD-L2 mRNA or polypeptide. PD-L2 encodes an immune inhibitory receptor ligand (programmed death-ligand 2) and is also known as CD273, B7DC, Btdc, PDL2, PDCD1LG2, PDCD1L2, and bA574F11.2. In some embodiments, a PD-L2 gene is a human PD-L2 gene. An exemplary PD-L2 gene is represented by NCBI Gene ID No. 80380, and an exemplary PD-L2 polypeptide is represented by NCBI Reference Sequence NP_079515. In some embodiments, a translocation of the present disclosure results in an IGH breakpoint that is 1.3Mb or less from a TSS of PD-L2. In some embodiments, a translocation of the present disclosure results in overexpression of PD-L2, *e.g.,* 2-fold, 3-fold, 5-fold, 10-fold, or greater than 10-fold overexpression of PD-L2.

In some embodiments, a targeted therapy inhibits expression and/or activity of a protein product of PD-L1 and/or PD-L2. In some embodiments, a targeted therapy inhibits binding of PD-L1 to its receptor, PD-1. In some embodiments, a targeted therapy inhibits binding of PD-L2 to its receptor, PD-1. In some embodiments, a targeted therapy comprises an antagonist that binds to PD-L1, PD-L2, or PD-1. In some embodiments, the antagonist is an antibody, a small molecule, a nucleic acid, a polypeptide (*e.g.*, antibody), carbohydrate, a lipid, a metal, or a toxin. In some embodiments, the targeted therapy comprises a PD-1 antagonist, such as an anti-PD-1 antibody or immunoadhesin. Exemplary PD-1 antibodies known in the art include, without limitation, nivolumab (also known as ONO-4538, BMS-936558, MDX-1106, and OPDIVO®, Bristol-Myers Squibb), pembrolizumab (also known as MK-3475, lambrolizumab, and KEYTRUDA®; Merck), MEDI-0680 (AMP-514; AstraZeneca), PDR001 (CAS Registry No. 1859072-53-9; Novartis), REGN2810 (LIBTAYO® or cemiplimab-rwlc; Regeneron), BGB-108 (BeiGene), BGB-A317 (BeiGene), BI 754091, JS-001 (Shanghai Junshi), STI-A1110 (Sorrento), INCSHR-1210 (Incyte), PF-06801591 (Pfizer), TSR-042 (also known as ANB011; Tesaro/AnaptysBio), AM0001 (ARMO Biosciences), ENUM 244C8 (Enumeral Biomedical Holdings), ENUM 388D4 (Enumeral Biomedical Holdings) Other PD-1 antagonists include, e.g., AUNP-12 (PierreFabre/Aurigene). In some embodiments, the PD-1 axis binding antagonist comprises tislelizumab (BGB-A317), BGB-108, STI-A1110, AM0001, BI 754091, sintilimab (IBI308), cetrelimab (JNJ-63723283), toripalimab (JS-001), camrelizumab (SHR-1210, INCSHR-1210, HR-301210), MEDI-0680 (AMP-514), MGA-012 (INCMGA 0012), spartalizumab (PDR00I), PF-06801591, cemiplimab (REGN-2810, REGEN2810), dostarlimab (TSR-042, ANB011), FITC-YT-16 (PD-1 binding peptide), APL-501 or CBT-501 or genolimzumab (GB-226), AB-122, AK105, AMG 404, BCD-100, F520, HLX10, HX008, JTX-4014, LZM009, Sym021, PSB205, AMP-224 (fusion protein targeting PD-1), CX-188 (PD-1 probody), AGEN-2034, GLS-010, budigalimab (ABBV-181), AK-103, BAT-1306, CS-1003, AM-0001, TILT-123, BH-2922, BH-2941, BH-2950, ENUM-244C8, ENUM-388D4, HAB-21, H EISCOI 11-003, IKT-202, MCLA-134, MT-17000, PEGMP-7, PRS-332, RXI-762, STI-1110, VX-M-10, XmAb-23104, AK-112, HLX-20, SSI-361, AT-16201, SNA-01, AB122, PD1-PIK, PF-06936308, RG-7769, CAB PD-1 Abs, AK-123, MEDI-3387, MEDI-5771, 4H1128Z-E27, REMD-288, SG-001, BY-24.3, CB-201, IBI-319, ONCR-177, Max-1, CS-4100, JBI-426, CCC-0701, CCX- 4503, or a derivative thereof. In some embodiments, the targeted therapy comprises a PD-L1 antagonist, such as an anti-PD-L1 antibody or immunoadhesin. Exemplary PD-L1 antibodies known in the art include, without limitation, atezolizumab (also known as MPDL3280A, RG7446, and TECENTRIQ®; Roche), MDX-1105 (BMS-936559; Bristol Myers Squibb), durvalumab (also known as MED14736 and IMFINZI®; Medimmune/AstraZeneca), avelumab (also known as MSB0010718C and BAVENCIO®; Merck KGaA), LY3300054 (Eli Lilly), STI-A1014 (Sorrento), KN035 (Suzhou Alphamab), FAZ053 (Novartis), and CX-072 (CytomX Therapeutics). Other PD-L1 antagonists include, *e.g.,* GS-4224 and CA-170 (also known as AUPM-170). In some embodiments, the PD-L1 axis binding antagonist comprises BGB-A333, SHR-1316 (HTI-1088), CK-301, BMS-936559, envafolimab (KN035, ASC22), CS1001, MDX-1105 (BMS-936559), LY3300054, STI-A1014, FAZ053, CX-072, INCB086550, GNS-1480, CA-170, CK-301, M-7824, HTI-1088 (HTI-131 , SHR-1316), MSB-2311, AK- 106, AVA-004, BBI-801, CA-327, CBA-0710, CBT-502, FPT-155, IKT-201, 1KT-703, 10-103, JS-003, KD-033, KY-1003, MCLA-145, MT-5050, SNA-02, BCD-135, APL-502 (CBT-402 or TQB2450), IMC-001, KD-045, INBRX-105, KN-046, IMC-2102, IMC-2101, KD-005, IMM-2502, 89Zr-CX-072, 89Zr-DFO-6E11, KY-1055, MEDI-1109, MT-5594, SL-279252, DSP-106, Gensci-047, REMD-290, N-809, PRS-344, FS-222, GEN-1046, BH-29xx, FS-118, or a derivative thereof.

As used herein, the term "LHX2" refers to a gene encoding LHX2 mRNA or polypeptide. LHX2 encodes a LIM homeobox 2 protein and is also known as LH2 and hLhx2. In some embodiments, an LHX2 gene is a human LHX2 gene. An exemplary LHX2 gene is represented by NCBI Gene ID No. 9355, and an exemplary LHX2 polypeptide is represented by NCBI Reference Sequence NP_004780. In some embodiments, a translocation of the present disclosure results in an IGH breakpoint that is 1.3Mb or less from a TSS of LHX2. In some embodiments, a translocation of the present disclosure results in overexpression of LHX2, *e.g.,* 2-fold, 3-fold, 5-fold, 10-fold, or greater than 10-fold overexpression of LHX2.

In some embodiments, a targeted therapy inhibits expression and/or activity of a protein product of LHX2. Exemplary LHX2 inhibitors include, *e.g.,* miR-124.

As used herein, the term "LHX4" refers to a gene encoding LHX4 mRNA or polypeptide. LHX4 encodes a LIM homeobox 4 protein and is also known as CPHD4. In some embodiments, an LHX4 gene is a human LHX4 gene. An exemplary LHX4 gene is represented by NCBI Gene ID No. 89884, and an exemplary LHX4 polypeptide is represented by NCBI Reference Sequence NP_203129. In some embodiments, a translocation of the present disclosure results in an IGH breakpoint that is 1.3Mb or less from a TSS of LHX4. In some embodiments, a translocation of the present disclosure results in overexpression of LHX4, *e.g.,* 2-fold, 3-fold, 5-fold, 10-fold, or greater than 10-fold overexpression of LHX4.

In some embodiments, a targeted therapy inhibits expression and/or activity of a protein product of LHX4.

As used herein, the term "PAX5" refers to a gene encoding PAX5 mRNA or polypeptide. PAX5 encodes a paired box 5 transcription factor and is also known as ALL3 and BSAP. In some embodiments, a PAX5 gene is a human PAX5 gene. An exemplary PAX5 gene is represented by NCBI Gene ID No. 5079, and an exemplary PAX5 polypeptide is represented by NCBI Reference Sequence NP_001267476. In some embodiments, a translocation of the present disclosure results in an IGH breakpoint that is 1.3Mb or less from a TSS of PAXS. In some embodiments, a translocation of the present disclosure results in overexpression of PAX5, *e.g.,* 2-fold, 3-fold, 5-fold, 10-fold, or greater than 10-fold overexpression of PAX5.

In some embodiments, a targeted therapy inhibits expression and/or activity of a protein product of PAX5. In some embodiments, a PAX5 inhibitor inhibits activity and/or expression of a signaling pathway component downstream of BCR signaling, *e.g.,* CD79a and/or Syk. In some embodiments, a PAX5 inhibitor inhibits acts by agonizing CD22. *See, e.g.,* Cozma, D. et al. (2007) J. Clin. Invest. 117:2602-2610.

As used herein, the term "IGF2BP1" refers to a gene encoding IGF2BP1 mRNA or polypeptide. IGF2BP1 encodes an insulin-like growth factor 2 mRNA binding protein 1 and is also known as IMP1, ZBP1, CRDBP, IMP-1, CRD-BP, and VICKZ1. In some embodiments, an IGF2BP1 gene is a human IGF2BP1 gene. An exemplary IGF2BP1 gene is represented by NCBI Gene ID No. 10642, and an exemplary IGF2BP1 polypeptide is represented by NCBI Reference Sequence NP_001153895. In some embodiments, a translocation of the present disclosure results in an IGH breakpoint that is 1.3Mb or less from a TSS of IGF2BP1. In some embodiments, a translocation of the present disclosure results in overexpression of IGF2BP1, *e.g.,* 2-fold, 3-fold, 5-fold, 10-fold, or greater than 10-fold overexpression of IGF2BP1.

In some embodiments, a targeted therapy inhibits expression and/or activity of a protein product of IGF2BP1. In some embodiments, an IGF2BP1 inhibitor inhibits expression of IGF2BP1 *(e.g.,* as with antisense oligonucleotides targeting IGF2BP1).

As used herein, the term "TNFSF13" refers to a gene encoding TNFSF13 mRNA or polypeptide. TNFSF13 encodes a TNF superfamily protein that acts as a ligand for TNFRSF17/BCMA and is also known as APRIL, CD256, TALL2, ZTNF2, TALL-2, TNLG7B, TRDL-1, and UNQ383/PRO715. In some embodiments, an TNFSF13 gene is a human TNFSF13 gene. An exemplary TNFSF13 gene is represented by NCBI Gene ID No. 8741, and an exemplary TNFSF13 polypeptide is represented by NCBI Reference Sequence NP_001185551. In some embodiments, a translocation of the present disclosure results in an IGH breakpoint that is 1.3Mb or less from a TSS of TNFSF13. In some embodiments, a translocation of the present disclosure results in overexpression of TNFSF13, *e.g.,* 2-fold, 3-fold, 5-fold, 10-fold, or greater than 10-fold overexpression of TNFSF13.

In some embodiments, a targeted therapy inhibits expression and/or activity of a protein product of TNFSF13. In some embodiments, a targeted therapy inhibits an interaction between TNFSF13 and TNFRSF17/BCMA. In some embodiments, a targeted therapy inhibits APRIL and BAFF. In some embodiments, a therapy targeting TNFSF13 comprises an antibody that binds TNFSF13, *e.g.,* an anti-TNFSF13 or anti-APRIL antibody. Exemplary TNFSF13 inhibitors include, *e.g.,* BION-1301 and atacicept. In some embodiments, a therapy targeting TNFSF13 comprises an inhibitor of BCMA, such as an anti-BCMA antibody, anti-BCMA CAR or T cell expressing an anti-BCMA CAR, and so forth.

As used herein, the term "MALT1" refers to a gene encoding MALT1 mRNA or polypeptide. MALT1 encodes a caspase-like protease and is also known as MLT, MLT1, IMD12, and PCASP1. In some embodiments, a MALT1 gene is a human MALT1 gene. An exemplary MALT1 gene is represented by NCBI Gene ID No. 10892, and an exemplary MALT1 polypeptide is represented by NCBI Reference Sequence NP_006776. In some embodiments, a translocation of the present disclosure results in an IGH breakpoint that is 1.3Mb or less from a TSS of MALT1. In some embodiments, a translocation of the present disclosure results in overexpression of MALT1, *e.g.,* 2-fold, 3-fold, 5-fold, 10-fold, or greater than 10-fold overexpression of MALT1.

In some embodiments, a targeted therapy inhibits expression and/or activity of a protein product of MALT1. Exemplary MALT1 inhibitors include, *e.g.,* JNJ-67856633, mepazine, chlorpromazine, MI-2, beta-lapachone analogs, and oxepinochromenones.

As used herein, the term "FOXP1" refers to a gene encoding FOXP1 mRNA or polypeptide. FOXP1 encodes a forkhead box (FOX) subfamily P transcription factor and is also known as MFH, QRF1, 12CC4, hFKH1B, and HSPC215. In some embodiments, a FOXP1 gene is a human FOXP1 gene. An exemplary FOXP1 gene is represented by NCBI Gene ID No. 27086, and an exemplary FOXP1 polypeptide is represented by NCBI Reference Sequence NP_001012523. In some embodiments, a translocation of the present disclosure results in an IGH breakpoint that is 1.3Mb or less from a TSS of FOXP1. In some embodiments, a translocation of the present disclosure results in overexpression of FOXP1, *e.g.,* 2-fold, 3-fold, 5-fold, 10-fold, or greater than 10-fold overexpression of FOXP1.

In some embodiments, a targeted therapy inhibits expression and/or activity of a protein product of FOXP1.

As used herein, the term "IL3" refers to a gene encoding IL3 mRNA or polypeptide. IL3 encodes a cytokine and is also known as IL-3, MCGF, and MULTI-CSF. In some embodiments, an IL3 gene is a human IL3 gene. An exemplary IL3 gene is represented by NCBI Gene ID No. 3562, and an exemplary IL3 polypeptide is represented by NCBI Reference Sequence NP_000579. In some embodiments, a translocation of the present disclosure results in an IGH breakpoint that is 1.3Mb or less from a TSS of IL3. In some embodiments, a translocation of the present disclosure results in overexpression of IL3, *e.g.,* 2-fold, 3-fold, 5-fold, 10-fold, or greater than 10-fold overexpression of IL3.

In some embodiments, a targeted therapy inhibits expression and/or activity of a protein product of IL3. In some embodiments, a therapy targeting IL3 comprises an antibody that binds IL3, *e.g.,* an anti-IL3 antibody, or a receptor of IL3, *e.g.,* an anti-IL3R antibody. In some embodiments, a therapy targeting IL3 inhibits binding between IL3 and an IL3 receptor, *e.g.,* IL3RA.

As used herein, the term "miR-125b-1" refers to a gene encoding miR-125b-1 RNA. MALT1 encodes a microRNA and is also known as MIRN125B1 and mir-125b-1. In some embodiments, a miR-125b-1 gene is a human miR-125b-1 gene. An exemplary miR-125b-1 gene is represented by NCBI Gene ID No. 406911, and an exemplary miR-125b-1 polynucleotide is represented by NCBI Reference Sequence NR_029671. In some embodiments, a translocation of the present disclosure results in an IGH breakpoint that is 1.3Mb or less from a TSS of miR-125b-1. In some embodiments, a translocation of the present disclosure results in overexpression of miR-125b-1, *e.g.,* 2-fold, 3-fold, 5-fold, 10-fold, or greater than 10-fold overexpression of miR-125b-1.

In some embodiments, a targeted therapy inhibits expression and/or activity of miR-125b-1. In some embodiments, a targeted therapy modulates expression and/or activity of a target of miR-125b-1, *e.g.,* of an mRNA whose expression is regulated by miR-125b-1.

As used herein, the term "MUC1" refers to a gene encoding MUC1 mRNA or polypeptide. MUC1 encodes a membrane-bound mucin polypeptide and is also known as EMA, MCD, PEM, PUM, KL-6, MAM6, MCKD, PEMT, CD227, H23AG, MCKD1, MUC-1, ADMCKD, ADMCKDI, CA 15-3, MUC-1/X, MUC1/ZD, and MUC-1/SEC. In some embodiments, a MUC1 gene is a human MUC1 gene. An exemplary MUC1 gene is represented by NCBI Gene ID No. 4582, and an exemplary MUC1 polypeptide is represented by NCBI Reference Sequence NP_001018016. In some embodiments, a translocation of the present disclosure results in an IGH breakpoint that is 1.3Mb or less from a TSS of MUC1. In some embodiments, a translocation of the present disclosure results in overexpression of MUC1, *e.g.,* 2-fold, 3-fold, 5-fold, 10-fold, or greater than 10-fold overexpression of MUC1.

In some embodiments, a targeted therapy inhibits expression and/or activity of a protein product of MUC1. In some embodiments, a targeted therapy inhibits homodimerization of MUC1. In some embodiments, a therapy targeting MUC1 comprises an antibody that binds MUC1 or a specific glycoform thereof, *e.g.,* an anti-MUC1 antibody, including multi- or bispecific antibodies that target MUC1 and another antigen *(e.g.,* that target MUC1 and an immune cell, such as an anti-CD3/MUC1 antibody). In some embodiments, a therapy targeting MUC1 comprises an antibody that binds the C-terminal domain of MUC1, MUC1-C. In some embodiments, a therapy targeting MUC1 comprises a chimeric antigen receptor (CAR) that binds MUC1 *(e.g.,* an anti-MUC1 CAR), or a T cell or NK cell expressing an anti-MUC1 CAR. In some embodiments, a therapy targeting MUC1 comprises a vaccine targeting MUC1 *(e.g.,* comprising one or more MUC1 antigenic peptide(s) or polynucleotide(s) encoding one or more MUC1 antigenic peptide(s)) with an optional adjuvant *(e.g.,* MUC1 peptide-poly-ICLC vaccine). In some embodiments, a therapy targeting MUC1 comprises a dendritic cell comprising, expressing, and/or loaded with one or more MUC1 antigenic peptide(s) *(e.g.,* Tn-MUC-1), or a T cell *(e. g.,* a CTL) that specifically reacts with, or has been activated by, such a dendritic cell *(e.g.,* MUC1-peptide-DC-CTL). In some embodiments, a therapy targeting MUC1 comprises a virus or viral vector (such as a replication-defective adenovirus or vaccinia virus) encoding one or more human MUC1 peptide(s) or polypeptide(s) (e.g., ETBX-061, rV-DF3/MUC1, or MVA-MUC1-IL2). Exemplary MUC1 inhibitors include, *e.g.,* MUC1 CAR T cells, MUC1 CAR pNK cells, MUC1-poly-ICLC, dendritic cells expressing MUC1 *(e.g.,* Tn-MUC1), ETBX-061, MUC1-peptide-DC-CTL, rV-DF3/MUC1, MVA-MUC1-IL2, anti-CD3/MUC1 bispecific antibody, MUC1-KLH vaccine, GO-203, GO-203-2C, SKM1-02, SKM1-13, SKM1-20, BTH1704, ONT-10, gatipotuzumab (also known as PankoMab-GEX), clivatuzumab and antibody-drug conjugates thereof *(e.g.,* hPAM4-Cide or IMMU-107), PANVAC, TG4010, and tecemotide (L-BLP25).

As used herein, the term "SOX5" refers to a gene encoding SOX5 mRNA or polypeptide. SOX5 encodes an SRY-related HMG-box (SOX) transcription factor and is also known as L-SOX5, LAMSHF, L-SOX5B, and L-SOX5F. In some embodiments, a SOX5 gene is a human SOX5 gene. An exemplary SOX5 gene is represented by NCBI Gene ID No. 6660, and an exemplary SOX5 polypeptide is represented by NCBI Reference Sequence NP_001248343. In some embodiments, a translocation of the present disclosure results in an IGH breakpoint that is 1.3Mb or less from a TSS of SOX5. In some embodiments, a translocation of the present disclosure results in overexpression of SOX5, *e.g.,* 2-fold, 3-fold, 5-fold, 10-fold, or greater than 10-fold overexpression of SOX5.

In some embodiments, a targeted therapy inhibits expression and/or activity of a protein product of SOX5.

As used herein, the term "MYCN" refers to a gene encoding MYCN mRNA or polypeptide. MYCN, the n-Myc proto-oncogene, encodes a bHLH transcription factor and is also known as NMYC, ODED, MODED, N-myc, and bHLHe37. In some embodiments, a MYCN gene is a human MYCN gene. An exemplary MYCN gene is represented by NCBI Gene ID No. 4613, and an exemplary MYCN polypeptide is represented by NCBI Reference Sequence NP_001280157. In some embodiments, a translocation of the present disclosure results in an IGH breakpoint that is 1.3Mb or less from a TSS of MYCN. In some embodiments, a translocation of the present disclosure results in overexpression of MYCN, *e.g.,* 2-fold, 3-fold, 5-fold, 10-fold, or greater than 10-fold overexpression of MYCN.

In some embodiments, a targeted therapy inhibits expression and/or activity of a protein product of MYCN. In some embodiments, a therapy targeting cells that overexpress MYCN comprises a BET inhibitor, including without limitation GSK525762, BMS-986158, OTX015, and JQ1 *(see, e.g.,* Puissant, A. et al. (2013) Cancer Discov. 3:308-323). In some embodiments, a therapy targeting cells that overexpress MYCN comprises an alcohol dehydrogenase inhibitor, *e.g.,* an ALDH18A1 inhibitor such as YG1702 *(see, e.g.,* Guo, Y.F. et al. (2020) Science Translational Medicine 12:eaax8694), or an EZH2 inhibitor such as GSK2816126, E7438, tazemetostat, CPI-1205, GSK343, 3-deazaneplanocin A, EPZ005687, EI1, GSK126, and UNC1999. In some embodiments, a MYC inhibitor of the present disclosure *(e.g.,* as described in greater detail *supra)* is used to target cells that overexpress MYCN, including without limitation 10074-G5 and 10058-F4 *(see, e.g.,* Muller, I. et al. (2014) PLoS One 9:e97285). In some embodiments, a MYCN inhibitor inhibits binding between MYCN and Max. In some embodiments, a MYCN inhibitor inhibits a MYCN-Aurora A complex, such as an Aurora A inhibitor including without limitation CD532 (Calbiochem), alisertib (also known as MLN8237), and CCT137690, as well as antisense oligonucleotides targeting MYCN or Aurora A. In some embodiments, a therapy targeting cells that overexpress MYCN comprises an inhibitor of CKS1B, AHCY, BLM, and/or PKMYT1 (see, e.g., Chayka, O. et al. (2014) J. Biol. Chem. 290:2198-2212). In some embodiments, a therapy targeting cells that overexpress MYCN comprises an inhibitor of ribosomal biogenesis and/or RNA polymerase I, including without limitation CX-5461 and quarfloxin (CX-3543) (see, e.g., Hald, O.H. et al. (2019) Oncogene 38:2800-2813). In some embodiments, a MYCN inhibitor induces targeting of MYCN for degradation via ubiquin ligase activity, such as proteolysis targeting chmieras (PROTAC); see, e.g., Raina, K. et al. (2016) Proc. Natl. Acad. Sci. USA 113:7124 and Winter, G.E. et al. (2015) Science 348:1376-1381. In some embodiments, a MYCN inhibitor inhibits MYCN-mediated transcription, including without limitation CDK7 or PLK1 inhibitors.

In some embodiments, the oncogene is MYC; the targeted therapy comprises a MYC inhibitor; and the cancer is DLBCL, B-cell lymphoma, Burkitt lymphoma, multiple myeloma, ALL, B-ALL, NHL, MCL, mature B cell neoplasm, MPN, skin lymphoma, CLL, or lymphoproliferative disease.

In some embodiments, the oncogene is BCL2; the targeted therapy comprises a BCL2 inhibitor; and the cancer is DLBCL, FL, B-cell lymphoma, ALL, CLL, HL, NHL, bone marrow lymph proliferative disease, or lymphoproliferative disease.

In some embodiments, the oncogene is BCL6; the targeted therapy comprises a BCL6 inhibitor; and the cancer is DLBCL, FL, multiple myeloma, SMZL, NHL, PMBCL, or plasmablastic lymphoma.

In some embodiments, the oncogene is CCND1; the targeted therapy comprises a CDK inhibitor; and the cancer is multiple myeloma, B-cell lymphoma, AML, DLBCL, or FL.

In some embodiments, the oncogene is MAF; the targeted therapy comprises a MAF inhibitor; and the cancer is multiple myeloma, CLL, ALL, AITL, or DLBCL.

In some embodiments, the oncogene is MAFB; the targeted therapy comprises a MAFB inhibitor; and the cancer is multiple myeloma, ALL, B-ALL, or DLBCL.

In some embodiments, the oncogene is WHSC1 or FGFR3; the targeted therapy comprises a WHSC1 inhibitor, an FGFR3 inhibitor, or a pan-FGFR inhibitor; and the cancer is multiple myeloma or DLBCL.

In some embodiments, the targeted therapy comprises a peptide or polypeptide that inhibits expression and/or activity of a protein product of the oncogene. For example, in some embodiments, the peptide or polypeptide binds to the protein product of an oncogene of the present disclosure In some embodiments, the peptide or polypeptide binds to and inhibits one or more functions of the protein product of an oncogene of the present disclosure. In some embodiments, the targeted therapy comprises an antibody that inhibits expression and/or activity of a protein product of the oncogene. For example, in some embodiments, the antibody binds to the protein product of an oncogene of the present disclosure, e.g., an oncogene that is expressed on a cell surface. In some embodiments, the antibody binds to and inhibits one or more functions of the protein product of an oncogene of the present disclosure. In some embodiments, the antibody binds to and inhibits expression of the protein product of an oncogene of the present disclosure, e.g., cell-surface expression. In some embodiments, the antibody binds to the protein product of an oncogene of the present disclosure and induces cell death of a cell expressing the protein product, e.g., via antibody-dependent cellular cytotoxicity (ADCC), antibody-dependent cellular phagocytosis (ADCP), or cell death mediated by a toxin conjugated to the antibody (e.g., as with an antibody-drug conjugate, ADC). In some embodiments, the targeted therapy comprises a polypeptide that inhibits expression and/or activity of a protein product of the oncogene. Exemplary antibodies of the present disclosure are described in greater detail supra.

In some embodiments, the targeted therapy comprises a targeted inhibitor, compound, or small molecule that inhibits expression and/or activity of a protein product of the oncogene. In some embodiments, the targeted therapy comprises a targeted inhibitor, compound, or small molecule that inhibits expression and/or activity of one or more components of an oncogenic signaling pathway that involves the oncogene (e.g., an oncogenic signaling pathway in which the oncogene participates and/or interacts with one or more components thereof). For example, an oncogene that induces signaling via the MAPK pathway could be targeted by an inhibitor acting at any level of the MAPK pathway. In some embodiments, the targeted therapy comprises a compound or small molecule that inhibits one or more enzymatic activities of a protein product of an oncogene described herein, e.g., a competitive or non-competitive inhibitor. Exemplary compounds of the present disclosure are described in greater detail supra.

In some embodiments, a targeted therapy of the present disclosure comprises a nucleic acid that inhibits expression and/or activity of a protein product of an oncogene described herein. For example, in some embodiments, the targeted therapy comprises an antisense nucleic acid, ribozyme, siRNA, shRNA, miRNA, gRNA, or triple helix nucleic acid. In some embodiments, the nucleic acid may inhibit transcription, translation, and/or post-transcriptional stability of an mRNA molecule encoding the oncogene, e.g., via an antisense oligonucleotide described herein. In some embodiments, the nucleic acid may inhibit expression of and/or direct the modification of DNA or RNA encoding the oncogene, resulting in mutation or decreased/eliminated expression, e.g., via RNA editing or CRISPR-Cas9-mediated gene editing. In some embodiments, a targeted therapy inhibits oncogene expression by promoting G4 (G-quadruplex structure involving folded guanine tetrads) formation and/or stabilization in an oncogene promoter, leading to downregulated oncogene transcription.

In some embodiments, the nucleic acids comprise antisense molecules, dsRNAs, siRNAs, shRNAs, ribozymes, gRNAs, or triple helix molecules, which hybridize to a nucleic acid encoding an oncogene described herein, or a transcription regulatory region, and block or reduce mRNA expression of the oncogene. Accordingly, nucleic acid molecules that are nucleic acid inhibitors, e.g., antisense molecules, siRNAs, shRNAs, ribozymes, gRNAs, or triple helix molecules to an oncogene -encoding nucleic acid molecule are provided.

In some embodiments, the targeted therapy is an antisense nucleic acid molecule. An "antisense" nucleic acid can include a nucleotide sequence which is complementary to a "sense" nucleic acid encoding a protein, *e.g.,* complementary to the coding strand of a double-stranded cDNA molecule or complementary to an mRNA sequence. The antisense nucleic acid can be complementary to an entire fusion coding strand, or to only a portion thereof. In another embodiment, the antisense nucleic acid molecule is antisense to a "noncoding region" of the coding strand of a nucleotide sequence encoding an oncogene (e.g., the 5' and 3' untranslated regions). Anti-sense agents can include, for example, from about 8 to about 80 nucleobases (*i.e.,* from about 8 to about 80 nucleotides), *e.g.,* about 8 to about 50 nucleobases, or about 12 to about 30 nucleobases. Antisense compounds include ribozymes, external guide sequence (EGS) oligonucleotides (oligozymes), and other short catalytic RNAs or catalytic oligonucleotides which hybridize to the target nucleic acid and modulate its expression. Antisense compounds can include a stretch of at least eight consecutive nucleobases that are complementary to a sequence in the target gene. An oligonucleotide need not be 100% complementary to its target nucleotide sequence to be specifically hybridizable. An oligonucleotide is specifically hybridizable when binding of the oligonucleotide to the target interferes with the normal function of the target molecule to cause a loss of utility, and there is a sufficient degree of complementarity to avoid non-specific binding of the oligonucleotide to non-target sequences under conditions in which specific binding is desired, *i.e.,* under physiological conditions in the case of *in vivo* assays or therapeutic treatment or, in the case of *in vitro* assays, under conditions in which the assays are conducted.

Hybridization of antisense oligonucleotides with mRNA can interfere with one or more of the normal functions of mRNA. The functions of mRNA to be interfered with include all key functions such as, for example, translocation of the RNA to the site of protein translation, translation of protein from the RNA, splicing of the RNA to yield one or more mRNA species, and any catalytic activity which may be engaged in by the RNA. Binding of specific protein(s) to the RNA may also be interfered with by antisense oligonucleotide hybridization to the RNA.

Exemplary antisense compounds include DNA or RNA sequences that specifically hybridize to the target nucleic acid, *e.g.,* the mRNA encoding an oncogene described herein. The complementary region can extend for between about 8 to about 80 nucleobases. The compounds can include one or more modified nucleobases. Modified nucleobases may include, e.g., 5-substituted pyrimidines such as 5-iodouracil, 5-iodocytosine, and C5-propynyl pyrimidines such as C5-propynylcytosine and C5-propynyluracil. Other suitable modified nucleobases include $N^4$ --($C_1$ -$C_{12}$) alkylaminocytosines and $N^4,N^4$ --($C_1$ -$C_{12}$) dialkylaminocytosines. Modified nucleobases may also include 7-substituted-8-aza-7-deazapurines and 7-substituted-7-deazapurines such as, for example, 7-iodo-7-deazapurines, 7-cyano-7-deazapurines, 7-aminocarbonyl-7-deazapurines. Examples of these include 6-amino-7-iodo-7-deazapurines, 6-amino-7-cyano-7-deazapurines, 6-amino-7-aminocarbonyl-7-deazapurines, 2-amino-6-hydroxy-7-iodo-7-deazapurines, 2-amino-6-hydroxy-7-cyano-7-deazapurines, and 2-amino-6-hydroxy-7-aminocarbonyl-7-deazapurines. Furthermore, $N^6$ --($C_1$ -$C_{12}$) alkylaminopurines and $N^6,N^6$ --($C_1$ -$C_{12}$) dialkylaminopurines, including $N^6$ -methylaminoadenine and $N^6,N^6$ - dimethylaminoadenine, are also suitable modified nucleobases. Similarly, other 6-substituted purines including, for example, 6-thioguanine may constitute appropriate modified nucleobases. Other suitable nucleobases include 2-thiouracil, 8-bromoadenine, 8-bromoguanine, 2-fluoroadenine, and 2-fluoroguanine. Derivatives of any of the aforementioned modified nucleobases are also appropriate. Substituents of any of the preceding compounds may include $C_1$ -$C_{30}$ alkyl, $C_2$ -$C_{30}$ alkenyl, $C_2$ -$C_{30}$ alkynyl, aryl, aralkyl, heteroaryl, halo, amino, amido, nitro, thio, sulfonyl, carboxyl, alkoxy, alkylcarbonyl, alkoxycarbonyl, and the like. Descriptions of other types of nucleic acid agents are also available. See, e.g., U.S. Patent Nos. 4,987,071; 5,116,742; and 5,093,246; Woolf et al. (1992) Proc Natl Acad Sci USA; Antisense RNA and DNA, D.A. Melton, Ed., Cold Spring Harbor Laboratory, Cold Spring Harbor, N.Y. (1988); 89:7305-9; Haselhoff and Gerlach (1988) Nature 334:585-59; Helene, C. (1991) Anticancer Drug Des. 6:569-84; Helene (1992) Ann. N. Y. Acad. Sci. 660:27-36; and Maher (1992) Bioassays 14:807-15.

In yet another embodiment, the antisense nucleic acid molecule is an α-anomeric nucleic acid molecule. An α-anomeric nucleic acid molecule forms specific double-stranded hybrids with complementary RNA in which, contrary to the usual β-units, the strands run parallel to each other (Gaultier et al. (1987) Nucleic Acids. Res. 15:6625-6641). The antisense nucleic acid molecule can also comprise a 2'-o-methylribonucleotide (Inoue et al. (1987) Nucleic Acids Res. 15:6131-6148) or a chimeric RNA-DNA analogue (Inoue et al. (1987) FEBS Lett. 215:327-330).

The antisense nucleic acid molecules are typically administered to a subject (e.g., by direct injection at a tissue site), such that they hybridize with or bind to cellular mRNA and/or genomic DNA encoding an oncogene described herein to thereby inhibit expression of the protein, *e.g.,* by inhibiting transcription and/or translation. Alternatively, antisense nucleic acid molecules can be modified to target selected cells and then be administered systemically. For systemic administration, antisense molecules can be modified such that they specifically bind to receptors or antigens expressed on a selected cell surface, *e.g.,* by linking the antisense nucleic acid molecules to peptides or antibodies which bind to cell surface receptors or antigens. In some embodiments, an antisense nucleic acid is formulated in a delivery vehicle. The antisense nucleic acid molecules can also be delivered to cells using a vector. To achieve sufficient intracellular concentrations of the antisense molecules, vector constructs in which the antisense nucleic acid molecule is placed under the control of a strong RNA polymerase II or RNA polymerase III promoter can be used.

In another embodiment, the nucleic acid inhibitor featured in the disclosure is a ribozyme. A ribozyme having specificity for a nucleic acid encoding an oncogene can include one or more sequences complementary to the nucleotide sequence of an oncogene disclosed herein, and a sequence having known catalytic sequence responsible for mRNA cleavage (*see* U.S. Pat. No. 5,093,246 or Haselhoff and Gerlach (1988) Nature 334:585-591). For example, a derivative of a *Tetrahymena* L-19 IVS RNA can be constructed in which the nucleotide sequence of the active site is complementary to the nucleotide sequence to be cleaved in a fusion-encoding mRNA. See, e.g., Cech et al. U.S. Patent No. 4,987,071; and Cech et al. U.S. Patent No. 5,116,742. Alternatively, fusion mRNA can be used to select a catalytic RNA having a specific ribonuclease activity from a pool of RNA molecules. *See, e.g.,* Bartel, D. and Szostak, J.W. (1993) Science 261:1411-1418.

Inhibition of an oncogene described herein can be accomplished by targeting nucleotide sequences complementary to the regulatory region of the oncogene to form triple helical structures that prevent transcription of the fusion gene in target cells. See generally, Helene, C. (1991) Anticancer Drug Des. 6:569-84; Helene, C. i (1992) Ann. N.Y. Acad. Sci. 660:27-36; and Maher, L.J. (1992) Bioassays 14:807-15. The potential sequences that can be targeted for triple helix formation can be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

In some embodiments, the nucleic acid inhibitor is a dsRNA molecule. dsRNAs having a duplex structure of between about 20 and 23 base pairs, *e.g.,* 21, base pairs are effective at inducing RNA interference (RNAi) (Elbashir et al., (EMBO) 2001, 20:6877-6888). However, others have found that shorter or longer RNA duplex structures can also be effective (Chu and Rana (2007) RNA 14:1714-1719; Kim et al. (2005) Nat Biotech 23:222-226)

In one embodiment, the dsRNA, is unmodified, and does not comprise, e.g., chemical modifications and/or conjugations known in the art or described herein. In another embodiment, the dsRNA, is chemically modified to enhance stability or other beneficial characteristics. The dsRNA can be synthesized and/or modified by methods well established in the art, such as those described in "Current protocols in nucleic acid chemistry," Beaucage, S.L. et al. (Edrs.), John Wiley & Sons, Inc., New York, NY, USA, While a target sequence of a dsRNA can be generally about 15-30 nucleotides, there is wide variation in the suitability of particular sequences in this range for directing cleavage of any given target RNA. Various software packages and the guidelines set out herein provide guidance for the identification of optimal target sequences for any given gene target, but an empirical approach can also be taken in which a "window" or "mask" of a given size (as a non-limiting example, 21 nucleotides) is literally or figuratively (including, *e.g.,* in silico) placed on the target RNA sequence to identify sequences in the size range that can serve as target sequences. By moving the sequence "window" progressively one nucleotide upstream or downstream of an initial target sequence location, the next potential target sequence can be identified, until the complete set of possible sequences is identified for any given target size selected. This process, coupled with systematic synthesis and testing of the identified sequences (using assays as described herein or as known in the art) to identify those sequences that perform optimally can identify those RNA sequences that, when targeted with a dsRNA molecule, mediate the best inhibition of target gene expression. Thus, while the sequences identified herein represent effective target sequences, it is contemplated that further optimization of inhibition efficiency can be achieved by progressively "walking the window" one nucleotide upstream or downstream of the given sequences to identify sequences with equal or better inhibition characteristics.

In some embodiments, the nucleic acid inhibitor is a small interfering ribonucleic acid (siRNA) molecule. siRNAs are small double stranded RNAs (dsRNAs) that optionally include overhangs. For example, the duplex region of an siRNA is about 18 to 25 nucleotides, e.g., about 19, 20, 21, 22, 23, or 24 nucleotides. Typically, the siRNA sequences are exactly complementary to the target mRNA. dsRNAs and siRNAs in particular can be used to silence gene expression in mammalian cells (e.g., human cells). siRNAs also include short hairpin RNAs (shRNAs) with 29-base-pair stems and 2-nucleotide 3' overhangs. See, e.g., Clemens et al. (2000) Proc. Natl. Acad. Sci. USA 97:6499-6503; Billy et al. (2001) Proc. Natl. Sci. USA 98:14428-14433; Elbashir et al. (2001) Nature. 411:494-8; Yang et al. (2002) Proc. Natl. Acad. Sci. USA 99:9942-9947; Siolas et al. (2005), Nat. Biotechnol. 23(2):227-31; U.S. Patent Publication No. 20040086884; U.S. Patent Publication No. 20030166282; U.S. Patent Publication No. 20030143204; U.S. Patent Publication No. 20040038278; and U.S. Patent Publication No. 20030224432.

A nucleic acid inhibitor can be modified to enhance or obtain beneficial characteristics. For example, a nucleic acid inhibitor can be modified at the base moiety, sugar moiety or phosphate backbone to improve, e.g., the stability, hybridization, or solubility of the molecule. For non-limiting examples of synthetic oligonucleotides with modifications see Toulmé (2001) Nature Biotech. 19:17 and Faria et al. (2001) Nature Biotech. 19:40-44. Such phosphoramidite oligonucleotides can be effective antisense agents.

A nucleic acid inhibitor molecule can be modified to include one or more bridged nucleic acids (BNAs). A bridged nucleic acid is a nucleotide bearing a conformationally restricted sugar moiety. Oligonucleotides containing BNAs show high binding affinity with RNA complementary strands, and are more tolerant to endonucleolytic and exonucleolytic degradation (Roongjang, S. et al. (2007) Nucleic Acids Symp Ser (Oxf) 51:113-114). Exemplary BNAs include, e.g., 2'4'-BNA (also known as LNA (see below); 3'-amino2',4'-BNA, 3',4'-BNA; BNA$^{COC}$; BNA$^{NC}$, and BNA$^{(ME)}$. The structure of the BNA will influence the binding affinity of the nucleic acid molecule with complementary single stranded DNA and double stranded DNA, as well as its enzymatic stability against nuclease degradation. The synthesis and purification of BNA molecules can be performed using standard protocols, (e.g., see Imanishi T, et al. (2002) Chem. Commun. 16: 1653-1659).

In some embodiments, the nucleic acid can be modified to generate peptide nucleic acids (see Hyrup B. et al. (1996) Bioorganic & Medicinal Chemistry 4: 5-23). As used herein, the terms "peptide nucleic acid" or "PNA" refers to a nucleic acid mimic, *e.g.,* a DNA or RNA mimic, in which the deoxyribose or ribose phosphate backbone is replaced by a pseudopeptide backbone and only the four natural nucleobases are retained. The neutral backbone of a PNA can allow for specific hybridization to DNA and RNA under conditions of low ionic strength. PNAs of nucleic acid inhibitor molecules can be used in therapeutic and diagnostic applications. For example, PNAs can be used as antisense, antigene, siRNA, or RNAi agents for sequence-specific modulation of gene expression by, for example, inducing transcription or translation arrest or inhibiting replication. PNAs of nucleic acid inhibitor molecules can also be used in the analysis of single base pair mutations in a gene, (*e.g.*, by PNA-directed PCR clamping); as 'artificial restriction enzymes' when used in combination with other enzymes, (*e.g.*, S1 nucleases (Hyrup B. *et al.* (1996) *supra*)); or as probes or primers for DNA sequencing or hybridization (Hyrup B. *et al.* (1996) *supra*; Perry-O'Keefe *supra*).

The synthesis of PNA oligomers can be performed using standard solid phase peptide synthesis protocols as described in Hyrup B. *et al.* (1996) *supra* and Perry-O'Keefe et al. Proc. Natl. Acad. Sci. 93: 14670-675. Representative U.S. patents that teach the preparation of PNA compounds include, e.g., U.S. Patent Nos. 5,539,082; 5,714,331; and 5,719,262, Additional PNA compounds suitable for use in RNA molecules are described in, for example, in Nielsen et al., Science, 1991, 254, 1497-1500.

The nucleic acid inhibitor molecules can also be modified to include one or more locked nucleic acids (LNA). A locked nucleic acid is a nucleotide having a modified sugar moiety in which the sugar moiety comprises an extra bridge connecting the 2' and 4' carbons. This structure effectively "locks" the ribose in the 3'-endo structural conformation. LNA containing nucleic acid molecules possess high affinity to complementary DNA and RNA and improved mismatch discrimination relative to unmodified nucleic acid molecules (Jepson, J., et al. (2004) Oligonucleotides 14:130-146). The addition of locked nucleic acids to siRNAs has been shown to increase siRNA stability in serum, and to reduce off-target effects (Elmen, J. et al. (2005) Nucleic Acids Research 33(1):439-447; Mook, OR. et al. (2007) Mol Canc Ther 6(3):833-843; Grunweller, A. et al. (2003) Nucleic Acids Research 31(12):3185-3193). Representative U.S. Patents that teach the preparation of locked nucleic acid nucleotides include, e.g., the following: U.S. Patent Nos. 6,268,490; 6,670,461; 6,794,499; 6,998,484; 7,053,207; 7,084,125; and 7,399,845.

A nucleic acid inhibitor molecule can also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl anal other 8-substituted adenines and guanines, 5-halo, particularly 5-bromo, 5-trifluoromethyl and other 5-substituted uracils and cytosines, 7-methylguanine and 7-methyladenine, 8-azaguanine and 8-azaadenine, 7-deazaguanine and 7-daazaadenine and 3-deazaguanine and 3-deazaadenine. Further nucleobases include those disclosed in U.S. Pat. No. 3,687,808, those disclosed in Modified Nucleosides in Biochemistry, Biotechnology and Medicine, Herdewijn, P. ed. Wiley-VCH, 2008; those disclosed in The Concise Encyclopedia Of Polymer Science And Engineering, pages 858-859, Kroschwitz, J. L, ed. John Wiley & Sons, 1990, those disclosed by Englisch et al., Angewandte Chemie, International Edition, 1991, 30, 613, and those disclosed by Sanghvi, Y S., Chapter 15, dsRNA Research and Applications, pages 289-302, Crooke, S. T. and Lebleu, B., Ed., CRC Press, 1993. Certain of these nucleobases are particularly useful for increasing the binding affinity of the oligomeric compounds featured in the disclosure. These include 5-substituted pyrimidines, 6-azapyrimidines and N-2, N-6 and 0-6 substituted purines, including 2-aminopropyladenine, 5-propynyluracil and 5-propynylcytosine. 5-methylcytosine substitutions have been shown to increase nucleic acid duplex stability by 0.6-1.2°C (Sanghvi, Y. S., Crooke, S. T. and Lebleu, B., Eds., dsRNA Research and Applications, CRC Press, Boca Raton, 1993, pp. 276-278) and are exemplary base substitutions, even more particularly when combined with 2'-O-methoxyethyl sugar modifications.

Potentially stabilizing modifications to the ends of nucleic acid inhibitor molecules can include N- (acetylaminocaproyl)-4-hydroxyprolinol (Hyp-C6-NHAc), N-(caproyl-4-hydroxyprolinol (Hyp-C6), N-(acetyl-4-hydroxyprolinol (Hyp-NHAc), thymidine-2'-0-deoxythymidine (ether), N-(aminocaproyl)-4-hydroxyprolinol (Hyp-C6-amino), 2-docosanoyl-uridine-3"- phosphate, inverted base dT(idT) and others. Disclosure of this modification can be found, for example, in PCT Publication No. WO 2011/005861.

In other embodiments, the nucleic acid inhibitor molecule may include other appended groups such as peptides (*e.g.,* for targeting host cell receptors *in vivo*), or agents facilitating transport across the cell membrane (see, e.g., Letsinger et al. (1989) Proc. Natl. Acad. Sci. USA 86:6553-6556; Lemaitre et al. (1987) Proc. Natl. Acad. Sci. USA 84:648-652; PCT Publication No. WO 88/09810) or the blood-brain barrier (see, e.g., PCT Publication No. WO 89/10134). In addition, oligonucleotides can be modified with hybridization-triggered cleavage agents (see, *e.g.,* Krol et al. (1988) Bio-Techniques 6:958-976) or intercalating agents (*See, e.g.,* Zon (1988) Pharm. Res. 5:539-549). To this end, the oligonucleotide may be conjugated to another molecule, (*e.g.,* a peptide, hybridization triggered cross-linking agent, transport agent, or hybridization-triggered cleavage agent).

In some embodiments, modifications to the oncogene-targeting nucleic acid molecules can include, for example, end modifications, *e.g.,* 5'-end modifications (phosphorylation, conjugation, or inverted linkages) or 3'-end modifications (conjugation, DNA nucleotides, inverted linkages, *etc.*); base modifications, *e.g.,* replacement with stabilizing bases, destabilizing bases, or bases that base pair with an expanded repertoire of partners, removal of bases (abasic nucleotides), or conjugated bases; sugar modifications (*e.g.,* at the 2'-position or 4'-position) or replacement of the sugar; and/or backbone modifications, including modification or replacement of the phosphodiester linkages. Specific examples include, e.g. nucleic acid molecules containing modified backbones or no natural internucleoside linkages. Nucleic acid molecules having modified backbones include, among others, those that do not have a phosphorus atom in the backbone.

Modified nucleic acid backbones include, for example, phosphorothioates, chiral phosphorothioates, phosphorodithioates, phosphotriesters, aminoalkylphosphotriesters, methyl and other alkyl phosphonates including 3'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates having normal 3'-5' linkages, 2'-5'-linked analogs of these, and those having inverted polarity wherein the adjacent pairs of nucleoside units are linked 3'-5' to 5'-3' or 2'-5' to 5'-2'. Various salts, mixed salts and free acid forms are also included.

Modified nucleic acid backbones that do not include a phosphorus atom therein have backbones that are formed by short chain alkyl or cycloalkyl internucleoside linkages, mixed heteroatoms and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and $CH_2$ component parts.

Some embodiments include nucleic acid inhibitor molecules with phosphorothioate backbones and oligonucleosides with heteroatom backbones, and in particular $--CH_2--NH--CH_2-$, $--CH_2--N(CH_3)--O--CH_2--$[known as a methylene (methylimino) or MMI backbone], $--CH_2--O-N(CH_3)--CH_2--$, $--CH_2--N(CH_3)--N(CH_3)--CH_2--$ and $--N(CH_3)--CH_2--CH_2--$[wherein the native phosphodiester backbone is represented as $--O--P--O--CH_2--$] of U.S. Patent No. 5,489,677, and the amide backbones of U.S. Patent No. 5,602,240.

Modified nucleic acid inhibitor molecules can also contain one or more substituted sugar moieties. The nucleic acid, e.g., RNA, molecules can include one of the following at the 2'-position: OH; F; O-, S-, or N-alkyl; O-, S-, or N-alkenyl; O-, S- or N-alkynyl; or O-alkyl-O-alkyl, wherein the alkyl, alkenyl and alkynyl can be substituted or unsubstituted $C_1$ to $C_{10}$ alkyl or $C_2$ to $C_{10}$ alkenyl and alkynyl. Exemplary suitable modifications include $O[(CH_2)_nO]_mCH_3$, $O(CH_2)_{.n}OCH_3$, $O(CH2)_nH_2$, $O(CH_2)_nCH_3$, $O(CH_2)_nONH_2$, and $O(CH_2)_nON[(CH_2)_nCH_3)]_2$, where n and m are from 1 to about 10. In other embodiments, dsRNAs include one of the following at the 2' position: $C_1$ to $C_{10}$ lower alkyl, substituted lower alkyl, alkaryl, aralkyl, O-alkaryl or O-aralkyl, SH, $SCH_3$, OCN, Cl, Br, CN, $CF_3$, $OCF_3$, $SOCH_3$, $SO_2CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a reporter group, an intercalator, a group for improving the pharmacokinetic properties of an RNA molecule, or a group for improving the pharmacodynamic properties of an RNA molecule, and other substituents having similar properties. In some embodiments, the modification includes a 2'-methoxyethoxy (2'-O-$CH_2CH_2OCH_3$, also known as 2'-O-(2-methoxyethyl) or 2'-MOE) (Martin et al., Helv. Chim. Acta, 1995, 78:486-504) *i.e.,* an alkoxy-alkoxy group. Another exemplary modification is 2'-dimethylaminooxyethoxy, *i.e.,* a $O(CH_2)_2ON(CH_3)_2$ group, also known as 2'-DMAOE, and 2'-dimethylaminoethoxyethoxy (also known in the art as 2'-O-dimethylaminoethoxyethyl or 2'-DMAEOE), *i.e.,* 2'-O--$CH_2$--O--$CH_2$--$N(CH_2)_2$.

Other modifications can include 2'-methoxy (2'-$OCH_3$), 2'-aminopropoxy (2'-$OCH_2CH_2CH_2NH_2$) and 2'-fluoro (2'-F). Similar modifications can also be made at other positions on the RNA of an RNA molecule, particularly the 3' position of the sugar on the 3' terminal nucleotide or in 2'-5' linked dsRNAs and the 5' position of 5' terminal nucleotide. RNA molecules can also have sugar mimetics such as cyclobutyl moieties in place of the pentofuranosyl sugar.

In some embodiments, a targeted therapy of the present disclosure is administered in combination with another therapy, such as a second therapeutic agent or a different therapeutic modality, *e.g.,* anti-cancer agents, and/or in combination with surgical and/or radiation procedures.

For example, the second therapeutic agent can be a cytotoxic or a cytostatic agent. Exemplary cytotoxic agents include anti-microtubule agents, topoisomerase inhibitors, taxanes, antimetabolites, mitotic inhibitors, alkylating agents, intercalating agents, agents capable of interfering with a signal transduction pathway, and agents that promote apoptosis and radiation. In yet other embodiments, the methods can be used in combination with immunodulatory agents, *e.g.,* IL-1, 2, 4, 6, or 12, or interferon alpha or gamma, or immune cell growth factors such as GM-CSF.

In some embodiments, the second therapeutic agent can be an immunotherapeutic or immunomodulating therapy, *e.g.,* a compound-, antibody-, or cell-based immunotherapy. Examples of immunotherapies include, without limitation, a checkpoint inhibitor, cancer vaccine, cell-based therapy, T cell receptor (TCR)-based therapy, adjuvant immunotherapy, cytokine immunotherapy, or oncolytic virus therapy. In some embodiments, the cancer immunotherapy comprises a small molecule, nucleic acid, polypeptide, carbohydrate, toxin, cell-based, or binding agent therapeutic agent. Examples of cancer immunotherapies are described in greater detail *infra* but are not intended to be limiting.

In some embodiments, the cancer immunotherapy comprises a cancer vaccine. In some embodiments, the cancer immunotherapy comprises a cell-based therapy. In some embodiments, the cancer immunotherapy comprises a T cell-based therapy, *e.g.,* a CD8+ or CD4+ T cell-based therapy. In some embodiments, the cancer immunotherapy comprises an adoptive T cell-based therapy. In some embodiments, the T cells are autologous or allogeneic to the recipient. In some embodiments, the T cell-based therapy comprises a chimeric antigen receptor (CAR)-T-based therapy. This approach involves engineering a CAR that specifically binds to an antigen of interest (*e.g.*, a protein product of an oncogene described herein) and comprises one or more intracellular signaling domains for T cell activation. The CAR is then expressed on the surface of engineered T cells (CAR-T) and administered to a patient, leading to a T-cell-specific immune response against cancer cells expressing the antigen. In some embodiments, the T cell-based therapy comprises T cells expressing a recombinant T cell receptor (TCR). This approach involves identifying a TCR that specifically binds to an antigen of interest, which is then used to replace the endogenous or native TCR on the surface of engineered T cells that are administered to a patient, leading to a T-cell-specific immune response against cancer cells expressing the antigen. In some embodiments, the T cell-based therapy comprises tumor-infiltrating lymphocytes (TILs). For example, TILs can be isolated from a tumor or cancer of the present disclosure, then isolated and expanded *in vitro.* In some embodiments, the cell-based therapy comprises a dendritic cell-based therapy, *e.g.,* a dendritic cell vaccine. Dendritic cell vaccines (such as Sipuleucel-T, also known as APC8015 and PROVENGE®) are vaccines that involve administration of dendritic cells that act as APCs to present one or more cancer-specific antigens, *e.g.,* an oncogene of the present disclosure, to the patient's immune system. In some embodiments, the cancer immunotherapy comprises a TCR-based therapy. In some embodiments, the cancer immunotherapy comprises administration of one or more TCRs or TCR-based biologics that specifically bind an oncogenic protein product of the present disclosure. In some embodiments, the cancer immunotherapy comprises adjuvant immunotherapy. In some embodiments, the cancer immunotherapy comprises cytokine immunotherapy. In some embodiments, the cancer immunotherapy comprises oncolytic virus therapy.

In some embodiments, the cancer immunotherapy comprises a checkpoint inhibitor. As is known in the art, a checkpoint inhibitor targets at least one immune checkpoint protein to alter the regulation of an immune response, e.g, down-modulating or inhibiting an immune response. immune checkpoint proteins include, *e.g..* CTLA4, PD-L1, PD-1, PD-L2, VISTA, B7-H2, B7-H3, B7-H4, B7-H6, 2B4, ICOS, HVEM, CEACAM, LAIR1, CD80, CD86, CD276, VTCN1, MHC class I, MHC class II, GALS, adenosine, TGFR, CSF1R, MICA/B, arginase, CD160, gp49B, PIR-B, KIR family receptors, TIM-1, TIM-3, TIM-4, LAG-3, BTLA, SIRPalpha (CD47), CD48, 2B4 (CD244), B7.1, B7.2, ILT-2, ILT-4, TIGIT, LAG-3, BTLA, IDO, OX40, and A2aR. In some embodiments, a checkpoint inhibitor decreases the activity of a checkpoint protein that negatively regulates immune cell function, *e.g..,* in order to enhance T cell activation and/or an anti-cancer immune response; in other embodiments, a checkpoint inhibitor increases the activity of a checkpoint protein that positively regulates immune cell function, *e.g..,* in order to enhance T cell activation and/or an anti-cancer immune response In some embodiments, the checkpoint inhibitor is an antibody. In some embodiments, the checkpoint inhibitor is an antibody. Examples of checkpoint inhibitors include, without limitation, a PD-L1 axis binding antagonist (e.g., an anti-PD-L1 antibody, e.g., atezolizumab (MPDL3280A)), an antagonist directed against a co-inhibitory molecule (e.g., a CTLA4 antagonist (e.g., an anti-CTLA4 antibody), a TIM-3 antagonist (e.g., an anti-TIM-3 antibody), or a LAG-3 antagonist (e.g., an anti-LAG-3 antibody)), or any combination thereof. In some embodiments, the checkpoint inhibitor is an antagonist of CTLA4, such as a small molecule antagonist of CTLA4 or an anti-CTLA4 antibody, *e.g.,* ipilimumab (YERVOY®, CAS Registry Number: 477202-00-9; also known as BMS-734016, MDX-010, and MDX-101).

Further provided are the targeted therapies of the present disclosure (*e.g.*, a targeted therapy that inhibits expression and/or activity of a protein product of an oncogene of the present disclosure) for use in any of the methods described herein. For example, a targeted therapy of the present disclosure may find use in a method of treating or delaying progression of cancer as described herein.

Yet further provided are the targeted therapies of the present disclosure (*e.g.*, a targeted therapy that inhibits expression and/or activity of a protein product of an oncogene of the present disclosure) for use in the manufacture of a medicament, *e.g.* for use in any of the methods described herein. For example, a targeted therapy of the present disclosure may find use in the manufacture of a medicament, *e.g.* for use in treating or delaying progression of cancer as described herein.

The targeted therapies described herein can be administered by any suitable method, including, for example, intravenously, intramuscularly, subcutaneously, intradermally, percutaneously, intraarterially, intraperitoneally, intralesionally, intracranially, intraarticularly, intraprostatically, intrapleurally, intratracheally, intrathecally, intranasally, intravaginally, intrarectally, topically, intratumorally, peritoneally, subconjunctival, intravesicularly, mucosally, intrapericardially, intraumbilically, intraocularly, intraorbitally, orally, topically, transdermal, intravitreally (e.g., by intravitreal injection), by eye drop, by inhalation, by injection, by implantation, by infusion, by continuous infusion, by localized perfusion bathing target cells directly, by catheter, by lavage, in cremes, or in lipid compositions. The targeted therapies described herein can also be administered systemically or locally. The method of administration can vary depending on various factors (e.g., the compound or composition being administered and the severity of the condition, disease, or disorder being treated). In some instances, the targeted therapy is administered intravenously, intramuscularly, subcutaneously, topically, orally, transdermal, intraperitoneally, intraorbitally, by implantation, by inhalation, intrathecally, intraventricularly, or intranasally. Dosing can be by any suitable route, e.g., by injections, such as intravenous or subcutaneous injections, depending in part on whether the administration is brief or chronic. Various dosing schedules including but not limited to single or multiple administrations over various time-points, bolus administration, and pulse infusion are contemplated herein

Targeted therapies described herein (optionally comprising any additional therapeutic agent) may be formulated, dosed, and administered in a fashion consistent with good medical practice. Factors for consideration in this context include the particular disorder being treated, the particular subject being treated, the clinical condition of the individual patient, the cause of the disorder, the site of delivery of the agent, the method of administration, the scheduling of administration, and other factors known to medical practitioners. The therapeutic agent need not be, but is optionally formulated with and/or administered concurrently with one or more agents currently used to prevent or treat the disorder in question. The effective amount of such other agents depends on the amount of the targeted therapy present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

*Antibody Preparation*

An antibody according to any of the above embodiments may incorporate any of the features, singly or in combination, as described below:

**1. Antibody Affinity**

In certain embodiments, an antibody provided herein has a dissociation constant (Kd) of $\leq 1 \mu M$, $\leq 100$ nM, $\leq 10$ nM, $\leq 1$ nM, $\leq 0.1$ nM, $\leq 0.01$ nM, or $\leq 0.001$ nM (e.g. $10^{-8}$ M or less, e.g. from $10^{-8}$ M to $10^{-13}$ M, e.g., from $10^{-9}$ M to $10^{-13}$ M).

In one embodiment, Kd is measured by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of ($^{125}$I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER$^{®}$ multi-well plates (Thermo Scientific) are coated overnight with 5 $\mu$g/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [$^{125}$I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20$^{®}$) in PBS. When the plates have dried, 150 $\mu$l/well of scintillant (MICROSCINT-20$^{™}$; Packard) is added, and the plates are counted on a TOPCOUNT$^{™}$ gamma counter (Packard) for ten minutes. Concentrations of each Fab that give less than or equal to 20% of maximal binding are chosen for use in competitive binding assays.

According to another embodiment, Kd is measured using a BIACORE$^{®}$ surface plasmon resonance assay. For example, an assay using a BIACORE$^{®}$-2000 or a BIACORE $^{®}$-3000 (BIAcore, Inc., Piscataway, NJ) is performed at 25°C with immobilized antigen CM5 chips at ~10 response units (RU). In one embodiment, carboxymethylated dextran biosensor chips (CM5, BIACORE, Inc.) are activated with *N*-ethyl-*N'*- (3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 $\mu$g/ml (~0.2 $\mu$M) before injection at a flow rate of 5 $\mu$l/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 $\mu$l/min. Association rates ($k_{on}$) and dissociation rates ($k_{off}$) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio $k_{off}/k_{on}$. See, e.g., Chen et al., J. Mol. Biol. 293:865-881 (1999). If the on-rate exceeds 106 M-1 s-1 by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25oC of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO ™ spectrophotometer (ThermoSpectronic) with a stirred cuvette.

## 2. Antibody Fragments

In certain embodiments, an antibody provided herein is an antibody fragment. Antibody fragments include, but are not limited to, Fab, Fab', Fab'-SH, F(ab')$_2$, Fv, and scFv fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies. vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')$_2$ fragments comprising salvage receptor binding epitope residues and having increased in vivo half-life, see U.S. Patent No. 5,869,046.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

## 3. Chimeric and Humanized Antibodies

In certain embodiments, an antibody provided herein is a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g, CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall' Acqua et al., .Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al.J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 4. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HUMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies of the invention may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., FAMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

**6. Multispecific Antibodies**

In certain embodiments, an antibody provided herein is a multispecific antibody, e.g. a bispecific antibody. Multispecific antibodies are monoclonal antibodies that have binding specificities for at least two different sites. In certain embodiments, one of the binding specificities is for an immune checkpoint protein of the present disclosure and the other is for any other antigen. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al.J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to an immune checkpoint protein as well as another, different antigen (see, US 2008/0069820, for example).

**7. Antibody Variants**

In certain embodiments, amino acid sequence variants of the antibodies provided herein are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, e.g., antigen-binding.

**a) Substitution, Insertion, and Deletion Variants**

In certain embodiments, antibody variants having one or more amino acid substitutions are provided. Sites of interest for substitutional mutagenesis include the HVRs and FRs. Conservative substitutions are shown in Table A under the heading of "conservative substitutions." More substantial changes are provided in Table A under the heading of "other exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**TABLE A**

| Original Residue | Other Exemplary Substitutions | Conservative Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gin |
| Asp (D) | Glu; Asn | Glu |
| Cys (C) | Ser; Ala | Ser |
| Gln (Q) | Asn; Glu | Asn |
| Glu (E) | Asp; Gln | Asp |

(continued)

| Original Residue | Other Exemplary Substitutions | Conservative Substitutions |
|---|---|---|
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gin; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu,
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g.* a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

**b) Glycosylation variants**

In certain embodiments, an antibody of the present disclosure is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about $\pm$ 3 amino acids upstream or downstream of position 297, i.e., between positions 294 and 300, due to minor sequence variations in antibodies. Such fucosylation variants may have improved ADCC function. See, e.g., US Patent Publication Nos. US2003/0157108; US2004/0093621. Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570, WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams *et al.*, especially at Example 11), and knockout cell lines, such as alpha-1,6-fucosyltransferase gene, *FUT8,* knockout CHO cells (see, e.g., Yamane-Ohnuki et al. Biotech. Bioeng. 87: 614 (2004); Kanda, Y. et al., Biotechnol. Bioeng., 94(4):680-688 (2006); and WO2003/085107).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

**c) Fc region variants**

In certain embodiments, one or more amino acid modifications may be introduced into the Fc region of an antibody of the present disclosure, thereby generating an Fc region variant. The Fc region variant may comprise a human Fc region sequence (*e.g.*, a human IgG1, IgG2, IgG3 or IgG4 Fc region) comprising an amino acid modification (*e.g.* a substitution) at one or more amino acid positions.

In certain embodiments, the present disclosure contemplates an antibody variant that possesses some but not all effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and *in vivo* clearance/half life determinations can also be performed using methods known in the art (see, e.g., Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e.,* either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

### d) Cysteine engineered antibody variants

In certain embodiments, it may be desirable to create cysteine engineered antibodies, e.g., "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, e.g., in U.S. Patent No. 7,521,541.

### e) Antibody Derivatives

In certain embodiments, an antibody provided herein may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of an antibody and nonproteinaceous moiety that may be selectively heated by exposure to radiation are provided. In one embodiment, the nonproteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

Therapeutic formulations of the immune checkpoint inhibitors, e.g, PD-L1 axis binding antagonists (e.g., an anti-PD-L1 antibody (eg., MPDL3280A)) and antagonists directed against a co-inhibitory molecule (e.g., a CTLA-4 antagonist (e.g., an anti-CTLA-4 antibody), a TIM-3 antagonist (e.g., an anti-TIM-3 antibody), or a LAG-3 antagonist (e.g., an anti-LAG-3 antibody)) used in accordance with the present invention are prepared for storage by mixing the antagonist having the desired degree of purity with optional pharmaceutically acceptable carriers, excipients, or stabilizers in the form of lyophilized formulations or aqueous solutions. For general information concerning formulations, see, e.g., Gilman et al. (eds.) The Pharmacological Bases of Therapeutics, 8th Ed., Pergamon Press, 1990; A. Gennaro (ed.). Remington's Pharmaceutical Sciences, 18th Edition, Mack Publishing Co., Pennsylvania, 1990, Avis et al. (eds.) Pharmaceutical Dosage Forms: Parenteral Medications Dekker, New York, 1993, Lieberman et al. (eds.) Pharmaceutical Dosage Forms: Tablets Dekker, New York, 1990; Lieberman et al. (eds.), Pharmaceutical Dosage Forms: Disperse Systems Dekker, New York, 1990, and Walters (ed.) Dermatological and Transdermal Formulations (Drugs and the Pharmaceutical Sciences), Vol 1 19, Marcel Dekker, 2002.

Acceptable carriers, excipients, or stabilizers are non-toxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride, benzalkonium chloride, benzethonium chloride; phenol, butyl or benzyl alcohol; alkyl parabens such as methyl or propyl paraben; catechol, resorcinol; cyclohexanol, 3-pentanol, and m-cresol); low molecular weight (less than about 10 residues) polypeptides: proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium, metal complexes (e.g., Zn-protein complexes); and/or non-ionic surfactants such as TWEEN™, PLURONICS™, or polyethylene glycol (PEG).

The formulation herein may also contain more than one active compound, for example, those with complementary activities that do not adversely affect each other. The type and effective amounts of such medicaments depend, for example, on the amount and type of antagonist present in the formulation, and clinical parameters of the subjects.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition. Osol, A. Ed. (1980).

Sustained-release preparations may be prepared Suitable examples of sustained-release preparations include semi-permeable matrices of solid hydrophobic polymers containing the antagonist, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,91 9), copolymers of L-glutamic acid and $\gamma$ ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid.

The formulations to be used for in vivo administration must be sterile. This is readily accomplished by filtration through sterile filtration membranes.

It is to be understood that any of the above articles of manufacture may include an immunoconjugate described herein in place of or in addition to an immune checkpoint inhibitor.

## IV. Articles of Manufacture, Kits, and Detection Reagents

Provided herein are kits or articles of manufacture for detecting a presence of a translocation of the present disclosure, *e.g.*, a translocation comprising a breakpoint in the IGH gene/locus. In some embodiments, the kits comprise one or more oligonucleotides. In some embodiments, the one or more oligonucleotides can be used to detect (*e.g.*, in a sample of the present disclosure) an IGH breakpoint and/or oncogene TSS of the present disclosure, or to measure, detect, or infer a distance therebetween. For example, the oligonucleotide(s) may be suitable for detecting a presence of a translocation, IGH breakpoint, or TSS of the present disclosure by fluorescence *in situ* hybridization (FISH).

In some embodiments, the kits or articles of manufacture further comprise instructions, *e.g.,* related to any of the methods described herein. In some embodiments, the instructions are for administering a targeted therapy of the present disclosure, *e.g.*, responsive at least in part to detection (*e.g.*, in a sample of the present disclosure) of a translocation of the present disclosure, such as a translocation comprising a breakpoint in the IGH gene that is 1.3Mb or less from a transcription start site (TSS) of an oncogene of the present disclosure.

A kit featured in the disclosure can include a carrier, *e.g.*, a means being compartmentalized to receive in close confinement one or more container means. In one embodiment the container contains an oligonucleotide, *e.g.*, a primer or probe as described above. The components of the kit are useful, for example, to diagnose or identify a mutation in a tumor sample in a patient. The probe or primer of the kit can be used in any sequencing or nucleotide detection assay known in the art, *e.g.,* a sequencing assay, *e.g.,* an NGS method, RT-PCR, or *in situ* hybridization.

In some embodiments, the components of the kit are useful, for example, to diagnose or identify an IGH breakpoint described herein in a tumor sample in a patient, and to accordingly identify an appropriate therapeutic agent to treat the cancer.

A kit featured in the disclosure can include, *e.g.,* assay positive and negative controls, nucleotides, enzymes (*e.g.,* RNA or DNA polymerase or ligase), solvents or buffers, a stabilizer, a preservative, a secondary antibody, *e.g.,* an anti-HRP antibody (IgG) and a detection reagent.

An oligonucleotide can be provided in any form, *e.g.,* liquid, dried, semi-dried, or lyophilized, or in a form for storage in a frozen condition.

Typically, an oligonucleotide, and other components in a kit are provided in a form that is sterile. An oligonucleotide, *e.g.,* an oligonucleotide that contains a mutation, *e.g.,* a fusion nucleic acid molecule described herein, or an oligonucleotide complementary to a fusion nucleic acid molecule described herein, is provided in a liquid solution, the liquid solution generally is an aqueous solution, *e.g.,* a sterile aqueous solution. When the oligonucleotide is provided as a dried form, reconstitution generally is accomplished by the addition of a suitable solvent. The solvent, *e.g.,* sterile buffer, can optionally be provided in the kit.

The kit can include one or more containers for the composition containing an oligonucleotide in a concentration suitable for use in the assay or with instructions for dilution for use in the assay. In some embodiments, the kit contains separate containers, dividers or compartments for the oligonucleotide and assay components, and the informational material. For example, the oligonucleotides can be contained in a bottle or vial, and the informational material can be contained in a plastic sleeve or packet. In other embodiments, the separate elements of the kit are contained within a single, undivided container. For example, an oligonucleotide composition is contained in a bottle or vial that has attached thereto the informational material in the form of a label. In some embodiments, the kit includes a plurality (*e.g.*, a pack) of individual containers, each containing one or more unit forms (*e.g.,* for use with one assay) of an oligonucleotide. For example, the kit includes a plurality of ampoules, foil packets, or blister packs, each containing a single unit of oligonucleotide for use in sequencing or detecting a mutation in a tumor sample. The containers of the kits can be air tight and/or waterproof. The container can be labeled for use.

In one embodiment, the kit can include informational material for performing and interpreting the sequencing or diagnostic. In another embodiment, the kit can provide guidance as to where to report the results of the assay, e.g., to a treatment center or healthcare provider. The kit can include forms for reporting the results of a sequencing or diagnostic assay described herein, and address and contact information regarding where to send such forms or other related information; or a URL (Uniform Resource Locator) address for reporting the results in an online database or an online application (e.g., an app). In another embodiment, the informational material can include guidance regarding whether a patient should receive treatment with a particular chemotherapeutic drug, depending on the results of the assay.

The informational material of the kits is not limited in its form. In many cases, the informational material, e.g., instructions, is provided in printed matter, e.g., a printed text, drawings, and/or photographs, e.g., a label or printed sheet. However, the informational material can also be provided in other formats, such as computer readable material, video recording, or audio recording. In another embodiment, the informational material of the kit is contact information, *e.g.,* a physical address, email address, website, or telephone number, where a user of the kit can obtain substantive information about the sequencing or diagnostic assay and/or its use in the methods described herein. The informational material can also be provided in any combination of formats.

In some embodiments, a biological sample is provided to an assay provider, *e.g.,* a service provider (such as a third-party facility) or a healthcare provider, who evaluates the sample in an assay and provides a read out. For example, in one embodiment, an assay provider receives a biological sample from a subject, such as a blood or tissue sample, *e.g.,* a biopsy sample or a nucleic acid sample, and evaluates the sample using an assay described herein, *e.g.,* a sequencing assay or *in situ* hybridization assay, and determines whether the sample contains a fusion molecule described herein. The assay provider, *e.g.,* a service provider or healthcare provider, can then conclude that the subject is, or is not, a candidate for a particular drug or a particular cancer treatment regimen.

The assay provider can provide the results of the evaluation, and optionally, conclusions regarding one or more of diagnosis, prognosis, or appropriate therapy options to, for example, a healthcare provider, or patient, or an insurance company, in any suitable format, such as by mail or electronically, or through an online database. The information collected and provided by the assay provider can be stored in a database.

The disclosure also provides nucleic acid molecules useful as probes. Such nucleic acid probes can be designed based on the nucleotide sequence of a fusion nucleic acid molecule described herein.

Probes based on the sequence of a fusion nucleic acid molecule as described herein can be used to detect transcripts or genomic sequences corresponding to one or more fusion nucleic acid molecules featured in the disclosure. The probe typically comprises a label group attached thereto, *e.g.,* a radioisotope, a fluorescent compound, an enzyme, or an enzyme co-factor. Such probes can be used as part of a test kit for identifying cells or tissues which express the fusion protein (*e.g.*, a fusion molecule described herein), such as by measuring levels of a nucleic acid molecule encoding the protein in a sample of cells from a subject, *e.g.*, detecting mRNA levels or determining whether a gene encoding the protein has been mutated or deleted.

Probes featured in the disclosure include those that will specifically hybridize to a nucleotide sequence described herein. Typically, these probes are 12 to 20, *e.g.,* 17 to 20 nucleotides (longer for large insertions). Such molecules can be labeled according to any technique known in the art, such as with radiolabels, fluorescent labels, enzymatic labels, sequence tags, biotin, or other ligands. As used herein, a probe that "specifically hybridizes" to a fusion gene sequence will hybridize under high stringency conditions.

A probe can typically contain a specific mutated or translocated sequence (or a complementary sequence thereof) described herein. Typically, a nucleic acid probe will encompass only one mutation. Such molecules may be labeled and can be used as allele-specific probes to detect the mutation of interest.

In some embodiments, the probe specifically hybridizes to a nucleic acid comprising an inversion resulting in a fusion nucleic acid molecule. In other embodiments, the probe specifically hybridizes to a nucleic acid comprising an inversion resulting in a fusion nucleic acid molecule. In other embodiments, the probe specifically hybridizes to a nucleic acid comprising a deletion resulting in a fusion nucleic acid molecule.

Isolated pairs of allele specific oligonucleotide probes are also provided, where the first probe of the pair specifically hybridizes to the mutant allele, and the second probe of the pair specifically hybridizes to the wildtype allele. Probe pairs can be designed and produced for any of the fusion nucleic acid molecules described herein and are useful in detecting a somatic mutation in a sample.

Probes can be used that contain DNA segments that are essentially complementary to DNA base sequences existing in different portions of chromosomes. Examples of probes useful according to the disclosure, and labeling and hybridization of probes to samples are described in two U.S. patents to Vysis, Inc., U.S. Patent Nos. 5,491,224 and 6,277,569 to Bittner, et al.

EXAMPLES

The invention will be more fully understood by reference to the following examples. They should not, however, be construed as limiting the scope of the invention. It is understood that the examples and embodiments described herein are for illustrative purposes only and that various modifications or changes in light thereof will be suggested to persons skilled in the art and are to be included within the spirit and purview of this application and scope of the appended claims.

***Example 1: Distribution of distances between oncogene transcriptional start sites (TSS's) and IGH breakpoints in cases with IGH translocations***

As noted above, translocations involving the immunoglobulin heavy chain locus (IGH) are observed in various hematological malignancies, and are considered driver alterations in these cancers, as they lead to increased expression of specific oncogenes. However, the relationship between the oncogene overexpression and the distance of the rearranged oncogene from the IGH enhancer region has not been systematically studied. Whether an IGH rearrangement leads to target oncogene overexpression impacts patient care, highlighting the need to understand the distance at which IGH enhancers can effectively alter gene expression.

Recent development of targeted hybridization capture of DNA/RNA coupled with next-generation sequencing (NGS) has enabled reliable concurrent interrogation for the presence of multiple IGH translocations in a single sample with simultaneous analysis of RNA expression levels of oncogenes potentially affected by these translocations in a quantitative manner (He, J. (2016) Blood 127:3004-3014). One advantage of this approach is that the precise positions of translocation breakpoints can be delineated at the nucleotide level resolution.

To probe the distance at which IGH enhancers can effectively alter gene expression, this Example analyzed precise breakpoint locations obtained by targeted next-generation sequencing (NGS) in a large collection of clinical cases profiled in the context of routine care. This study utilized 1,000 hematological cancer cases with detected IGH rearrangements, the largest collection so far described in the literature, in a database obtained in routine clinical care to analyze the distances between IGH translocation breakpoints and the transcription start site (TSS) of corresponding oncogenes, and the impact of that distance on targeted oncogene overexpression.

*Materials and Methods*

**Clinical Samples and FISH Results**

Clinical samples profiled by FoundationOne® Heme testing between June 2013 and July 2019 were analyzed for IGH translocations. FISH analysis interrogating IGH translocations was obtained from the pathology reports accompanying patient samples. When NGS and FISH detected the same IGH translocations, the results were considered as "concordant". When an IGH translocation was detected by one method but not by the other, or when the two methods detected different IGH translocations, the results were considered as "discordant". Cases positive for FISH rearrangements but without specific IGH-involvement were not included in this concordance analysis.

**Comprehensive Genomic Profiling and RNA Expression Measurement**

Comprehensive genomic profiling was performed using the FoundationOne® Heme test that involves hybridization capture of specific DNA and RNA regions, followed by NGS sequencing and assembly The laboratory and computational methods employed in the FoundationOne® Heme assay for IGH-translocation detection, determination of genomic coordination of translocation breakpoints, and normalized RNA expression measurement have been described in detail (He, J. (2016) Blood 127:3004-3014).

**Data Analysis**

For RNA expression analysis, only cases with available RNA data were included. Furthermore, because activation of RAF/RAS pathway has been shown to upregulate expression of oncogenes such as CCND1 and MYC, both in published studies (Kerkhoff, E. and Rapp, U.R. (1997) Mol. Cell. Biol. 17:2576-2586; Li, X.S. et al. (2015) Genet. Mol. Res. 14:10752-10759; Liu, J.J. et al. (1995) Mol. Cell. Biol. 15:3654-3663; Smalley, K.S.M. et al. (2008) Mol. Cancer Ther. 7:2876-2883) and in our dataset (data not shown), cases in our dataset with BRAF, RAF1, KRAS, or NRAS alterations were excluded from expression analysis. In addition, cases with amplification of oncogene involved in a rearrangement with IGH were also excluded from expression analysis.

Normalized RNA levels of oncogenes were measured as part of the FoundationOne® Heme analysis (He, J. (2016) Blood 127:3004-3014). The normalized control RNA levels for each oncogene were calculated as the average of oncogene RNA levels in cancer-type-matched cases without IGH-translocation.

An oncogene was considered as overexpressed when its RNA level was $\geq$ 2-fold than normalized control average expression of the corresponding oncogene in cancer-type-matched cases. Comparing oncogene expression level of each case to the control average expression with the same cancer type eliminated the expression difference of oncogenes between difference cancer types. The percentages of overexpressed cases with the presence of IGH-translocation were then compared with the percentage of cases overexpressing that oncogene when no IGH translocation was detected and with the percentage of cases overexpressing that oncogene when an IGH translocation involving a different chromosome was detected.

**Statistics**

Figures and statistics were generated and calculated in either Microsoft Excel, GraphPad Prism software, or R v3.5.1. The p values are based on the Mann-Whitney U test results.

**Genomic Coordinates**

Genomic coordinates of oncogene TSS's were obtained from Human Genome Browser at UCSC (Kent, W.J. et al. (2002) Genome Res. 12:996-1006). The distance between translocation breakpoints and TSS's were determined by subtracting genomic coordinates of breakpoints from those of TSS's. Absolute values were used when the results were negative.

*Results*

2,129 clinical cases with IGH translocations profiled between June 2013 and July 2019 were identified. For the purpose of this study, 310 cases with 8 well-studied IGH translocations that have documented diagnostic, prognostic, or therapeutic value were selected, including t(3;14) (IGH-BCL6), t(4;14) (IGH-MMSET/FGFR3), t(8;14) (IGH-MYC), t(11;14) (IGH-CCND1), t(14;16) (IGH-MAF), t(14;18) (IGH-BCL2), and t(14;20) (IGH-MAFB). *See* Table 1. In Table 1, the number of IGH translocations and controls that were analyzed in this study are listed by karyotypes. The oncogene involved in each translocation type is listed in the parentheses. Cancer types are listed for each IGH translocation type, with number of cases of each cancer type listed in parentheses.

**Table 1.** IGH translocations analyzed.

| IGH translocations^ (IGH-oncogene) | No. of cases | Cancer Types (number of cases, percentage) |
|---|---|---|
| t(11;14) (IGH-CCND1) | 98 | MM (92, 94%), B-cell lymphoma (NOS) (2, 2%), AML (2, 2%), DLBCL (1, 1%), FL (1, 1%) |
| t(8;14) (IGH-MYC) | 65 | DLBCL (28, 43%), B-Cell lymphoma (NOS) (11, 17%), Burkitt Lymphoma (6, 9%), MM (5, 8%), ALL (3, 5%), B-ALL (3, 5%), NHL (NOS) (2, 3%), MCL (1, 2%), Mature B-Cell neoplasm (NOS) (1, 2%), MPN (1, 2%), Skin lymphoma (1, 2%), CLL (1, 2%), Unknown primary malignant neoplasm (NOS) (1, 2%), Lymphoproliferative disease (NOS) (1, 2%) |
| t(3;14) (IGH-BCL6) | 24 | DLBCL (14, 58.3%), FL (5, 20.8%), MM (1, 4.2%), MZL (1, 4.2%), NHL (NOS) (1, 4.2%), PMBCL (1, 4.2%), Plasmablastic lymphoma (1, 4.2%) |
| t(14;18) (IGH-BCL2) | 58 | DLBCL (25, 43.1%), FL (24, 41.3%), B-cell lymphoma (NOS) (4, 6.7%), ALL (1, 1.7%), CLL (1, 1.7%), HL (1, 1.7%), NHL (NOS) (1, 1.7%), Proliferative disease (NOS) (1, 1.7%) |
| t(14;20) (IGH-MAFB) | 13 | MM (10, 76.9%), ALL (1, 7.7%), B-ALL (1, 7.7%), DLBCL (1, 7.7%) |
| t(4;14) (IGH-MMSET(WHSC1)/FGFR3) | 27 | MM (26, 96.3%), DLBCL (1, 3.7%) |
| t(14;16) (IGH-MAF) | 25 | MM (20, 80%), CLL (2, 8%), ALL (1, 4%), AITL (1, 4%), DLBCL (1, 4%), |
| Total | 310 | N/A |
| **Controls** | No. of cases | Cancer Types (number of cases) |
| No IGH translocation* controls | 347 | Samples were selected to with matched cancer types as corresponding IGH translocations |

^ IGH-involving rearrangement detected by NGS sequencing
* No IGH-involving rearrangement detected by NGS sequencing
ALL: Acute Lymphocytic Lymphoma; AML: Acute Myelocytic Leukemia; AITL: Angioimmunoblastic T-cell lymphoma; B-ALL: B-cell ALL; CLL: Chronic Lymphocytic Lymphoma; DLBCL: Diffuse Large B-Cell Lymphoma; FL: Follicular Lymphoma: HL: Hodgkins Lymphoma; MCL: Mantle Cell Lymphoma; MM: Multiple Myeloma: MPN: Myeloproliferative Neoplasm; MZL: Marginal Zone Lymphoma; NHL: non-Hodgkins lymphoma; NOS: Not Otherwise Specified; PMBCL: Peripheral Mediastinal B-cell Lymphoma

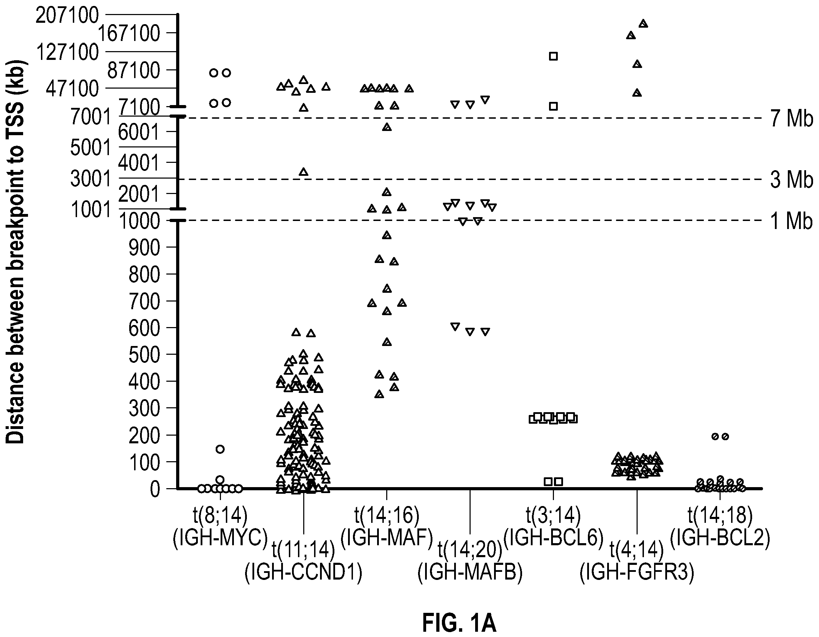

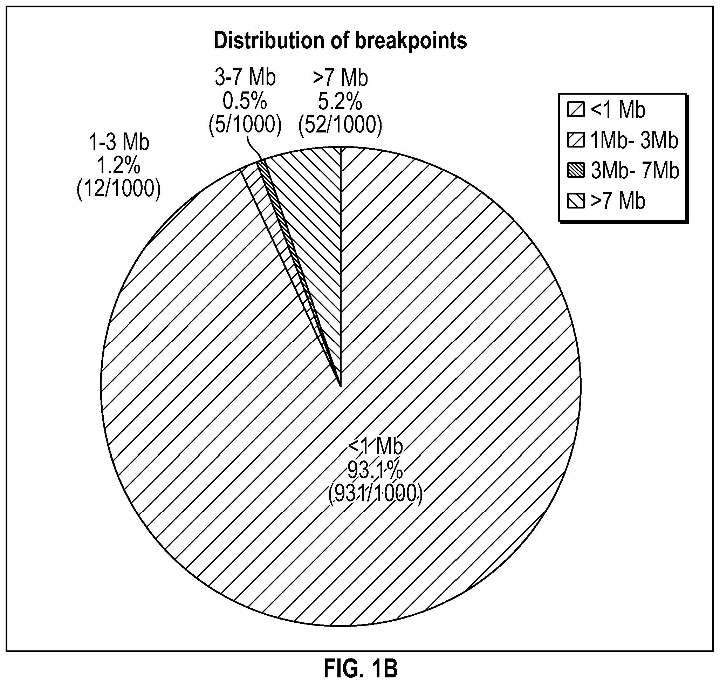

The distance between IGH translocation breakpoints and TSS's of the targeted oncogenes was distributed over a wide range: 87% of the breakpoints were within 1 Mb of TSS's **(FIGS. 1A & 1B)**, consistent with prior findings (Walker, B.A. et al. (2014) Blood Cancer 4:e191; Walker, B.A. et al. (2013) Blood 121:3413-3419). However, 13% of cases showed breakpoints that were >1 Mb from the TSS's of corresponding oncogenes, a phenomenon less appreciated in the literature (**FIGS. 1A & 1B**).

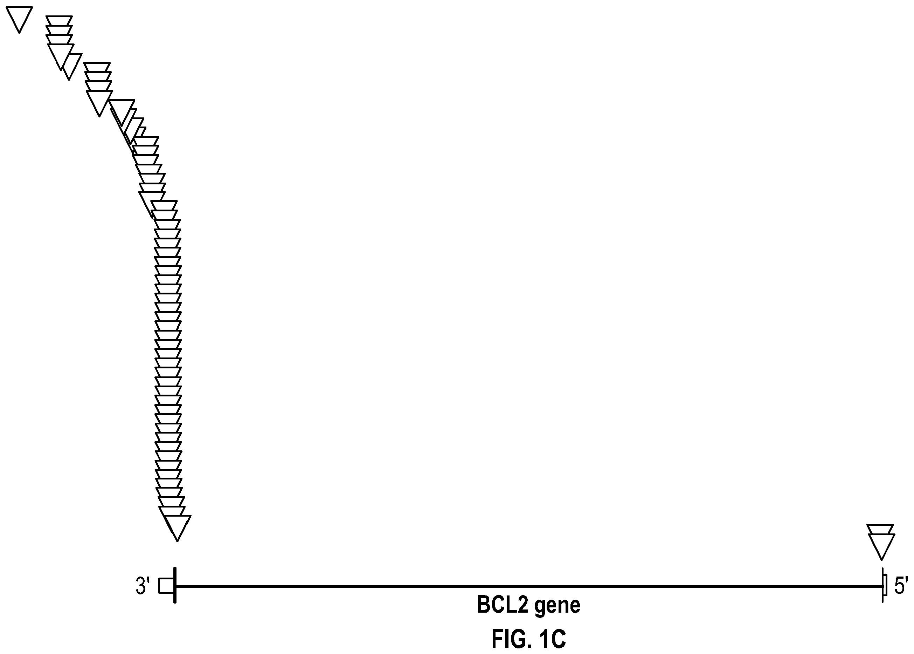

Analyses showed distinct distribution patterns of breakpoint distances for different translocations. Most of the breakpoints in t(8; 14), t(4; 14) and t(14;18) localized within 5 kilobases (kb), 200 kb and 100 kb from TSS's of *MYC, FGFR3,* and *BCL2,* respectively, whereas most of the t(3; 14) breakpoints were clustered at either approximately 1-10 kb or 240 kb from the TSS of *BCL6.* In contrast, most of the t(11; 14), t(14;16) and t(14;20) breakpoints were more evenly spread out within 0-600 kb, 350 kb-3 Mb, and 580 kb-2 Mb, respectively (**FIG**. 1A). Ultra-long distant translocations with breakpoints >7 Mb away from their canonically targeted oncogenes were also identified (**FIG. 1A**), suggesting that these may impact the expression of other genes, although the impact of these events on targeted oncogene expression has not been systematically examined. All breakpoints of t(14;18) were within 300 kb from TSS of *BCL2* (**FIG. 1A**), with the majority of breakpoints located within the *BCL2* 3' UTR, consistent with what has been previously reported (**FIG. 1C**) (Cleary, M.L. et al. (1988) Translocation 47:19-28). The differences of breakpoint distributions between translocations may suggest different mechanisms for breakpoint generation, differential contributions of cognate oncogene regulatory regions and/or differential suite of gene expression-mediating factors in different tumors towards IGH-mediated overexpression.

### Example 2: Oncogene overexpression is highly correlated with distances from the IGH breakpoints

Although IGH translocations have been associated with overexpression of targeted oncogenes, systematic analysis of the relationship between oncogene expression and the distance from the oncogene to the IGH breakpoint has not been described. In addition, not all FISH-detected IGH translocations lead to target oncogene overexpression (Santra, M. et al. (2003) Blood 101 :2374-2376). It was hypothesized that the breakpoints of some translocations may be too far away from the TSS's of oncogenes for the IGH enhancers to drive oncogene overexpression. Alternatively, it is possible that a fraction of translocations failed to overexpress targeted oncogenes regardless of the distance from the oncogene to IGH enhancers.

To explore this question, data from the FoundationOne® Heme-profiled cases with IGH translocations was used, which included both the precise location of translocation breakpoints and the normalized RNA expression levels of oncogenes typically targeted by these translocations, obtained by the DNA and RNA sequencing of the nucleic acids from each cancer sample. In this context, the analysis was focused on t(8;14), t(11;14), t(14; 16) and t(14;20) due to their wide range of distances between IGH translocation breakpoints and targeted oncogene TSSs (**FIG. 1A**).

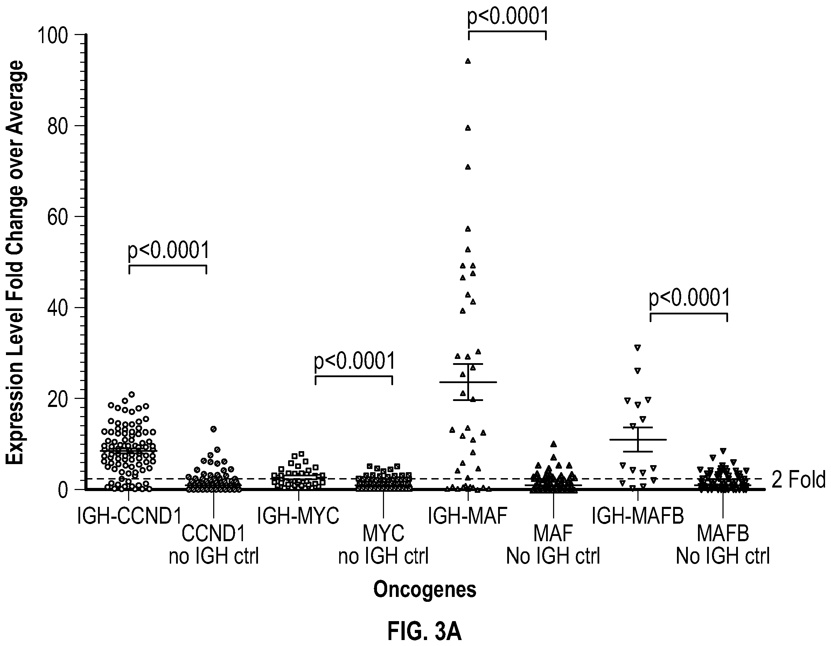

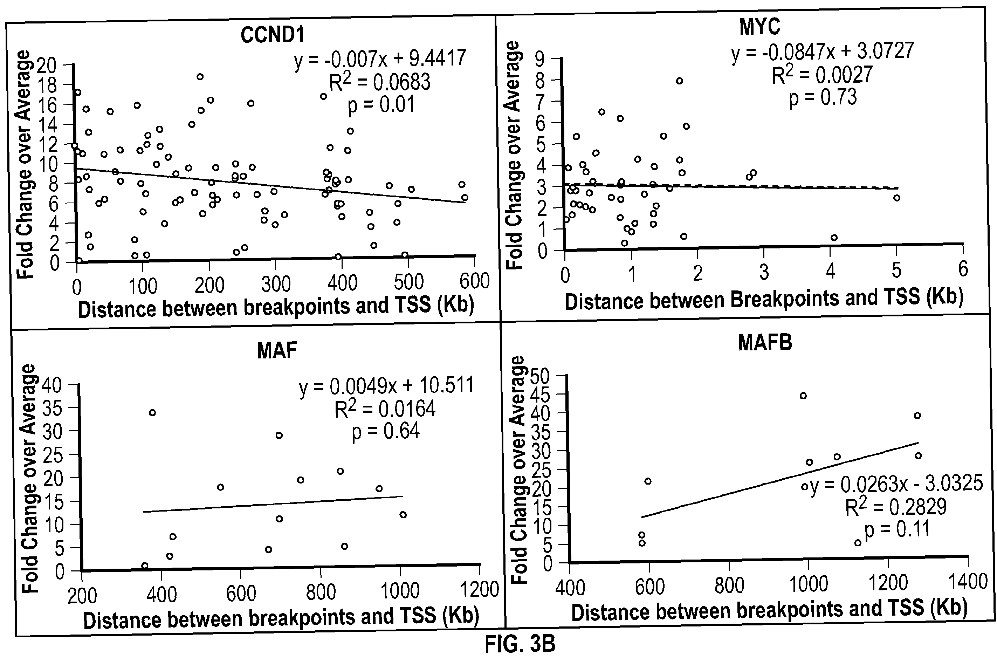

Analysis of disease- and control-normalized RNA expression revealed that in 60-100% of the cases (**FIGS. 2A-2D**), the targeted oncogenes were overexpressed when breakpoints with IGH were less than 1 Mb from TSS's of the corresponding oncogene, while none (0/46) of the translocations with breakpoints $\geq$ 3 Mb away from TSS's were associated with overexpression of the corresponding oncogene. Notably, 12-14% of cases lacking a detected IGH translocation by NGS sequencing also exhibited oncogene overexpression (**FIGS. 2A-2D,** *ctrl No IGH* bars), consistent with IG-independent mechanisms for overexpression observed in the literature for a subset of hematologic malignancies (Affer, M. et al. (2014) Leukemia 28:1725-1735). However, cases harboring IGH translocations featured overexpression of the corresponding oncogenes at a significantly higher incidence (**FIGS. 2A-2E**) and at significantly higher RNA expression levels (**FIGS. 2E & 3**) than the non-IGH controls. Importantly, all (12/12) translocations with breakpoint-oncogene distance between 1 Mb and 3 Mb in cases with t(14;16) and t(14;20) were associated with strong *MAF* and *MAFB* overexpression of 4-fold or higher (**FIG. 2E**, left panel) whereas only 5% (9/173) of controls exhibited *MAF* or *MAFB* overexpression 4-fold or higher and only 15% (27/173) of controls exhibited *MAF* or *MAFB* overexpression of 2-fold or higher (**FIG. 2E**, right panel).

Interestingly, there were no IGH-MYC or IGH-CCND1 cases with IGH breakpoints located between 900KB and 3MB from oncogene TSS (**FIG. 1A**), which may reflect gene-specific preferences for translocation hotspots, possibly due to chromatin regions sensitive to translocations, or may reflect effective boundaries for IGH-mediated upregulation of these oncogenes. Due to the absence of cases with longer distance breakpoints, it is impossible to conclude whether such longer-distance events would lead to *CCND1/MYC* upregulation.

To further define the maximal distance at which IGH enhancers efficiently influence target gene expression, the t(14;16) and t(14;20) MM cases with IGH breakpoints located between 1-3 Mb from TSS's of *MAF* and *MAFB* were examined. In 11 of 12 cases, the translocation breakpoints were within 1-1.3 Mb, with only one case exhibiting a larger distance to the IGH breakpoint (2.1 Mb). As mentioned above, in all 12 cases, *MAF* or *MAFB* genes were strongly overexpressed, with expression levels at least 4-fold higher than the normalized average expression for MM lacking an IG rearrangement (FIG. 2E, left panel). This result suggests the distance of 1.3 Mb from oncogene TSS's to IGH as a functional cutoff for IGH-based overexpression, a distance within which detection of an IGH breakpoint strongly correlates with target oncogene overexpression. Regarding the single case of t(14;16) with IGH breakpoint located 2.1 Mb away from *MAF* that showed *MAF* overexpression, the scarcity of translocations with breakpoints between 1.3 Mb and 3 Mb, as well as the observation that 7.9% of MM samples lacking t(14;16) overexpress *MAF* via alternative mechanisms precludes firm conclusions regarding whether such events drive oncogene overexpression.

When disease- and gene-normalized RNA overexpression data from different translocations were pooled, the percentage of cases with target oncogene overexpression in the 0-1 Mb and 1-1.3 Mb distance ranges was significantly higher than that in the >1.3 Mb or no IGH control groups (**FIG. 2F**), indicating that distance from the translocation breakpoint to the oncogene TSS is an important determinant of IGH-mediated oncogene overexpression. Importantly, these results indicate that translocated IGH enhancers strongly upregulate oncogene expression that are located ≤1.3 Mb from the translocation breakpoints, while they have minimal effect on expression when oncogenes are farther away from the IGH breakpoint (the only overexpressed sample in the >1.3 Mb bucket in **FIG. 2C** is located 2.1 Mb away from MAF TSS, as seen in **FIG. 2B**). Without wishing to be bound to theory, it is thought that IGH rearrangements with breakpoints > 1.3 Mb away from the appreciated oncogenes may instead drive tumorigenesis via de-regulation of other oncogenes.

Interestingly, although oncogenes were likely to be overexpressed when translocation breakpoints were located within the 0-1.3 Mb range from their TSS's, oncogene overexpression levels within this range varied widely (**FIGS. 3A & 3B**). One possible explanation for such varied expression levels is that the amplitude of oncogene expression may be affected by the distance from the IGH breakpoint. To examine this, disease- and control-normalized RNA expression fold change was plotted against distance between translocation breakpoints and TSS for each oncogene (**FIG. 3B**). *CCND1* expression showed a small but significant decrease in expression with increasing distance from the IGH breakpoint, whereas other examined oncogenes (*MYC, MAF,* and *MAFB*) did not show a significant correlation between expression levels and distance, possibly due to insufficient data. This suggests that within the effective distance range, the distance is not a strong determinant of the magnitude of oncogene expression, although some impact may exist for certain genes. To test whether IGH-mediated oncogene overexpression was specific to the targeted oncogene, oncogene expression was assessed in cases where IGH was rearranged with chromosomes other than the one where the oncogene in question is located. The fraction of cases overexpressing oncogenes in these cases was similar to the cases with no detected IGH rearrangements (**FIGS. 2A-2D**), consistent with the specific nature of IGH-mediated oncogene overexpression.

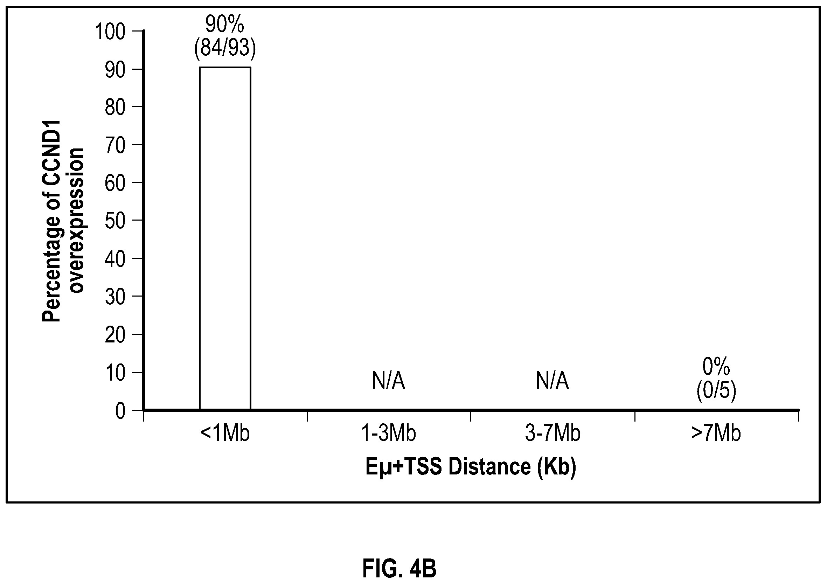

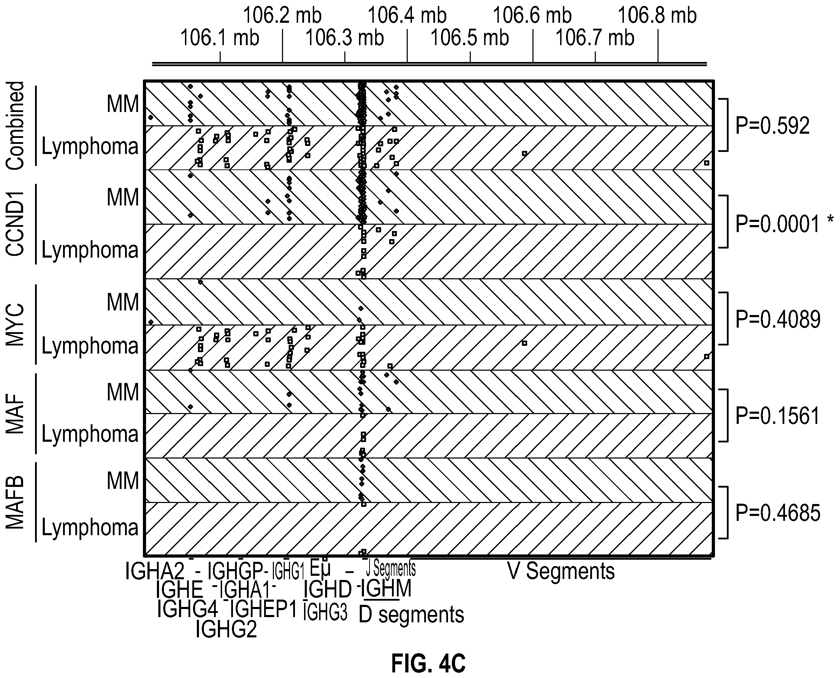

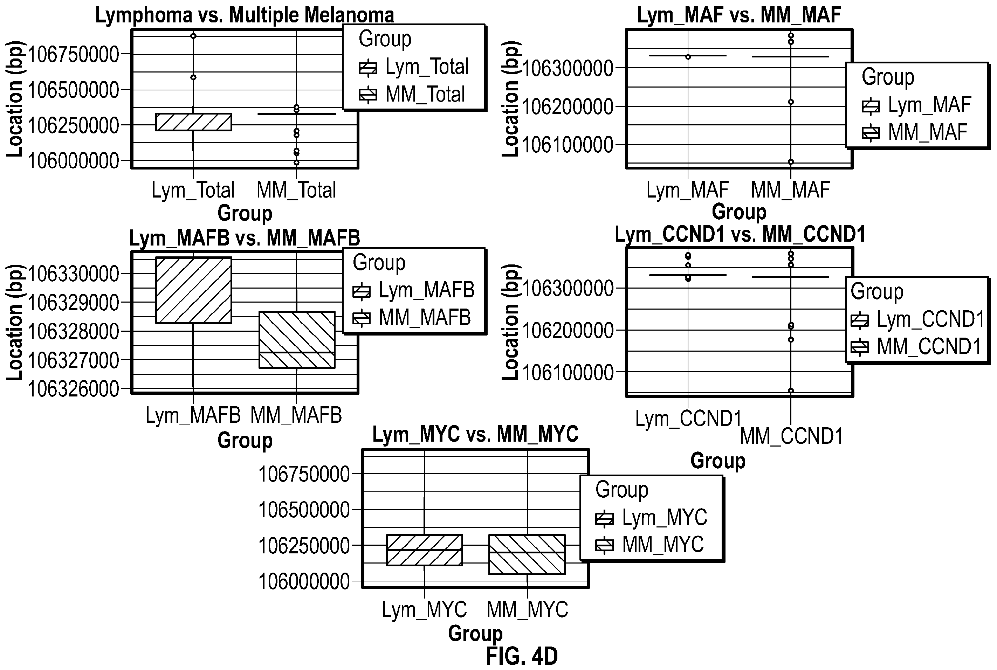

Next, the extent to which the breakpoint distribution within the IGH locus might contribute to oncogene overexpression was examined. In this context, the distances between breakpoints within the IGH locus and the IGH enhancer E$\mu$ did not affect the outcome of the analysis (**FIGS. 4A & 4B**). It was then examined whether cancer type-specific IGH breakpoints might be influencing IGH breakpoint distribution. When different IGH translocations were pooled together, there was no statistical difference between MM and lymphomas in the distribution of the IGH breakpoints. When different IGH translocations were analyzed individually, only IGH-CCND1 showed statistically different IGH breakpoint distribution between MM and lymphomas (**FIGS. 4C & 4D**). These results indicate that in general, cancer type is not the major determinant of breakpoint distribution, although certain IGH translocations (e.g. IGH-CCND1) may favor specific breakpoint regions over others in different cancer types. These data also suggest that the breakpoint distribution within the IGH locus is not the major determinant of IGH-mediated target oncogene overexpression.

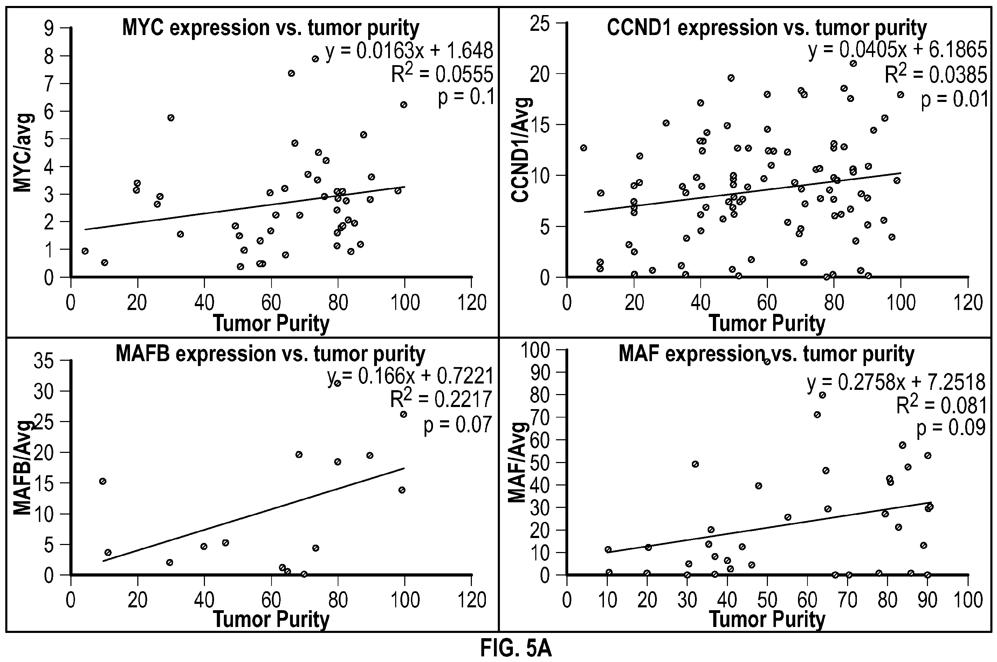

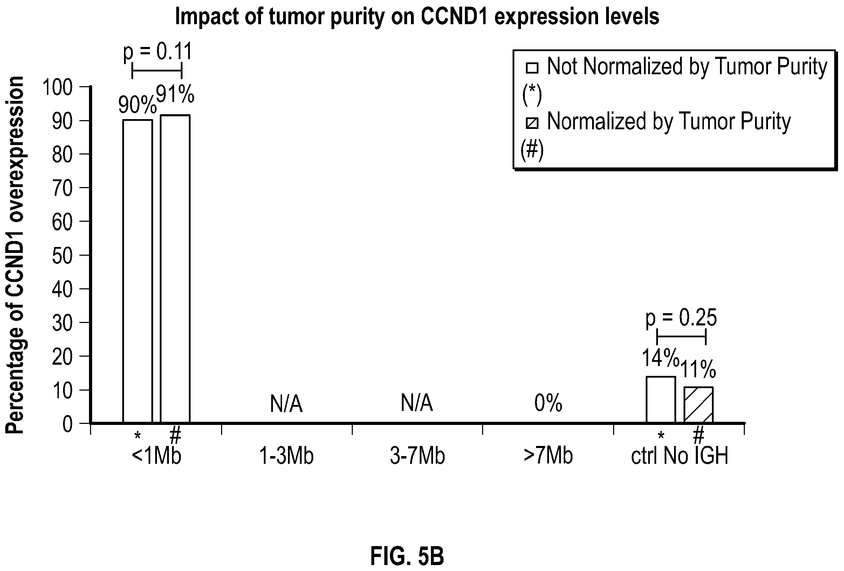

Finally, the extent to which sample tumor purity fraction might have impacted the analysis was tested. First, the correlation between the normalized oncogene expression levels and tumor purity fraction was analyzed for each IGH translocation in this study. The correlation coefficients were low for each oncogene, and correlation between tumor purity and expression levels were not statistically significant, except for IGH-CCND1 (**FIG. 5A**). To further explore the impact that sample tumor purity may have on expression levels, expression levels of *CCND1* was normalized in IGH-CCND1-positive samples, as well as in non-IGH controls by sample tumor purity, and the patterns of overexpression in each distance range were reanalyzed, as depicted in **FIG. 1A**. It was found that tumor purity fraction did not impact the percentage of samples exhibiting *CCND1* overexpression (**FIG. 5B**). Therefore, these data indicate that the normalization used was sufficient to negate potential complicating effects of sample tumor purity on oncogene expression in this study.

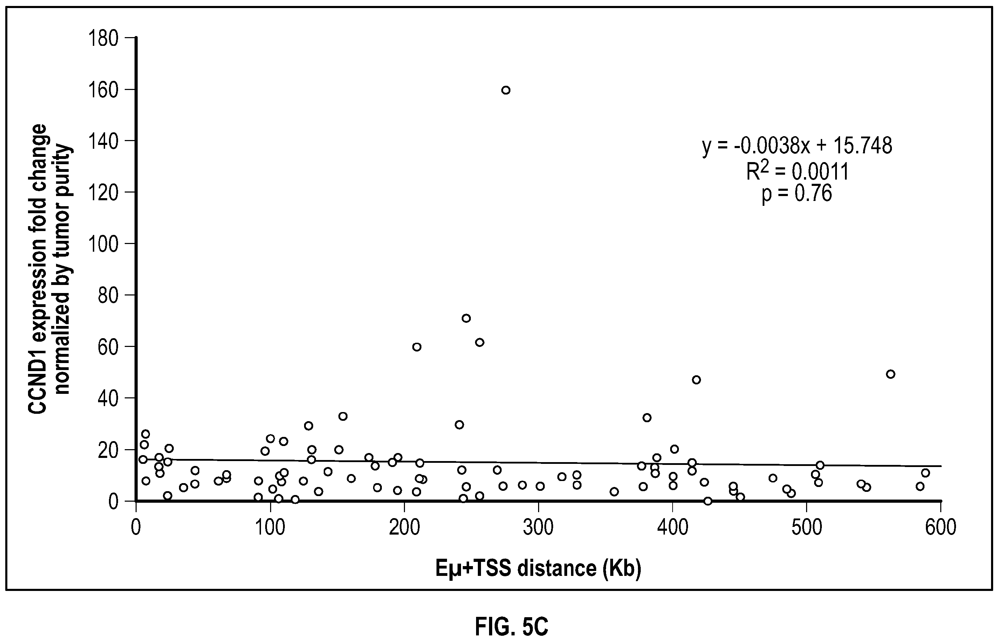

Moreover, when *CCND1* expression levels normalized by tumor purity were plotted against E$\mu$+TSS distance (distance between breakpoint within the IGH locus to E$\mu$ plus the distance between IGH breakpoint and TSS) (**FIG. 5C**), the relationship between the expression level and distance within the 0-1.3 Mb range from the *CCND1* TSS were no longer significant (compare to **FIG. 3B**), supporting the conclusion that within this effective distance range, the magnitude of oncogene overexpression varies independently of the IGH-driven mechanisms.

Taken together, these results demonstrate that oncogene expression is highly likely to be upregulated when IGH translocation breakpoints are within 1.3 Mb from the oncogene's TSS, whereas at larger distances, especially > 2.1 Mb away from TSS, IGH enhancers have no appreciable effect on targeted oncogene expression. Within the 0-1.3 Mb range, the oncogene expression levels do not exhibit a strong correlation with the distance from IGH. These results underscore the importance of assessing oncogene expression when testing for the presence of IGH rearrangements, as even within the effective distance range, a small fraction of IGH translocations fail to upregulate expression of the targeted oncogene, and define an effective boundary of 1.3Mb, beyond which oncogene expression is unlikely to be affected by the IGH enhancers.

***Example 3: IGH translocations detected by NGS are highly concordant with FISH analysis***

To assess the concordance between the IGH rearrangements identified by NGS and by FISH, the NGS sequencing results from this study were compared with the FISH analysis obtained from the pathology reports accompanying patient samples. Of the 83 samples with the available FISH data, 72 (86.7%) showed complete concordance between the two methods. Among the concordant cases with available RNA data, 71.4% (20/28) exhibited overexpression of the IGH-targeted oncogenes (Tables 2-4). Among the 11 cases with the discordant NGS and FISH results, 7 (63.6%) were the cases where IGH translocations were detected by NGS and not by FISH, 2 (18.2%) were the cases where IGH translocations were detected by FISH but not by NGS, and 2 (18.2%) were the cases where NGS and FISH detected different IGH translocations (Tables 3 and 5), suggesting that NGS may have higher sensitivity in detecting IGH translocations than FISH. NGS findings of clinically significant IGH rearrangements such as IGH-BCL2 and IGH-BCL6 in FISH-negative cases may impact treatment decisions for these patients.

**Table 2.** Summary of concordance between results of NGS testing with external FISH testing.

|  | Number of cases | Concordant cases | Percentage |
|---|---|---|---|
| Current study | 83 | 72 | 86.7 |
| Previous study* | 38 | 35 | 92 |
| Total | 121 | 107 | 88.4 |

**Table 3.** Summary of correlation between results of NGS testing with external FISH testing.

A. Concordance

|  | Current study (n=83) | | | Previous study*. (n=38) | Total Percentage |
|---|---|---|---|---|---|
|  | Concordant | Percentage | Concordant | Percentage |  |
| **Concordant** | **72** | **86.7** | **35** | **92** | **88.4** |
|  | Concordance by type | | | | |
| detected by both methods | 50 | 60.2 | 13 | 34.2 | 52 |
| No IGH rearrangement detected by either method | 22 | 26.5 | 22 | 57.9 | 36.4 |

B. Discordance

|  | Current study (n=83) | | Previous study*. (n=38) | | Total Percentage |
|---|---|---|---|---|---|
|  | Discordant | Percentage | Discordant | Percentage |  |
| **Discordant** | **11** | **13.3** | **3** | **7.9** | **11.6** |
|  | Discordance by type | | | | |
| Detected by NGS only | 7 | 8.4 | 2 | 5.3 | 7.4 |
| Overlapping but not identical IGH rearrangements | 1 | 1.2 | 0 | 0 | 0.8 |
| detected by NGS and FISH |  |  |  |  |  |
| Different IGH rearrangements detected by NGS and FISH | 1 | 1.2 | 0 | 0 | 0.8 |
| Detected by FISH only | 2 | 2.4 | 1 | 2.6 | 2.5 |

* He, J. (2016) Blood 127:3004-3014.

**Table 4.** List of samples with concordant NGS and FISH results.

| Case number | Rearrang ement | Tumor Type | Distance (kb) | Expression Fold change over control avg |
|---|---|---|---|---|
| 1 | MYC | Diffuse large B cell lymphoma (DLBCL) | 0.2 | 2.8 |
| 2 | | Diffuse large B cell lymphoma (DLBCL) | 1.1 | 4.3 |
| 3 | | Diffuse large B cell lymphoma (DLBCL) | 1.4 | 3.9 |
| 4 | | Diffuse large B cell lymphoma (DLBCL) | 1.9 | 3.6 |
| 5 | | B-cell lymphoma (NOS) | 0.1 | 1.7 |
| 6 | | B-cell lymphoma (NOS) | 1.5 | 5.3 |
| 7 | | Burkitt lymphoma | 7.4 | Not Determined |
| 8 | | Burkitt lymphoma | 1.3 | Not Determined |
| 9 | | Burkitt lymphoma | 0 | Not Determined |
| 10 | | Burkitt lymphoma | 1.2 | Not Determined |
| 11 | BCL2 | Mature B-cell neoplasm (NOS) | 1.3 | Not Determined |
| 12 | | Mature B-cell neoplasm (NOS) | 3.0 | Not Determined |
| 13 | | Bone marrow lymph proliferative disease (NOS) | 195.3 | Not Determined |
| 14 | | B-cell lymphoma (NOS) | 3.3 | 8.4 |
| 15 | | B-cell lymphoma (NOS) | 1.5 | 4.4 |
| 16 | | Follicular lymphoma | 27.0 | 1.3 |
| 17 | | Follicular lymphoma | 0.0 | 1.8 |
| 18 | | Follicular lymphoma | 3.0 | 2.5 |
| 19 | | Diffuse large B cell lymphoma (DLBCL) | 2.9 | 1.0 |
| 20 | | Non-Hodgkins lymphoma (NOS) | 3.0 | Not Determined |
| 21 | MYC, BCL2 | Diffuse large B cell lymphoma (DLBCL) | 2.7 (MYC), 27 (BCL2) | 5.9 (MYC), 6.4 (BCL2) |
| 22 | | Diffuse large B cell lymphoma (DLBCL) | 1.1 (MYC), 3 (BCL2) | 1.2 (MYC), 1.7 (BCL2) |
| 23 | | Diffuse large B cell lymphoma (DLBCL) | 0.7 (MYC), 2.5 (BCL2) | 2.4 (MYC), 2 (BCL2) |
| 24 | | Diffuse large B cell lymphoma (DLBCL) | 0.1 (MYC), 3.0 (BCL2) | 3.9 (MYC), 0.6 (BCL2) |
| 25 | | Diffuse large B-cell lymphoma (NOS) | 2.1 (MYC), 3.0 (BCL2) | 1.6 (MYC), 1.8 (BCL2) |
| 26 | | B-cell lymphoma (NOS) | 0.11 (MYC), 3 (BCL2) | 2.8 (MYC), 2.4 (BCL2) |
| 27 | MYC, BCL6 | Diffuse large B cell lymphoma (DLBCL) | 0.9 (MYC), 270.0 (BCL6) | 3.0 (MYC), 1.4 (BCL6) |
| 28 | BCL2, BCL6 | B-cell lymphoma (NOS) | 26.1 (BCL2), 269.9 (BCL6) | 0.8 (BCL2), 0.7 (BCL6) |

(continued)

| Case number | Rearrangement | Tumor Type | Distance (kb) | Expression Fold change over control avg |
|---|---|---|---|---|
| 29 | MYC, BCL2, BCL6 | B-cell lymphoma (NOS) | 3.7 (MYC), 27 (BCL2), 270 (BCL6) | 3.0 (MYC), 2.9 (BCL2), 1.5 (BCL6) |
| 30 | MYEOV/CCND1 | Mantle cell lymphoma | 109.0 | Not Determined |
| 31 | | Mantle cell lymphoma | 109.0 | Not Determined |
| 32 | | Mantle cell lymphoma | 86.2 | Not Determined |
| 33 | | Mantle cell lymphoma | 108.7 | Not Determined |
| 34 | | Mantle cell lymphoma | 109.0 | Not Determined |
| 35 | | Mantle cell lymphoma | 170.7 | Not Determined |
| 36 | | Mantle cell lymphoma | 34.0 | Not Determined |
| 37 | | Mantle cell lymphoma | 109.0 | Not Determined |
| 38 | | Mantle cell lymphoma | 109.0 | Not Determined |
| 40 | | Mantle cell lymphoma | 109.0 | Not Determined |
| 41 | | Mantle cell lymphoma | 39.7 | Not Determined |
| 42 | | Mantle cell lymphoma | 4.5 | Not Determined |
| 43 | | Mantle cell lymphoma | 219.3 | Not Determined |
| 44 | PAX5 | B-cell lymphoma (NOS) | 198.6 | 4.2 |
| 45 | BCL3 | Chronic Lymphocytic Leukemia (CLL) | 1175.0 | 0.7 |
| 46 | | Chronic Lymphocytic Leukemia (CLL) | 7.0 | 2.0 |
| 47 | MAF | Multiple myeloma | 1078.1 | 25.2 |
| 48 | MAFB | Multiple myeloma | 600.1 | 21.5 |
| 49 | TCL6 | Chronic Lymphocytic Leukemia (CLL) | 466.3 | 8.0 |
| 50 | MMSET/FGFR3 | Multiple myeloma | 81.1 | 89.7 |
| 51 | Not detected by either method | B-cell lymphoma (NOS) | N/A | N/A |
| 52 | | Chronic Lymphocytic Leukemia (CLL) | N/A | N/A |
| 53 | | Mantle cell lymphoma | N/A | N/A |
| 54 | | Mantle cell lymphoma | N/A | N/A |
| 55 | | Mantle cell lymphoma | N/A | N/A |
| 56 | | Diffuse large B cell lymphoma (DLBCL) | N/A | N/A |

(continued)

| Case number | Rearrangement | Tumor Type | Distance (kb) | Expression Fold change over control avg |
|---|---|---|---|---|
| 57 | | Diffuse large B cell lymphoma (DLBCL) | N/A | N/A |
| 58 | | Diffuse large B cell lymphoma (DLBCL) | N/A | N/A |
| 59 | | Diffuse large B cell lymphoma (DLBCL) | N/A | N/A |
| 60 | | Diffuse large B cell lymphoma (DLBCL) | N/A | N/A |
| 61 | | Diffuse large B cell lymphoma (DLBCL) | N/A | N/A |
| 62 | Not detected by either method | Diffuse large B cell lymphoma (DLBCL) | N/A | N/A |
| 63 | | Diffuse large B cell lymphoma (DLBCL) | N/A | N/A |
| 64 | | B-cell lymphoma (NOS) | N/A | N/A |
| 65 | | B-cell lymphoma (NOS) | N/A | N/A |
| 66 | | B-cell lymphoma (NOS) | N/A | N/A |
| 67 | | B-cell lymphoma (NOS) | N/A | N/A |
| 68 | | Multiple myeloma | N/A | N/A |
| 69 | | Multiple myeloma | N/A | N/A |
| 70 | | Multiple myeloma | N/A | N/A |
| 71 | | Myelodysplastic syndrome (MDS) | N/A | N/A |
| 72 | | Mantle cell lymphoma | N/A | N/A |

Not Determined: insufficient control samples to evaluate fold change
N/A: not available because no IGH rearrangements were detected.
Number of cases with RNA information: 28; number of cases with overexpressed IGH-targeted oncogenes: 20; ratio of cases with overexpressed IGH-targeted oncogenes: 71.4%.

**Table 5.** List of samples with discordant NGS and FISH results.

| IGH rearrangements detected by NGS only: | | | | | |
|---|---|---|---|---|---|
| Case number | Rearrangements detected by NGS | FISH results | Tumor Type | Distance from TSS (kb) | Normalized Expression Fold change |
| 73 | IGH-BCL2 | Negative for IGH-BCL2, IGH-BCL6 and IGH-MYC | B-cell Lymphoma (NOS) | 3 | 1.3 |
| 74 | IGH-BCL2 | Negative for IGH translocations | Chronic lymphocytic leukemia (CLL) | 196.1 | 2.8 |
| 75 | IGH-MYEOV/CCND1 | 1.6% of the cells contained CCND1 break-apart signal, the rest was normal. It was considered normal karyotype | Acute Myelocytic Leukemia (AML) | 54.643 | 15.1 |

(continued)

| Case number | Rearrangements detected by NGS | FISH results | Tumor Type | Distance from TSS (kb) | Normalized Expression Fold change |
|---|---|---|---|---|---|
| IGH rearrangements detected by NGS only: | | | | | |
| 76 | IGH-MYEOV/CCND1 | Negative for IGH translocations | Myelodysplastic-myeloproliferative neoplasm (MDS-MPN) | 253.6 | Not Determined |
| 77 | IGH-MYC | Negative for IGH-BCL2, IGH-BCL6 and IGH-MYC | Burkitt lymphoma | 0.4 | Not Determined |
| 78 | IGH-MYC | Negative for IGH-BCL2, IGH-BCL6 and IGH-MYC | Burkitt lymphoma | 0.53 | Not Deter-mined |
| 79 | IGH-16q11 rearran-gement | Negative for IGH transloca-tions | Chronic lympho-cytic leukemia (CLL) | N/A | N/A |
| Overlapping but not identical IGH rearrangements detected by NGS and FISH: | | | | | |
| 80 | IGH-BCL2, BCL6 rearrangement without a partner, no MYC rearrangement detected | Positive for MYC and BCL2 rearrangements, negative BCL6 rearrangement | Diffuse large B cell lymphoma (DLBCL) | 2.9 | 1.5 (BCL2); 0.6 (MYC); 1.1 (BCL6) |
| Different IGH rearrangements detected by NGS and FISH: | | | | | |
| 81 | IGH-BCL6 | Positive for IGH-BCL2; BCL6 translocation was not tested | Diffuse large B cell lymphoma (DLBCL) | 270.0 | 0.2 (BCL2); 1.2 (BCL6) |
| IGH rearrangements detected by FISH only: | | | | | |
| 82 | None | Positive for IGH-CCND1 | Multiple myeloma | N/A | 6.2 |
| 83 | None | Positive for IGH-CCND1 | Multiple myeloma | N/A | 2.9 |
| Not Determined: insufficient control samples to evaluate told change N/A: no oncogene was close to translocation breakpoint (sample 79) | | | | | |

In addition, in a different, non-overlapping patient cohort, NGS testing was concordant with FISH testing in 35/38 cases, identifying an IGH-BCL2 and an IGH-BCL6 rearrangements in 2/24 FISH-negative samples (He, J. (2016) Blood 127:3004-3014). Together with the data in this Example, the concordance of FMI testing with FISH testing in the two studies was 88.4% (107/121), with only 4% (5/121) of cases in which NGS testing did not detect an IGH rearrangement identified by FISH testing.

Importantly, these results show that NGS may detect IGH translocations with therapeutic implications in "unexpected" cancer types, where such translocations are rare and are therefore not routinely assessed. For example, IGH-BCL2 translocation was detected only by NGS in a CLL case (case 74), with overexpressed BCL2, which may suggest particular relevance of BCL2 inhibitors, such as venetoclax, for this patient. Another example is Case 76, in which IGH-MYEOV/CCND1 translocation was detected by NGS in a patient sample with MDS-MPN, and may suggest that CDK inhibitors, such as palbociclib, may benefit this patient (Table S3) and that this patient may also have an occult lymphoma.

Overall, these results show high concordance between NGS and FISH analyses, and low NGS failure rate for IGH translocation detection, as in 94.6% of cases NGS testing either confirmed FISH results or identified additional IGH rearrangements missed by FISH. The ability of NGS testing such as FoundationOne® Heme to detect clinically significant IGH rearrangements such as IGH-BCL2, in addition to interrogating clinically relevant copy number changes, point mutations, and indels, highlights the utility of the comprehensive genomic profiling approach in routine patient care.

*Conclusions*

This is thought to be the first study that systematically analyzed the relationship between IGH-mediated oncogene overexpression and the distance separating IGH from the targeted oncogene in a large set of primary patient samples. It was demonstrated that whether or not an oncogene is overexpressed is highly dependent on the distance from the IGH to the oncogene (**FIGS. 2A-2F**), while the location of the breakpoint within the IGH locus had no significant impact on target oncogene overexpression. More importantly, the range of breakpoint distances that were associated with oncogene overexpression were defined: oncogenes targeted by IGH translocations were overexpressed in most of the cases in which distances between IGH breakpoint and oncogene TSSs were $\leq$ 1.3 Mb, and no overexpression of oncogenes was detected when breakpoint distances were greater than 2.1 Mb. Overall, these results suggest that the distance between an oncogene and a breakpoint with IGH is a major determinant of oncogene overexpression and define an "effective distance range" for IGH-mediated oncogene overexpression.

Different translocations showed distinct distribution patterns of breakpoint distances from corresponding oncogene TSS's (**FIG. 1A**), suggesting that different translocations may use different mechanisms to generate breakpoints. Consistent with these findings, Greisman, H.A. et al. (2012) Blood 120:2864-2867 demonstrated that IGH-CCND1 and IGH-MYC or IGH-BCL6 translocations favored different DNA motifs for breakpoint generation. Finally, since individual cancer cases have unique features, differential expression and/or regulation of factors that facilitate gene expression may play a role in differential IGH-mediated expression.

Moreover, IGH breakpoints farther than 1 Mb from the targeted oncogene have been very rarely reported, and whether these translocations lead to long-distance overexpression of the targeted oncogene, or can even be reliably identified as long-distance events in the course of routine clinical workup, has previously not been extensively investigated. Here it was found that IGH translocation breakpoints located >1Mb away from the TSS's of corresponding oncogenes occurred in 6.9% of examined cases (**FIG. 1B**). Among them, IGH-MAF and IGH-MAFB translocations reliably showed overexpression of *MAF* and *MAFB,* respectively, when breakpoints were within 1-1.3 Mb range (**FIGS. 2A-2F**). These represent the first reported examples of long-distance translocation-induced overexpression. Notably, these translocations were much less frequent than short-distance ones.

Importantly, these analyses suggest that within the optimal distance range (0-1.3 Mb), there is a lack of strong correlation between oncogene expression levels and the distance between translocation breakpoints and oncogene TSS's (**FIG. 5C**). Given the wide variation of expression levels within this distance range (**FIGS. 3A & 3B**), these findings indicate that factors in addition to IGH enhancers influence the precise regulation of gene expression in these cancer cells. Alternatively, it is possible that in a fraction of cases, IGH enhancers actually drive alternative oncogenes despite proximity to the well-characterized oncogenes.

An important implication of this analysis is that IGH rearrangements located within a certain distance of a target oncogene can differentially impact patient outcome depending on the distance from the oncogene and oncogene expression.

Finally, it was demonstrated that NGS-based comprehensive genomic profiling can accurately and reliably identify diverse IGH rearrangements, including rare events and those appearing in unexpected disease types. NGS technology also provides high-resolution breakpoint information, allowing more accurate identification of partner genes within the effective distance range. Coupled with oncogene expression levels, as well as interrogation of various classes of other alterations, including point mutations, short insertions/deletions, rearrangements/fusions, chromosome/arm-level events, and copy number changes that can have important prognostic, diagnostic and therapeutic impact, comprehensive genomic profiling can be expected to enable more personalized management of patient care.

**Claims**

1. A targeted therapy that inhibits expression and/or activity of a protein product of an oncogene for use in a method of treating or delaying progression of cancer in an individual, wherein the individual has been diagnosed with cancer, said method comprising:

   (a) detecting a presence of a translocation comprising a first breakpoint in the IGH locus and a second breakpoint in a sample from the individual, wherein the second breakpoint is 1.3Mb or less from a TSS of an oncogene; and
   (b) administering to the individual an effective amount of the targeted therapy based at least in part on the detection of the second breakpoint 1.3Mb or less from the TSS of the oncogene in the sample.

2. The targeted therapy for use according to claim 1, wherein the targeted therapy comprises:

   (a) an antibody that specifically binds a protein product of the oncogene, optionally wherein the targeted therapy comprises an antibody that inhibits expression and/or activity of a protein product of the oncogene;
(b) a compound that inhibits an activity of a protein product of the oncogene, optionally wherein the targeted therapy comprises a compound that inhibits an enzymatic activity of the protein product of the oncogene, optionally wherein the compound is a competitive or a non-competitive inhibitor of the protein product; or
(c) a nucleic acid that inhibits expression of a protein product of the oncogene; optionally wherein the targeted therapy comprises an antisense nucleic acid, ribozyme, siRNA, shRNA, miRNA, gRNA, or triple helix nucleic acid that inhibits expression of a protein product of the oncogene.

3. The targeted therapy for use according to claim 1 or claim 2, wherein the targeted therapy:

(a) inhibits expression and/or activity of a protein product of, or activation of one or more signaling pathways stimulated by, CCND1, MYEOV, MYC, MAF, MAFB, BCL6, BCL2, FGFR3, WHSC1, BCL3, NBEAP1, BCL10, BCL11A, CCND2, CCND3, CCNE1, CD44, CDK6, CEBPA, CEBPB, CEBPD, CEBPE, CRLF2, ID4, DDX6, DUX4, EPOR, GPR34, FCRL4, FCGR2B, IRF4, IRF8, CD274, PDCD1LG2, LHX2, LHX4, PAX5, IGF2BP1, TNFSF13, MALT1, FOXP1, IL3, miR-125b-1, MUC1, SOX5, or MYCN; and/or
(b) comprises a cyclin-dependent kinase (CDK) inhibitor, an inhibitor of expression and/or activity of a protein product of MYC, a MAF inhibitor, a BCL6 inhibitor, a BCL2 inhibitor, a BCL3 inhibitor, BCL8 inhibitor, a BCL10 inhibitor, a BCL11A inhibitor, an FGFR3 inhibitor, a pan-FGFR inhibitor, a WHSC1 inhibitor, an anti-CD44 antibody or antibody:drug conjugate (ADC), a chimeric antigen receptor (CAR) targeting CD44 and/or a T cell expressing a CAR targeting CD44, a CEBPB inhibitor, a CEBPD inhibitor, a DUX4 inhibitor, an EPOR inhibitor, an anti-FCGR2B antibody, a PD-1 antagonist, a PD-L1 antagonist, a PD-L2 antagonist, a PAX5 inhibitor, an IGF2BP1 inhibitor, a TNFSF13 inhibitor, a BCMA inhibitor, a MALT1 inhibitor, an anti-IL3 antibody, an anti-IL3R antibody, an anti-MUC1 antibody, a CAR targeting MUC1 and/or a T cell expressing a CAR targeting MUC1, a MUC1 antigenic peptide, a dendritic cell loaded with one or more MUC1 antigenic peptide(s), a virus or viral vector encoding one or more human MUC1 peptide(s) or polypeptide(s), a BET inhibitor, an alcohol dehydrogenase inhibitor, or a MYCN inhibitor.

4. The targeted therapy for use according to any one of claims 1 to 3, wherein the oncogene is CCND1, MYEOV, MYC, MAF, MAFB, BCL6, BCL2, FGFR3, WHSC1, BCL3, NBEAP1, BCL10, BCL11A, CCND2, CCND3, CCNE1, CD44, CDK6, CEBPA, CEBPB, CEBPD, CEBPE, CRLF2, ID4, DDX6, DUX4, EPOR, GPR34, FCRL4, FCGR2B, IRF4, IRF8, CD274, PDCD1LG2, LHX2, LHX4, PAX5, IGF2BP1, TNFSF13, MALT1, FOXP1, IL3, miR-125b-1, MUC1, SOX5 or MYCN.

5. The targeted therapy for use according to any one of claims 1 to 4, wherein the cancer is multiple myeloma (MM), B-cell lymphoma, diffuse large B-cell lymphoma (DLBCL), B-cell lymphoma unclassified, acute myeloid leukemia (AML), follicular lymphoma (FL), Burkitt lymphoma, acute lymphoblastic leukemia (ALL), B-cell acute lymphoblastic leukemia (B-ALL), non-Hodgkin's lymphoma (NHL), B-cell non-Hodgkin's lymphoma (B-NHL), Waldenstroms lymphoma, extranodal lymphoma, mantle cell lymphoma (MCL), mature B cell neoplasm, hairy cell leukemia (HCL), hairy cell leukemia variant (HCL-v), splenic marginal zone lymphoma (SMZL), nodal marginal zone lymphoma, extranodal marginal zone lymphoma, MALT lymphoma, myeloproliferative neoplasm (MPN), skin lymphoma, lymphoproliferative disease, primary mediastinal large B-cell lymphoma (PMBCL), plasmablastic lymphoma, chronic lymphocytic leukemia (CLL), small cell lymphoma (SLL), Hodgkin's lymphoma (HL), bone marrow lymph proliferative disease, myelodysplastic syndrome, or angioimmunoblastic T-cell lymphoma (AITL).

6. The targeted therapy for use according to any one of claims 1 to 5, wherein:

(a) the oncogene is MYC, the targeted therapy comprises a MYC inhibitor, and the cancer is DLBCL, B-cell lymphoma, Burkitt lymphoma, multiple myeloma, ALL, B-ALL, NHL, MCL, mature B cell neoplasm, MPN, skin lymphoma, CLL, or lymphoproliferative disease;
(b) the oncogene is BCL2, the targeted therapy comprises a BCL2 inhibitor, and the cancer is DLBCL, FL, B-cell lymphoma, ALL, CLL, HL, NHL, bone marrow lymph proliferative disease, or lymphoproliferative disease;
(c) the oncogene is BCL6, the targeted therapy comprises a BCL6 inhibitor, and the cancer is DLBCL, FL, multiple myeloma, SMZL, NHL, PMBCL, or plasmablastic lymphoma;
(d) the oncogene is CCND1, the targeted therapy comprises a CDK inhibitor, and the cancer is multiple myeloma, B-cell lymphoma, AML, DLBCL, or FL;
(e) the oncogene is MAF, the targeted therapy comprises a MAF inhibitor, and the cancer is multiple myeloma, CLL, ALL, AITL, or DLBCL;
(f) the oncogene is MAFB, the targeted therapy comprises a MAFB inhibitor, and the cancer is multiple myeloma, ALL, B-ALL, or DLBCL; or

(g) the oncogene is WHSC1 or FGFR3, the targeted therapy comprises a WHSC1 inhibitor, an FGFR3 inhibitor, or a pan-FGFR inhibitor, and the cancer is multiple myeloma or DLBCL

7. A method of assessing overexpression of an oncogene in an individual, wherein the individual has been diagnosed with cancer, the method comprising:

(a) detecting a presence of a translocation comprising a first breakpoint in the IGH locus and a second breakpoint in a sample from the individual, wherein the second breakpoint is 1.3Mb or less from a TSS of the oncogene; and
(b) providing an assessment of overexpression of the oncogene when the second breakpoint is detected 1.3Mb or less from the TSS of the oncogene.

8. The targeted therapy for use according to any one of claims 1 to 6, or the method according to claim 7, wherein the translocation results in at least a 2-fold increase, at least a 5-fold increase, or at least a 20-fold increase in expression of the oncogene, as compared to a reference; optionally wherein the expression of the oncogene is expression of oncogene RNA or expression of oncogene polypeptide.

9. The targeted therapy for use according to any one of claims 1 to 6 or 8, or the method according to claim 7 or claim 8, wherein the second breakpoint is 1.0Mb or less, 500kb or less, 100kb or less, 10kb or less, or 1kb or less from the TSS of the oncogene.

10. The targeted therapy for use according to any one of claims 1 to 6, 8 or 9, or the method according to any one of claims 7 to 9, wherein the translocation is detected in DNA from the sample; optionally wherein the translocation is detected in the sample by one or more methods selected from the group consisting of next-generation sequencing (NGS), a nucleic acid hybridization assay, an amplification-based assay, a PCR-RFLP assay, real-time or quantitative PCR, sequencing, a screening analysis, FISH, spectral karyotyping or MFISH, comparative genomic hybridization, in situ hybridization, sequence-specific priming (SSP) PCR, HPLC, and mass-spectrometric genotyping.

11. The targeted therapy for use according to any one of claims 1 to 6 or 8 to 10, or the method according to any one of claims 7 to 10, wherein the sample is a nucleic acid sample; optionally wherein the nucleic acid sample comprises cell-free DNA (cfDNA) or circulating tumor DNA (ctDNA).

12. The targeted therapy for use according to any one of claims 1 to 6 or 8 to 10, or the method according to any one of claims 7 to 10, wherein the sample comprises fluid, protein, cells, or tissue; optionally wherein (a) the sample is from a tumor biopsy or specimen, or (b) the sample comprises a circulating tumor cell.

13. The targeted therapy for use according to any one of claims 1 to 6 or 8 to 12, or the method according to any one of claims 7 to 12, wherein the translocation comprises translocation of one or more enhancers of the IGH locus with a different region of the genome.

14. The targeted therapy for use according to any one of claims 1 to 6 or 8 to 13, or the method according to any one of claims 7 to 13, wherein the individual is a human.

**Patentansprüche**

1. Zielgerichtete Therapie, die die Expression und/oder Aktivität eines Proteinprodukts eines Onkogens hemmt, zur Verwendung in einem Verfahren zur Behandlung oder Verzögerung des Fortschreitens von Krebs bei einem Individuum, wobei bei dem Individuum Krebs diagnostiziert wurde, wobei das Verfahren umfasst:

(a) Nachweisen des Vorhandenseins einer Translokation, die einen ersten Bruchpunkt im IGH-Locus und einen zweiten Bruchpunkt umfasst, in einer Probe des Individuums, wobei der zweite Bruchpunkt 1,3 Mb oder weniger von einer TSS eines Onkogens entfernt ist; und
(b) Verabreichen einer wirksamen Menge der zielgerichteten Therapie an das Individuum, basierend zumindest teilweise auf dem Nachweis, dass der zweite Bruchpunkt 1,3 Mb oder weniger von der TSS des Onkogens entfernt ist, in der Probe.

2. Zielgerichtete Therapie zur Verwendung nach Anspruch 1, wobei die zielgerichtete Therapie umfasst:

(a) einen Antikörper, der spezifisch an ein Proteinprodukt des Onkogens bindet, gegebenenfalls wobei die zielgerichtete Therapie einen Antikörper umfasst, der die Expression und/oder Aktivität eines Proteinprodukts des Onkogens hemmt;

(b) eine Verbindung, die eine Aktivität eines Proteinprodukts des Onkogens hemmt, gegebenenfalls wobei die zielgerichtete Therapie eine Verbindung umfasst, die eine enzymatische Aktivität des Proteinprodukts des Onkogens hemmt, gegebenenfalls wobei die Verbindung ein kompetitiver oder ein nicht-kompetitiver Inhibitor des Proteinprodukts ist; oder

(c) eine Nukleinsäure, die die Expression eines Proteinprodukts des Onkogens hemmt; gegebenenfalls wobei die zielgerichtete Therapie eine Antisense-Nukleinsäure, ein Ribozym, eine siRNA, eine shRNA, eine miRNA, eine gRNA oder eine Tripelhelix-Nukleinsäure umfasst, die die Expression eines Proteinprodukts des Onkogens hemmt.

3.  Zielgerichtete Therapie zur Verwendung nach Anspruch 1 oder Anspruch 2, wobei die zielgerichtete Therapie:

(a) die Expression und/oder Aktivität eines Proteinprodukts von CCND1, MYEOV, MYC, MAF, MAFB, BCL6, BCL2, FGFR3, WHSC1, BCL3, NBEAP1, BCL10, BCL11A, CCND2, CCND3, CCNE1, CD44, CDK6, CEBPA, CEBPB, CEBPD, CEBPE, CRLF2, ID4, DDX6, DUX4, EPOR, GPR34, FCRL4, FCGR2B, IRF4, IRF8, CD274, PDCD1LG2, LHX2, LHX4, PAX5, IGF2BP1, TNFSF13, MALT1, FOXP1, IL3, miR-125b-1, MUC1, SOX5 oder MYCN oder die Aktivierung eines oder mehrerer durch diese stimulierte Signalwege hemmt; und/oder

(b) einen Inhibitor der Cyclin-abhängigen Kinase (CDK), einen Inhibitor der Expression und/oder Aktivität eines Proteinprodukts von MYC, einen MAF-Inhibitor, einen BCL6-Inhibitor, einen BCL2-Inhibitor, einen BCL3-Inhibitor, einen BCL8-Inhibitor, einen BCL10-Inhibitor, einen BCL11A-Inhibitor, einen FGFR3-Inhibitor, einen pan-FGFR-Inhibitor, einen WHSC1-Inhibitor, einen Anti-CD44-Antikörper oder ein Antikörper-Wirkstoff-Konjugat (ADC), einen auf CD44 abzielenden chimären Antigenrezeptor (CAR) und/oder eine T-Zelle, die einen auf CD44 abzielenden CAR exprimiert, einen CEBPB-Inhibitor, einen CEBPD-Inhibitor, einen DUX4-Inhibitor, einen EPOR-Inhibitor, einen Anti-FCGR2B-Antikörper, einen PD-1-Antagonisten, einen PD-L1-Antagonisten, einen PD-L2-Antagonisten, einen PAX5-Inhibitor, einen IGF2BP1-Inhibitor, einen TNFSF13-Inhibitor, einen BCMA-Inhibitor, einen MALT1-Inhibitor, einen Anti-IL3-Antikörper, einen Anti-IL3R-Antikörper, einen Anti-MUC1-Antikörper, einen auf MUC1 abzielenden CAR und/oder eine T-Zelle, die einen auf MUC1 abzielenden CAR exprimiert, ein MUC1-antigenes Peptid, eine dendritische Zelle, die mit einem oder mehreren MUC1-antigenen Peptiden beladen ist, ein Virus oder einen viralen Vektor, das/der ein oder mehrere humane MUCl-Peptide oder -Polypeptide kodiert, einen BET-Inhibitor, einen Alkoholdehydrogenase-Inhibitor oder einen MYCN-Inhibitor umfasst.

4.  Zielgerichtete Therapie zur Verwendung nach einem der Ansprüche 1 bis 3, wobei das Onkogen CCND1, MYEOV, MYC, MAF, MAFB, BCL6, BCL2, FGFR3, WHSC1, BCL3, NBEAP1, BCL10, BCL11A, CCND2, CCND3, CCNE1, CD44, CDK6, CEBPA, CEBPB, CEBPD, CEBPE, CRLF2, ID4, DDX6, DUX4, EPOR, GPR34, FCRL4, FCGR2B, IRF4, IRF8, CD274, PDCD1LG2, LHX2, LHX4, PAX5, IGF2BP1, TNFSF13, MALT1, FOXP1, IL3, miR-125b-1, MUC1, SOX5 oder MYCN ist.

5.  Zielgerichtete Therapie zur Verwendung nach einem der Ansprüche 1 bis 4, wobei der Krebs multiples Myelom (MM), B-Zell-Lymphom, diffuses großzelliges B-Zell-Lymphom (DLBCL), nicht klassifiziertes B-Zell-Lymphom, akute myeloische Leukämie (AML), follikuläres Lymphom (FL), Burkitt-Lymphom, akute lymphatische Leukämie (ALL), akute lymphatische B-Zell-Leukämie (B-ALL), Non-Hodgkin-Lymphom (NHL), B-Zell-Non-Hodgkin-Lymphom (B-NHL), Waldenström-Lymphom, extranodales Lymphom, Mantelzelllymphom (MCL), reife B-Zell-Neoplasie, Haarzellleukämie (HCL), Haarzellleukämie-Variante (HCL-v), splenisches Marginalzonenlymphom (SMZL), nodales Marginalzonenlymphom, extranodales Marginalzonenlymphom, MALT-Lymphom, myeloproliferative Neoplasie (MPN), Hautlymphom, lymphoproliferative Erkrankung, primäres mediastinales großzelliges B-Zell-Lymphom (PMBCL), plasmablastisches Lymphom, chronische lymphatische Leukämie (CLL), kleinzelliges Lymphom (SLL), Hodgkin-Lymphom (HL), lymphoproliferative Knochenmarkserkrankung, myelodysplastisches Syndrom oder angioimmunoblastisches T-Zell-Lymphom (AITL) ist.

6.  Zielgerichtete Therapie zur Verwendung nach einem der Ansprüche 1 bis 5, wobei:

(a) das Onkogen MYC ist, die zielgerichtete Therapie einen MYC-Inhibitor umfasst und der Krebs DLBCL, B-Zell-Lymphom, Burkitt-Lymphom, multiples Myelom, ALL, B-ALL, NHL, MCL, reife B-Zell-Neoplasie, MPN, Hautlymphom, CLL oder eine lymphoproliferative Erkrankung ist;

(b) das Onkogen BCL2 ist, die zielgerichtete Therapie einen BCL2-Inhibitor umfasst und der Krebs DLBCL, FL, B-Zell-Lymphom, ALL, CLL, HL, NHL, eine lymphoproliferative Knochenmarkserkrankung oder eine lymphoproliferative Erkrankung ist;

(c) das Onkogen BCL6 ist, die zielgerichtete Therapie einen BCL6-Inhibitor umfasst und der Krebs DLBCL, FL, multiples Myelom, SMZL, NHL, PMBCL oder plasmablastisches Lymphom ist;

(d) das Onkogen CCND1 ist, die zielgerichtete Therapie einen CDK-Inhibitor umfasst und der Krebs multiples Myelom, B-Zell-Lymphom, AML, DLBCL oder FL ist;

(e) das Onkogen MAF ist, die zielgerichtete Therapie einen MAF-Inhibitor umfasst und der Krebs multiples Myelom, CLL, ALL, AITL oder DLBCL ist;

(f) das Onkogen MAFB ist, die zielgerichtete Therapie einen MAFB-Inhibitor umfasst und der Krebs multiples Myelom, ALL, B-ALL oder DLBCL ist; oder

(g) das Onkogen WHSC1 oder FGFR3 ist, die zielgerichtete Therapie einen WHSC1-Inhibitor, einen FGFR3-Inhibitor oder einen pan-FGFR-Inhibitor umfasst und der Krebs multiples Myelom oder DLBCL ist.

7. Verfahren zur Beurteilung der Überexpression eines Onkogens bei einem Individuum, wobei bei dem Individuum Krebs diagnostiziert wurde, wobei das Verfahren umfasst:

(a) Nachweisen des Vorhandenseins einer Translokation, die einen ersten Bruchpunkt im IGH-Locus und einen zweiten Bruchpunkt umfasst, in einer Probe des Individuums, wobei der zweite Bruchpunkt 1,3 Mb oder weniger von einer TSS des Onkogens entfernt ist; und

(b) Bereitstellen einer Beurteilung der Überexpression des Onkogens, wenn der zweite Bruchpunkt 1,3 Mb oder weniger von der TSS des Onkogens entfernt nachgewiesen wird.

8. Zielgerichtete Therapie zur Verwendung nach einem der Ansprüche 1 bis 6 oder Verfahren nach Anspruch 7, wobei die Translokation zu einer mindestens 2-fachen, mindestens 5-fachen oder mindestens 20-fachen Steigerung der Expression des Onkogens im Vergleich zu einer Referenz führt; gegebenenfalls wobei es sich bei der Expression des Onkogens um die Expression von Onkogen-RNA oder die Expression von Onkogen-Polypeptid handelt.

9. Zielgerichtete Therapie zur Verwendung nach einem der Ansprüche 1 bis 6 oder 8 oder Verfahren nach Anspruch 7 oder 8, wobei der zweite Bruchpunkt 1,0 Mb oder weniger, 500 kb oder weniger, 100 kb oder weniger, 10 kb oder weniger oder 1 kb oder weniger von der TSS des Onkogens entfernt ist.

10. Zielgerichtete Therapie zur Verwendung nach einem der Ansprüche 1 bis 6, 8 oder 9 oder Verfahren nach einem der Ansprüche 7 bis 9, wobei die Translokation in der DNA der Probe nachgewiesen wird; gegebenenfalls wobei die Translokation in der Probe durch ein oder mehrere Verfahren nachgewiesen wird, die aus der Gruppe, bestehend aus Next-Generation-Sequenzierung (NGS), einem Nukleinsäurehybridisierungsassay, einem amplifikationsbasierten Assay, einem PCR-RFLP-Assay, Echtzeit- oder quantitativer PCR, Sequenzierung, einer Screening-Analyse, FISH, spektraler Karyotypisierung oder MFISH, vergleichender genomischer Hybridisierung, In-situ-Hybridisierung, sequenzspezifischer Priming-PCR (SSP-PCR), HPLC und massenspektrometrischer Genotypisierung, ausgewählt sind.

11. Zielgerichtete Therapie zur Verwendung nach einem der Ansprüche 1 bis 6 oder 8 bis 10 oder Verfahren nach einem der Ansprüche 7 bis 10, wobei die Probe eine Nukleinsäureprobe ist; gegebenenfalls wobei die Nukleinsäureprobe zellfreie DNA (cfDNA) oder zirkulierende Tumor-DNA (ctDNA) umfasst.

12. Zielgerichtete Therapie zur Verwendung nach einem der Ansprüche 1 bis 6 oder 8 bis 10 oder Verfahren nach einem der Ansprüche 7 bis 10, wobei die Probe Flüssigkeit, Protein, Zellen oder Gewebe umfasst; gegebenenfalls wobei (a) die Probe aus einer Tumorbiopsie oder einem Tumorpräparat stammt oder (b) die Probe eine zirkulierende Tumorzelle umfasst.

13. Zielgerichtete Therapie zur Verwendung nach einem der Ansprüche 1 bis 6 oder 8 bis 12 oder Verfahren nach einem der Ansprüche 7 bis 12, wobei die Translokation die Translokation eines oder mehrerer Enhancer des IGH-Locus in eine andere Region des Genoms umfasst.

14. Zielgerichtete Therapie zur Verwendung nach einem der Ansprüche 1 bis 6 oder 8 bis 13 oder Verfahren nach einem der Ansprüche 7 bis 13, wobei das Individuum ein Menschen ist.

**Revendications**

1. Thérapie ciblée qui inhibe l'expression et/ou l'activité d'un produit protéique d'un oncogène utilisable dans une méthode de traitement ou de retardement de la progression d'un cancer chez un individu diagnostiqué d'un cancer, ladite méthode comprenant :

    (a) la détection de la présence d'une translocation comprenant un premier point de rupture dans le locus IGH et un second point de rupture dans un échantillon provenant de l'individu, le second point de rupture étant situé à 1,3 Mb au plus d'un TSS d'un oncogène ; et
    (b) l'administration à l'individu d'une quantité efficace de l'agent de thérapie ciblée en se basant au moins en partie sur la détection du second point de rupture à 1,3 Mb au plus du TSS de l'oncogène dans l'échantillon.

2. Thérapie ciblée utilisable selon la revendication 1, la thérapie ciblée comprenant :

    (a) un anticorps qui se lie spécifiquement à un produit protéique de l'oncogène, la thérapie ciblée comprenant en option un anticorps qui inhibe l'expression et/ou l'activité d'un produit protéique de l'oncogène ;
    (b) un composé qui inhibe une activité d'un produit protéique de l'oncogène, la thérapie ciblée comprenant en option un composé qui inhibe une activité enzymatique du produit protéique de l'oncogène, le composé étant en option un inhibiteur compétitif ou non compétitif du produit protéique ; ou
    (c) un acide nucléique qui inhibe l'expression d'un produit protéique de l'oncogène ; la thérapie ciblée comprenant en option un acide nucléique antisens, un ribozyme, un ARNsi, un ARNsh, un ARNmi, un ARNg ou un acide nucléique à triple hélice qui inhibe l'expression d'un produit protéique de l'oncogène.

3. Thérapie ciblée utilisable selon la revendication 1 ou la revendication 2, la thérapie ciblée :

    (a) inhibant l'expression et/ou l'activité d'un produit protéique de, ou l'activation d'une ou de plusieurs voies de signalisation stimulées par CCND1, MYEOV, MYC, MAF, MAFB, BCL6, BCL2, FGFR3, WHSC1, BCL3, NBEAP1, BCL10, BCL11A, CCND2, CCND3, CCNE1, CD44, CDK6, CEBPA, CEBPB, CEBPD, CEBPE, CRLF2, ID4, DDX6, DUX4, EPOR, GPR34, FCRL4, FCGR2B, IRF4, IRF8, CD274, PDCD1LG2, LHX2, LHX4, PAX5, IGF2BP1, TNFSF13, MALT1, FOXP1, IL3, miR-125b-1, MUC1, SOX5 ou MYCN ; et/ou
    (b) comprenant un inhibiteur de kinase cycline-dépendante (CDK), un inhibiteur de l'expression et/ou de l'activité d'un produit protéique de MYC, un inhibiteur de MAF, un inhibiteur de BCL6, un inhibiteur de BCL2, un inhibiteur de BCL3, un inhibiteur de BCL8, un inhibiteur de BCL10, un inhibiteur de BCL11A, un inhibiteur de FGFR3, un inhibiteur pan-FGFR, un inhibiteur de WHSC1, un anticorps anti-CD44 ou un conjugué anticorps-médicament (ADC), un récepteur d'antigène chimérique (CAR) ciblant CD44 et/ou un lymphocyte T exprimant un CAR ciblant CD44, un inhibiteur de CEBPB, un inhibiteur de CEBPD, un inhibiteur de DUX4, un inhibiteur d'EPOR, un anticorps anti-FCGR2B, un antagoniste de PD-1, un antagoniste de PD-L1, un antagoniste de PD-L2, un inhibiteur de PAX5, un inhibiteur d'IGF2BP1, un inhibiteur de TNFSF 13, un inhibiteur de BCMA, un inhibiteur de MALT1, un anticorps anti-IL3, un anticorps anti-IL3R, un anticorps anti-MUC1, un CAR ciblant MUC1 et/ou un lymphocyte T exprimant un CAR ciblant MUC1, un peptide antigénique MUC1, une cellule dendritique chargée d'un ou plusieurs peptides antigéniques MUC1, un virus ou un vecteur viral codant pour un ou plusieurs peptides ou polypeptides MUC1 humains, un inhibiteur de BET, un inhibiteur de l'alcool déshydrogénase ou un inhibiteur de MYCN.

4. Thérapie ciblée utilisable selon l'une quelconque des revendications 1 à 3, dans laquelle l'oncogène est CCND1, MYEOV, MYC, MAF, MAFB, BCL6, BCL2, FGFR3, WHSC1, BCL3, NBEAP1, BCL10, BCL11A, CCND2, CCND3, CCNE1, CD44, CDK6, CEBPA, CEBPB, CEBPD, CEBPE, CRLF2, ID4, DDX6, DUX4, EPOR, GPR34, FCRL4, FCGR2B, IRF4, IRF8, CD274, PDCD1LG2, LHX2, LHX4, PAX5, IGF2BP1, TNFSF13, MALT1, FOXP1, IL3, miR-125b-1, MUC1, SOX5 ou MYCN.

5. Thérapie ciblée utilisable selon l'une quelconque des revendications 1 à 4, dans laquelle le cancer est un myélome multiple (MM), un lymphome à cellules B, un lymphome diffus à grandes cellules B (LDGCB), un lymphome à cellules B non classé, une leucémie myéloïde aiguë (LMA), un lymphome folliculaire (LF), un lymphome de Burkitt, une leucémie lymphoblastique aiguë (LLA), une leucémie lymphoblastique aiguë à cellules B (LLA-B), un lymphome non hodgkinien (LNH), un lymphome non hodgkinien à cellules B (LNH-B), un lymphome de Waldenström, un lymphome extraganglionnaire, un lymphome à cellules du manteau (LCM), une néoplasie à cellules B matures, une leucémie à tricholeucocytes (LT), une variante de la leucémie à tricholeucocytes (LT-V), un lymphome de la zone marginale splénique (LZMS), un lymphome de la zone marginale ganglionnaire, un lymphome de la zone marginale extraganglionnaire, un lymphome MALT, une néoplasie myéloproliférative (NMP), un lymphome cutané, une maladie lymphoproliférative, un lymphome médiastinal primitif à grandes cellules B (LMPGB), un lymphome plasmablastique, une leucémie lymphoïde chronique (LLC), un lymphome à petits lymphocytes (LPL), un lymphome hodgkinien (LH), une maladie lymphoproliférative de la moelle osseuse, un syndrome myélodysplasique ou un lymphome T angio-immunoblastique (LTAI).

6. Thérapie ciblée utilisable selon l'une quelconque des revendications 1 à 5, dans laquelle :

(a) l'oncogène est MYC, la thérapie ciblée comprend un inhibiteur de MYC, et le cancer est un LDGCB, un lymphome à cellules B, un lymphome de Burkitt, un myélome multiple, une LLA, une LLA-B, un LNH, un LCM, une néoplasie à cellules B matures, une NMP, un lymphome cutané, une LLC ou une maladie lymphoproliférative ;
(b) l'oncogène est BCL2, la thérapie ciblée comprend un inhibiteur de BCL2, et le cancer est un LDGCB, un LF, un lymphome à cellules B, une LLA, une LLC, un LH, un LNH, une maladie lymphoproliférative de la moelle osseuse ou une maladie lymphoproliférative ;
(c) l'oncogène est BCL6, la thérapie ciblée comprend un inhibiteur de BCL6, et le cancer est un LDGCB, un LF, un myélome multiple, un LZMS, un LNH, un LMPGB ou un lymphome plasmablastique ;
(d) l'oncogène est CCND1, la thérapie ciblée comprend un inhibiteur de CDK, et le cancer est un myélome multiple, un lymphome à cellules B, une LMA, un LDGCB ou un LF ;
(e) l'oncogène est MAF, la thérapie ciblée comprend un inhibiteur de MAF, et le cancer est un myélome multiple, une LLC, une LLA, un LTAI ou un LDGCB ;
(f) l'oncogène est MAFB, la thérapie ciblée comprend un inhibiteur de MAFB, et le cancer est un myélome multiple, une LLA, une LLA-B ou un LDGCB ; ou
(g) l'oncogène est WHSC1 ou FGFR3, le traitement ciblé comprend un inhibiteur de WHSC1, un inhibiteur de FGFR3 ou un inhibiteur pan-FGFR, et le cancer est un myélome multiple ou un LDGCB.

7. Méthode d'évaluation de la surexpression d'un oncogène chez un individu, dans laquelle l'individu a reçu un diagnostic de cancer, la méthode comprenant :

(a) la détection de la présence d'une translocation comprenant un premier point de rupture dans le locus IGH et un second point de rupture dans un échantillon provenant de l'individu, le second point de rupture étant situé à 1,3 Mb au plus d'un TSS de l'oncogène ; et
(b) la fourniture d'une évaluation de la surexpression de l'oncogène lorsque le second point de rupture est détecté à 1,3 Mb au plus du TSS de l'oncogène.

8. Thérapie ciblée utilisable selon l'une quelconque des revendications 1 à 6, ou méthode selon la revendication 7, dans laquelle la translocation entraîne une augmentation d'au moins 2 fois, d'au moins 5 fois ou d'au moins 20 fois de l'expression de l'oncogène, par rapport à une référence ; l'expression de l'oncogène étant en option l'expression de l'ARN de l'oncogène ou l'expression du polypeptide de l'oncogène.

9. Thérapie ciblée utilisable selon l'une quelconque des revendications 1 à 6 ou 8, ou méthode selon la revendication 7 ou la revendication 8, dans laquelle le second point de rupture est à 1,0 Mb au plus, à 500 kb au plus, à 100 kb au plus, à 10 kb au plus, ou à 1 kb au plus du TSS de l'oncogène.

10. Thérapie ciblée utilisable selon l'une quelconque des revendications 1 à 6, 8 ou 9, ou méthode selon l'une quelconque des revendications 7 à 9, dans laquelle la translocation est détectée dans l'ADN de l'échantillon ; la translocation étant en option détectée dans l'échantillon par une ou plusieurs méthodes choisies dans l'ensemble constitué par un séquençage de nouvelle génération (NGS), un dosage par hybridation d'acides nucléiques, un dosage basé sur l'amplification, un dosage PCR-RFLP, une PCR en temps réel ou quantitative, un séquençage, une analyse par criblage, une FISH, un caryotypage spectral ou MFISH, une hybridation génomique comparative, une hybridation in situ, une PCR à amorçage spécifique de séquence (SSP), la HPLC et un génotypage par spectrométrie de masse.

11. Thérapie ciblée utilisable selon l'une quelconque des revendications 1 à 6 ou 8 à 10, ou méthode selon l'une quelconque des revendications 7 à 10, dans laquelle l'échantillon est un échantillon d'acide nucléique ; dans laquelle l'échantillon d'acide nucléique comprend en option de l'ADN acellulaire (ADNac) ou de l'ADN tumoral circulant (ADNtc).

12. Thérapie ciblée utilisable selon l'une quelconque des revendications 1 à 6 ou 8 à 10, ou méthode selon l'une quelconque des revendications 7 à 10, dans laquelle l'échantillon comprend un fluide, une protéine, des cellules ou un tissu ; (a) l'échantillon provenant en option d'une biopsie ou d'un prélèvement de tumeur, ou (b) l'échantillon comprenant en option une cellule tumorale circulante.

13. Thérapie ciblée utilisable selon l'une quelconque des revendications 1 à 6 ou 8 à 12, ou méthode selon l'une quelconque des revendications 7 à 12, dans laquelle la translocation comprend la translocation d'un ou plusieurs amplificateurs du locus IGH dans une région différente du génome.

14. Thérapie ciblée utilisable selon l'une quelconque des revendications 1 à 6 ou 8 à 13, ou méthode selon l'une quelconque des revendications 7 à 13, dans laquelle l'individu est un humain.

**FIG. 1A**

**Distribution of breakpoints**

Legend:
- <1 Mb
- 1Mb- 3Mb
- 3Mb- 7Mb
- >7 Mb

3-7 Mb
0.5%
(5/1000)

>7 Mb
5.2%
(52/1000)

1-3 Mb
1.2%
(12/1000)

<1 Mb
93.1%
(931/1000)

FIG. 1B

BCL2 gene

FIG. 1C

FIG. 2A

FIG. 2B

FIG. 2C

EP 4 267 767 B1

FIG. 2D

**FIG. 2E**

Expression distribution of IGH-MAF/MAFB (1-3Mb) — MAF/MAFB expression distribution of controls

FIG. 2F

FIG. 3A

FIG. 3B

EP 4 267 767 B1

**FIG. 4A**

EP 4 267 767 B1

FIG. 4B

**FIG. 4C**

FIG. 4D

FIG. 5A

# Impact of tumor purity on CCND1 expression levels

**FIG. 5B**

FIG. 5C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63129286
- EP 404097 A
- WO 199301161 A
- US 4816567 A
- WO 199824893 A
- WO 199634096 A
- WO 199633735 A
- WO 199110741 A
- US 5545807 A
- US 5545806 A
- US 5569825 A
- US 5625126 A
- US 5633425 A
- US 5661016 A
- US 5428130 A
- US 4683195 A
- EP 430402 A
- WO 2005059504 A
- US 4987071 A
- US 5116742 A
- US 5093246 A
- US 20040086884 A
- US 20030166282 A
- US 20030143204 A
- US 20040038278 A
- US 20030224432 A
- US 5539082 A
- US 5714331 A
- US 5719262 A
- US 6268490 B
- US 6670461 B
- US 6794499 B
- US 6998484 B
- US 7053207 B
- US 7084125 B
- US 7399845 B
- US 3687808 A
- WO 2011005861 A
- WO 8809810 A
- WO 8910134 A
- US 5489677 A
- US 5602240 A
- WO 9316185 A
- US 5571894 A
- US 5587458 A
- US 5869046 A
- US 6248516 B1
- US 5821337 A
- US 7527791 B

- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 9308829 A
- US 5731168 A
- WO 2009089004 A1
- US 4676980 A
- US 20060025576 A1
- US 20080069820 A
- WO 2008077546 A
- US 20030157108 A
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1
- WO 2003085107 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S.

- US 5500362 A
- WO 5821337 A
- WO 2006029879 A
- WO 2005100402 A
- US 6737056 B
- US 7332581 B
- WO 2004056312 A
- US 6194551 B
- WO 9951642 A

- US 20050014934 A1, Hinton
- US 7371826 B
- US 5648260 A
- US 5624821 A
- WO 9429351 A
- US 7521541 B
- US 3773919 A
- US 5491224 A
- US 6277569 B, Bittner

**Non-patent literature cited in the description**

- **KÜPPERS, R.** *Nat. Rev. Cancer*, 2005, vol. 5, 251-262
- **JIMÉNEZ, C. et al.** *J. Mol. Diagnostics*, 2016, vol. 19, 1-8
- **KUEHL, W.M. ; BERGSAGEL, P.L.** *Nat. Rev. Cancer*, 2002, vol. 2, 175-187
- **JEFFRIES, S.J. et al.** *Haematologica*, 2014, vol. 99, 1334-1342
- **CHAPIRO, E. et al.** *Cancer Genet.*, 2013, vol. 206, 162-173
- **WOO, J.S. et al.** *Erp. Hematol. Oncol.*, 2014, vol. 3, 16
- **DUPAIN, C. et al.** *Mol. Ther. Nucleic Acid*, 2017, vol. 6, 315-326
- **BERGMAN, C. et al.** *Blood*, 2015, vol. 126, 2466-2474
- **XU-MONETTE, Z.Y. et al.** *Mod. Pathol.*, 2015, vol. 28, 1555-1573
- **DE BRAEKELEER, M. et al.** *Mod. Clin. Oncol.*, 2016, vol. 4, 682-694
- **MANIER, S. et al.** *Nat. Rev. Clin. Oncol.*, 2016
- **SAWYER, J. R.** *Cancer Genet.*, 2011, vol. 204, 3-12
- **HECHT, J.L. et al.** *J. Clin, Oncol.*, 2000, vol. 18, 3707-3721
- **MOORMAN, A. V. et al.** *J. Clin. Oncol.*, 2012, vol. 30, 3100-3108
- **RUSSELL, L.I. et al.** *J. Clin. Oncol.*, 2014, vol. 32, 1453-1462
- **ZELENETZ, A.D. et al.** *J. Natl. Compr. Canc. Netw.*, 2016, vol. 14, 196-231
- **KALFF, A. et al.** *Blood Cancer J.*, 2012, vol. 2, e89-8
- **LEONARD, J.P. et al.** *Blood*, 2013, vol. 119, 4597-4607
- **NUNEZ, G. et al.** *Proc. Natl. Acad. Sci.*, 1989, vol. 86, 4589-4593
- **HAN, Y. et al.** *Zhonghua Yi Xue Yi Chuan Xue Za Zhi*, 2013, vol. 30, 143-147
- **MONNI, O. et al.** *Leuk. Lymphoma*, 1999, vol. 34, 45-52
- **MAESHIMA, A.M. et al.** *Cancer Sci.*, 2013, vol. 104, 952-957
- **SOUERS, A.J. et al.** *Nat. Med.*, 2013, vol. 19, 202-208

- **ROSS, J. et al.** *Blood*, 2015, vol. 126, 2975
- **SANCHEZ-IZQUIERDO, D. et al.** *Blood*, 2003, vol. 101, 4539-4546
- **YE, H. et al.** *J. Pathol.*, 2005, vol. 205, 293-301
- **HO, L. et al.** *Blood*, 2005
- **DU, M.Q.** *Best Pract. Res. Clin. Haematol.*, 2017
- **SCHULZE-LUEHRMANN, J. ; GHOSH, S.** *Immunity*, 2006, vol. 25, 701-715
- **GOZZETTI, A. ; LE BEAU, M.M.** *Semin. Hematol.*, 2000, vol. 37, 320-333
- **SANTRA, M. et al.** *Blood*, 2003, vol. 101, 2374-2376
- **SAMBROOK et al.** Molecular Cloning: A Laboratory Manual. Cold Spring Harbor Laboratory Press, 2001
- Current Protocols in Molecular Biology. 2003
- PCR 2: A Practical Approach. Methods in Enzymology. Academic Press, Inc., 1995
- Antibodies. A Laboratory Manual, and Animal Cell Culture. 1987
- Oligonucleotide Synthesis. 1984
- Methods in Molecular Biology. Humana Press
- Cell Biology: A Laboratory Notebook. Academic Press, 1998
- Animal Cell Culture. 1987
- **J.P. MATHER ; P.E. ROBERTS.** Introduction to Cell and Tissue Culture. Plenum Press, 1998
- Cell and Tissue Culture: Laboratory Procedures. J. Wiley and Sons, 1993
- Handbook of Experimental Immunology
- Gene Transfer Vectors for Mammalian Cells. 1987
- PCR: The Polymerase Chain Reaction. 1994
- Current Protocols in Immunology. 1991
- Short Protocols in Molecular Biology. Wiley and Sons, 1999
- **C.A. JANEWAY ; P. TRAVERS.** *Immunobiology*, 1997
- **P. FINCH.** *Antibodies*, 1997
- Antibodies: A Practical Approach. IRL Press, 1988
- Monoclonal Antibodies: A Practical Approach. Oxford University Press, 2000

- Using Antibodies: A Laboratory Manual. Cold Spring Harbor Laboratory Press, 1999
- The Antibodies. Harwood Academic Publishers, 1995
- Cancer: Principles and Practice of Oncology. J.B. Lippincott Company, 1993
- **KABAT et al.** Sequences of Proteins of Immunological Interest. National Institute of Health, 1991
- **XU et al.** *Immunity*, 2000, vol. 13, 37-45
- **JOHNSON** ; **WU**. Methods in Molecular Biology. Human Press, 2003, vol. 248, 1-25
- **HAMERS-CASTERMAN et al.** *Nature*, 1993, vol. 363, 446-448
- **SHERIFF et al.** *Nature Struct. Biol.*, 1996, vol. 3, 733-736
- **KABAT et al.** Sequences of Proteins of Immunological Interest. National Institutes of Health, 1991
- **CHOTHIA** ; **LESK**. *J. Mol. Biol.*, 1987, vol. 196, 901-917
- **KABAT et al.** Sequences of Immunological Interest. National Institutes of Health, 1991
- **PLUCKTHUN**. The Pharmacology of Monoclonal Antibodies. Springer-Verlag, 1994, vol. 113, 269-315
- **HUDSON et al.** *Nat. Med.*, 2003, vol. 9, 129-134
- **HOLLINGER et al.** *Proc. Natl. Acad. Sci. USA*, 1993, vol. 90, 6444-6448
- **HUDSON et al.** *Nat. Med*, 2003, vol. 9, 129-134
- **KOHLER** ; **MILSTEIN**. *Nature*, 1975, vol. 256, 495-97
- **HONGO et al.** *Hybridoma*, 1995, vol. 14 (3), 253-260
- **HARLOW et al.** Antibodies: A Laboratory Manual. Cold Spring Harbor Laboratory Press, 1988
- **HAMMERLING et al.** Monoclonal Antibodies and T-Cell Hybridomas. Elsevier, 1981, 563-681
- **CLACKSON et al.** *Nature*, 1991, vol. 352, 624-628
- **MARKS et al.** *J. Mol. Biol.*, 1992, vol. 222, 581-597
- **SIDHU et al.** *J. Mol. Biol.*, 2004, vol. 338 (2), 299-31 0
- **LEE et al.** *J. Mol. Biol.*, 2004, vol. 340 (5), 1073-1093
- **FELLOUSE**. *Proc. Natl. Acad Sci USA*, 2004, vol. 101 (34), 12467-12472
- **LEE et al.** *J Immunol. Methods*, vol. 284 (1 -2), 119-132
- **JAKOBOVITS et al.** *Proc. Natl. Acad. Sci. USA*, 1993, vol. 90, 2551
- **JAKOBOVITS et al.** *Nature*, 1993, vol. 362, 255-258
- **BRUGGEMANN et al.** *Year in Immunol*, 1993, vol. 7, 33
- **MARKS et al.** *Bio/Technology*, 1992, vol. 10, 779-783
- **LONBERG et al.** *Nature*, 1994, vol. 368, 856-859
- **MORRISON**. *Nature*, 1994, vol. 368, 812-813
- **FISHWILD et al.** *Nature Biotechnol.*, 1996, vol. 14, 845-851
- **NEUBERGER**. *Nature Biotechnol.*, 1996, vol. 14, 826
- **LONBERG et al.** *Intern. Rev. Immunol.*, 1995, vol. 13, 65-93
- **MORRISON et al.** *Proc. Natl. Acad. Sci. USA*, 1984, vol. 81, 6851-6855
- **MULLIS et al.** *Cold Spring Harbor Symp. Quant. Biol.*, 1987, vol. 51, 263
- **ERLICH**. PCR Technology. Stockton Press, 1989
- **HE, J.** *Blood*, 2016, vol. 127, 3004-3014
- **METZKER, M.** *Nature Biotechnology Reviews*, 2010, vol. 11, 31-46
- **TRAPNELL C.** ; **SALZBERG S.L.** *Nature Biotech.*, 2009, vol. 27, 455-457
- **WARREN R. et al.** *Bioinformatics*, 2007, vol. 23, 500-501
- **BUTLER J. et al.** *Genome Res.*, 2008, vol. 18, 810-820
- **ZERBINO D.R.** ; **BIRNEY E.** *Genome Res.*, 2008, vol. 18, 821-829
- **ALBERTSON**. *EEBO J.*, 1984, vol. 3, 1227-1234
- **PINKEL**. *Proc. Natl. Acad. Sci. USA*, 1988, vol. 85, 9138-9142
- situ Hybridization Protocols. Methods in Molecular Biology. Humana Press, 1994, vol. 33
- **RAINA, K. et al.** *Proc. Natl. Acad. Sci. USA*, 2016, vol. 113, 7124
- **WINTER, G.E. et al.** *Science*, 2015, vol. 348, 1376-1381
- **CARABET, L.A. et al.** *Int. J. Mol. Sci.*, 2019, vol. 20, 120
- **PETERSEN, B.L.** ; **SEARS, R.C.** *BioDrugs*, 2019, vol. 33, 539-553
- **HAN, H. et al.** *Cancer Cell*, 2019, vol. 36, 483-497
- **CARDENAS, M.G. et al.** *Clin. Cancer Res.*, 2017, vol. 23, 885-893
- **MORRISON, M.J. et al.** *PLoS One*, 2018, vol. 13, e0197082
- **MARASCO, D. et al.** *Biochem J.*, 2009, vol. 422, 553-561
- *CHEMICAL ABSTRACTS*, 1859072-53-9
- **COZMA, D. et al.** *J. Clin. Invest.*, 2007, vol. 117, 2602-2610
- **PUISSANT, A. et al.** *Cancer Discov.*, 2013, vol. 3, 308-323
- **GUO, Y.F. et al.** *Science Translational Medicine*, 2020, vol. 12, eaax8694

- **MULLER, I. et al.** *PLoS One*, 2014, vol. 9, e97285
- **CHAYKA, O. et al.** *J. Biol. Chem.*, 2014, vol. 290, 2198-2212
- **HALD, O.H. et al.** *Oncogene*, 2019, vol. 38, 2800-2813
- **WOOLF et al.** *Proc Natl Acad Sci USA*, 1992
- Antisense RNA and DNA. Cold Spring Harbor Laboratory, 1988, vol. 89, 7305-9
- **HASELHOFF** ; **GERLACH**. *Nature*, 1988, vol. 334, 585-59
- **HELENE, C.** *Anticancer Drug Des.*, 1991, vol. 6, 569-84
- **HELENE**. *Ann. N. Y. Acad. Sci.*, 1992, vol. 660, 27-36
- **MAHER**. *Bioassays*, 1992, vol. 14, 807-15
- **GAULTIER et al.** *Nucleic Acids. Res.*, 1987, vol. 15, 6625-6641
- **INOUE et al.** *Nucleic Acids Res*, 1987, vol. 15, 6131-6148
- **INOUE et al.** *FEBS Lett.*, 1987, vol. 215, 327-330
- **HASELHOFF** ; **GERLACH**. *Nature*, 1988, vol. 334, 585-591
- **BARTEL, D.** ; **SZOSTAK, J.W.** *Science*, 1993, vol. 261, 1411-1418
- **HELENE, C. I**. *Ann. N.Y. Acad. Sci.*, 1992, vol. 660, 27-36
- **MAHER, L.J.** *Bioassays*, 1992, vol. 14, 807-15
- **ELBASHIR et al.** *EMBO*, 2001, vol. 20, 6877-6888
- **CHU** ; **RANA**. *RNA*, 2007, vol. 14, 1714-1719
- **KIM et al.** *Nat Biotech*, 2005, vol. 23, 222-226
- Current protocols in nucleic acid chemistry. John Wiley & Sons, Inc.
- **CLEMENS et al.** *Proc. Natl. Acad. Sci. USA*, 2000, vol. 97, 6499-6503
- **BILLY et al.** *Proc. Natl. Sci. USA*, 2001, vol. 98, 14428-14433
- **ELBASHIR et al.** *Nature*, 2001, vol. 411, 494-8
- **YANG et al.** *Proc. Natl. Acad. Sci. USA*, 2002, vol. 99, 9942-9947
- **SIOLAS et al.** *Nat. Biotechnol.*, 2005, vol. 23 (2), 227-31
- **TOULMÉ**. *Nature Biotech.*, 2001, vol. 19, 17
- **FARIA et al.** *Nature Biotech.*, 2001, vol. 19, 40-44
- **ROONGJANG, S. et al.** *Nucleic Acids Symp Ser (Oxf)*, 2007, vol. 51, 113-114
- **IMANISHI T et al.** *Chem. Commun.*, 2002, vol. 16, 1653-1659
- **HYRUP B. et al.** *Bioorganic & Medicinal Chemistry*, 1996, vol. 4, 5-23
- **PERRY-O'KEEFE et al.** *Proc. Natl. Acad. Sci.*, vol. 93, 14670-675
- **NIELSEN et al.** *Science*, 1991, vol. 254, 1497-1500
- **JEPSON, J. et al.** *Oligonucleotides*, 2004, vol. 14, 130-146
- **ELMEN, J. et al.** *Nucleic Acids Research*, 2005, vol. 33 (1), 439-447
- **MOOK, OR. et al.** *Mol Canc Ther*, 2007, vol. 6 (3), 833-843
- **GRUNWELLER, A. et al.** *Nucleic Acids Research*, 2003, vol. 31 (12), 3185-3193
- Modified Nucleosides in Biochemistry, Biotechnology and Medicine. Wiley-VCH, 2008
- The Concise Encyclopedia Of Polymer Science And Engineering. John Wiley & Sons, 1990, 858-859
- **ENGLISCH et al.** *Angewandte Chemie*, 1991, vol. 30, 613
- **SANGHVI, Y S.** dsRNA Research and Applications. CRC Press, 1993, 289-302
- dsRNA Research and Applications. CRC Press, 276-278
- **LETSINGER et al.** *Proc. Natl. Acad. Sci. USA*, 1989, vol. 86, 6553-6556
- **LEMAITRE et al.** *Proc. Natl. Acad. Sci. USA*, 1987, vol. 84, 648-652
- **KROL et al.** *Bio-Techniques*, 1988, vol. 6, 958-976
- **ZON**. *Pharm. Res.*, 1988, vol. 5, 539-549
- **MARTIN et al.** *Helv. Chim. Acta*, 1995, vol. 78, 486-504
- *CHEMICAL ABSTRACTS*, 477202-00-9
- **CHEN et al.** *J. Mol. Biol.*, 1999, vol. 293, 865-881
- **PRESTA et al.** *Cancer Res.*, 1997, vol. 57, 4593-4599
- **PLUCKTHÜN**. The Pharmacology of Monoclonal Antibodies. Springer-Verlag, 1994, vol. 113, 269-315
- **ALMAGRO** ; **FRANSSON**. *Front. Biosci.*, 2008, vol. 13, 1619-1633
- **RIECHMANN et al.** *Nature*, 1988, vol. 332, 323-329
- **QUEEN et al.** *Proc. Nat'l Acad. Sci. USA*, 1989, vol. 86, 10029-10033
- **KASHMIRI et al.** *Methods*, 2005, vol. 36, 25-34
- **PADLAN**. *Mol. Immunol.*, 1991, vol. 28, 489-498
- **DALL' ACQUA et al.** *Methods*, 2005, vol. 36, 43-60
- **OSBOURN et al.** *Methods*, 2005, vol. 36, 61-68
- **KLIMKA et al.** *Br. J. Cancer*, 2000, vol. 83, 252-260
- **SIMS et al.** *J. Immunol.*, 1993, vol. 151, 2296
- **CARTER et al.** *Proc. Natl. Acad. Sci. USA*, 1992, vol. 89, 4285

- **PRESTA et al.** *J. Immunol.*, 1993, vol. 151, 2623
- **BACA et al.** *J. Biol. Chem.*, 1997, vol. 272, 10678-10684
- **ROSOK et al.** *J. Biol. Chem.*, 1996, vol. 271, 22611-22618
- **VAN DIJK** ; **VAN DE WINKEL**. *Curr. Opin. Pharmacol.*, 2001, vol. 5, 368-74
- **LONBERG**. *Curr. Opin. Immunol.*, 2008, vol. 20, 450-459
- **LONBERG**. *Nat. Biotech.*, 2005, vol. 23, 1117-1125
- **KOZBOR**. *J. Immunol.*, 1984, vol. 133, 3001
- **BRODEUR et al.** Monoclonal Antibody Production Techniques and Applications. Marcel Dekker, Inc., 1987, 51-63
- **BOERNER et al.** *J. Immunol.*, 1991, vol. 147, 86
- **LI et al.** *Proc. Natl. Acad. Sci. USA*, 2006, vol. 103, 3557-3562
- **NI**. *Xiandai Mianyixue*, 2006, vol. 26 (4), 265-268
- **VOLLMERS** ; **BRANDLEIN**. *Histology and Histopathology*, 2005, vol. 20 (3), 927-937
- **VOLLMERS** ; **BRANDLEIN**. *Methods and Findings in Experimental and Clinical Pharmacology*, 2005, vol. 27 (3), 185-91
- **HOOGENBOOM et al.** Methods in Molecular Biology. Human Press, 2001, vol. 178, 1-37
- **MCCAFFERTY et al.** *Nature*, vol. 348, 552-554
- **MARKS et al.** J. Mol. Biol.. 1992, vol. 222, 581-597
- **MARKS** ; **BRADBURY**. Methods in Molecular Biology. Human Press, 2003, vol. 248, 161-175
- **SIDHU et al.** *J. Mol. Biol.*, 2004, vol. 338 (2), 299-310
- **FELLOUSE**. *Proc. Natl. Acad. Sci. USA*, 2004, vol. 101 (34), 12467-12472
- **LEE et al.** *J. Immunol. Methods*, 2004, vol. 284 (1-2), 119-132
- **WINTER et al.** *Ann. Rev. Immunol.*, 1994, vol. 12, 433-455
- **GRIFFITHS et al.** *FAMBO J*, 1993, vol. 12, 725-734
- **HOOGENBOOM** ; **WINTER**. *J. Mol. Biol.*, 1992, vol. 227, 381-388
- **MILSTEIN** ; **CUELLO**. *Nature*, 1983, vol. 305, 537
- **TRAUNECKER et al.** *EMBO J.*, 1991, vol. 10, 3655
- **BRENNAN et al.** *Science*, 1985, vol. 229, 81
- **KOSTELNY et al.** *J. Immunol.*, 1992, vol. 148 (5), 1547-1553
- **GRUBER et al.** *J. Immunol.*, 1994, vol. 152, 5368
- **TUTT et al.** *J. Immunol.*, 1991, vol. 147, 60
- **CHOWDHURY**. *Methods Mol. Biol.*, 2008, vol. 207, 179-196
- **CUNNINGHAM** ; **WELLS**. *Science*, 1989, vol. 244, 1081-1085
- **WRIGHT et al.** *TIBTECH*, 1997, vol. 15, 26-32
- **OKAZAKI et al.** *J. Mol. Biol.*, 2004, vol. 336, 1239-1249
- **YAMANE-OHNUKI et al.** *Biotech. Bioeng.*, 2004, vol. 87, 614
- **RIPKA et al.** *Arch. Biochem. Biophys.*, 1986, vol. 249, 533-545
- **KANDA, Y. et al.** *Biotechnol. Bioeng.*, 2006, vol. 94 (4), 680-688
- **RAVETCH** ; **KINET**. *Annu. Rev. Immunol.*, 1991, vol. 9, 457-492
- **HELLSTROM, I. et al.** *Proc. Nat'l Acad. Sci. USA*, 1986, vol. 83, 7059-7063
- **HELLSTROM, I et al.** *Proc. Nat 'l Acad. Sci. USA*, 1985, vol. 82, 1499-1502
- **BRUGGEMANN, M. et al.** *J. Exp. Med.*, 1987, vol. 166, 1351-1361
- **CLYNES et al.** *Proc. Nat'l Acad. Sci. USA*, 1998, vol. 95, 652-656
- **GAZZANO-SANTORO et al.** *J. Immunol. Methods*, 1996, vol. 202, 163
- **CRAGG, M.S. et al.** *Blood*, 2003, vol. 101, 1045-1052
- **CRAGG, M.S.** ; **M.J. GLENNIE**. *Blood*, 2004, vol. 103, 2738-2743
- **PETKOVA, S.B. et al.** *Int'l. Immunol.*, 2006, vol. 18 (12), 1759-1769
- **SHIELDS et al.** *J. Biol. Chem.*, 2001, vol. 9 (2), 6591-6604
- **IDUSOGIE et al.** *J. Immunol.*, 2000, vol. 164, 4178-4184
- **GUYER et al.** *J. Immunol.*, 1976, vol. 117, 587
- **KIM et al.** *J. Immunol.*, 1994, vol. 24, 249
- **DUNCAN** ; **WINTER**. *Nature*, 1988, vol. 322, 738-40
- **KAM et al.** *Proc. Natl. Acad. Sci. USA*, 2005, vol. 102, 11600-11605
- The Pharmacological Bases of Therapeutics. Pergamon Press, 1990
- Remington's Pharmaceutical Sciences. Mack Publishing Co., 1990
- Pharmaceutical Dosage Forms: Parenteral Medications Dekker. 1993
- Pharmaceutical Dosage Forms: Tablets Dekker. 1990
- Pharmaceutical Dosage Forms: Disperse Systems Dekker. 1990
- Dermatological and Transdermal Formulations (Drugs and the Pharmaceutical Sciences). Marcel Dekker, 2002, vol. 1 19
- Remington's Pharmaceutical Sciences. 1980
- **KERKHOFF, E.** ; **RAPP, U.R.** *Mol. Cell. Biol.*, 1997, vol. 17, 2576-2586

- **LI, X.S. et al.** *Genet. Mol. Res.*, 2015, vol. 14, 10752-10759
- **LIU, J.J. et al.** *Mol. Cell. Biol.*, 1995, vol. 15, 3654-3663
- **SMALLEY, K.S.M. et al.** *Mol. Cancer Ther.*, 2008, vol. 7, 2876-2883
- **KENT, W.J. et al.** *Genome Res.*, 2002, vol. 12, 996-1006
- **WALKER, B.A. et al.** *Blood Cancer*, 2014, vol. 4, e191
- **WALKER, B.A. et al.** *Blood*, 2013, vol. 121, 3413-3419
- **CLEARY, M.L. et al.** *Translocation*, 1988, vol. 47, 19-28
- **AFFER, M. et al.** *Leukemia*, 2014, vol. 28, 1725-1735
- **GREISMAN, H.A. et al.** *Blood*, 2012, vol. 120, 2864-2867